(19) Europäisches Patentamt
European Patent Office
Office européen des brevets

(11) EP 3 377 048 B1

(12) **EUROPEAN PATENT SPECIFICATION**

(45) Date of publication and mention of the grant of the patent:
**22.04.2020 Bulletin 2020/17**

(21) Application number: **16801029.6**

(22) Date of filing: **18.11.2016**

(51) Int Cl.:
***A61K 9/70*** *(2006.01)* ***A61K 31/485*** *(2006.01)*
***A61P 25/04*** *(2006.01)*

(86) International application number:
**PCT/GB2016/053600**

(87) International publication number:
**WO 2017/085502 (26.05.2017 Gazette 2017/21)**

(54) **ABUSE RESISTANT TRANSDERMAL DELIVERY DEVICES AND COMPOSITIONS COMPRISING AN OPIOID AGONIST AND A NON-TRANSDERMALLY DELIVERED N-OXIDE DERIVATIVE OF AN OPIOID ANTAGONIST FOR THE TREATMENT OF PAIN**

MISSBRAUCHSSICHERE TRANSDERMALE ABGABEVORRICHTUNGEN UND ZUSAMMENSETZUNGEN MIT EINEM OPIOIDAGONISTEN UND EINEM NICHT TRANSDERMAL VERABREICHTEN N-OXID-DERIVAT EINES OPIOID-ANTAGONISTEN ZUR SCHMERZBEHANDLUNG

DISPOSITIFS D'ADMINISTRATION TRANSDERMIQUES RÉSISTANTS AUX ABUS ET COMPOSITIONS COMPRENANT UN AGONISTE D'OPIOÏDE ET UN DÉRIVÉ N-OXYDE ADMINISTRÉ PAR VOIE NON TRANSDERMIQUE D'UN ANTAGONISTE D'OPIOÏDE POUR LE TRAITEMENT DE LA DOULEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **19.11.2015 GB 201520390**

(43) Date of publication of application:
**26.09.2018 Bulletin 2018/39**

(73) Proprietor: **Euro-Celtique S.A.**
**2350 Luxembourg (LU)**

(72) Inventor: **WHITELOCK, Steve**
**Cambridge**
**Cambridgeshire CB4 0GW (GB)**

(74) Representative: **Gordon, Kirsteen Helen**
**Marks & Clerk LLP**
**62-68 Hills Road**
**Cambridge CB2 1LA (GB)**

(56) References cited:
**WO-A1-2009/067275**
**WO-A1-2011/123866**
**WO-A2-03/070191**
**WO-A2-2005/081825**
**WO-A2-2008/070462**
**WO-A2-2009/120889**
**WO-A2-2011/009020**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 377 048 B1

## Description

BACKGROUND

The present invention relates to a transdermal delivery device comprising a N-oxide derivative of an opioid antagonist of formula (I), or a salt thereof, and an opioid agonist or a salt thereof. The invention also relates to a pharmaceutical composition for transdermal delivery comprising a N-oxide derivative of an opioid antagonist of formula (I), or a salt thereof, an opioid agonist or a salt thereof and an adhesive and/or a matrix-forming polymer and to methods for making the compositions. The invention also relates to medical uses of the transdermal delivery device and the composition.

INTRODUCTION

It is generally desirable to provide pharmaceuticals and delivery devices comprising the composition in an abuse-deterrent tamper-resistant form to maximise the chance that the pharmaceuticals are taken in the manner intended. This, in turn, ensures that the pharmaceutical is likely to have the full pharmacological effect desired. Even more significantly, the provision of pharmaceuticals and delivery devices in a tamper resistant form means that they are more difficult to abuse.

Pharmaceuticals comprising certain types of drugs are of course more likely to be targeted for abuse than others. Transdermal delivery devices comprising opioid agonists are frequently the target of abuse, mainly because they tend to contain relatively high amounts of opioid agonists for release over an extended period of time.

Opioid agonists are important pharmaceuticals for the treatment and management of pain. Abusers generally aim to modify delivery devices containing opioid agonists, particularly transdermal delivery devices that comprise relatively high amounts of opioid agonist, and then administer the opioid agonist in such a way that a high *in vivo* concentration is achieved over a short period of time so as to experience a euphorogenic effect. The opioid agonist-containing composition present in a transdermal delivery device may, for example, be extracted from the device and administered orally in a single dose. Another form of abuse that occurs is the extraction of opioid agonist-containing compositions from transdermal delivery devices to obtain a solution that may then be crudely administered by injection.

To minimise the possibility that abuse occurs, various strategies have been developed to provide opioid agonists, and in particular transdermal delivery devices, in tamper resistant form. For instance transdermal patches comprising an opioid agonist and an opioid antagonist separated by an impermeable barrier have been proposed so that the antagonist is only released if the patch is chewed or immersed in a solvent in an extraction process. Other delivery devices wherein the opioid agonist and the opioid antagonist are physically separated have also been disclosed.

The drawback of this type of tamper resistant transdermal delivery device, however, is that abusers may successfully isolate the portion or part of the delivery device that comprises the opioid agonist prior to attempting to extract the opioid agonist. If successful, if the abuser then chewed or extracted the isolated portion, he or she would likely achieve the delivery of a relatively high amount of opioid agonist in the absence of opioid antagonist which would result in the euphorogenic effect desired.

US2004/0033253 discloses a tamper resistant transdermal delivery device comprising an opioid, or a salt thereof, and an acyl opioid antagonist, or a salt thereof. The transdermal delivery device allows for an analgesically effective amount of the opioid to be transdermally administered to a subject. The acyl opioid antagonist is substantially skin impermeable so if the device is used as intended, i.e. transdermally, the acyl opioid antagonist does not affect the the analgesic effect of the opioid agonist. On the other hand, however, if the device is tampered with and an abuser tries to extract the opioid from the device then the acyl opioid anagonist is also extracted and it is hydrolysed in vivo to yield opioid antagonist which inhibits the euphoric effect of the opioid.

The acyl opioid antagonists in US2004/0033253 are described as opioid antagonists having one or more hydroxyl groups wherein a proton of the hydroxyl group is replaced with an acyl group. The specific examples recited in US2004/0033253 include acyl groups on different hydroxyl moieties. A wide range of acyl groups is also described.

WO2009/120889 describes compositions and transdermal delivery devices which comprise an opioid, an opioid agonist-antagonist or prodrugs thereof, in combination with an opioid antagonist. The opioid antagonist is insoluble in the dosage form or is not absorbable at a therapeutic rate or extent across the skin. This may be achieved by encapsulating or coating the antagonist or providing it in the form of nanoparticles or microparticles. If, however, the composition or device is abused, the encapsulating layer or coating is dissolved and the anatagonist is released to counteract the effect of the opioid agonist.

Nevertheless there is still a need for tamper resistant compositions, and in particular tamper resistant transdermal delivery devices, for opioid agonists.

SUMMARY OF INVENTION

Viewed from a first aspect, the present invention provides a transdermal delivery device comprising a pharmaceutical composition, wherein said composition comprises a N-oxide derivative of an opioid antagonist of formula (I), or a salt thereof, and an opioid agonist or salt thereof:

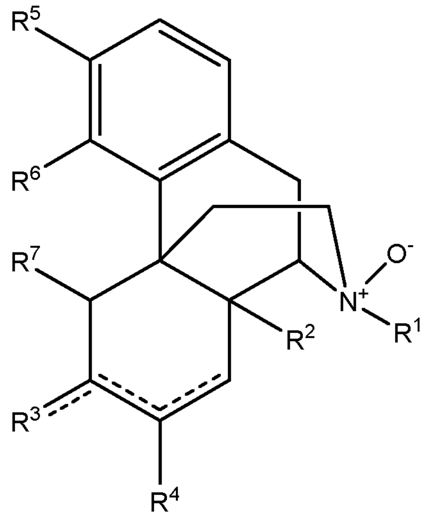

(I)

wherein

$R^1$ is selected from optionally substituted $C_{1-8}$ alkyl and optionally substituted $C_{2-8}$ alkenyl;
$R^2$ is selected from OH, H, $OC_{1-8}$ alkyl, NHCOR, $NR^1COR$, $CONR^1R$ and CONHR wherein R is a hydrocarbyl group or $R^2$ forms a bridge to the carbon to which $R^3$ is attached;
$R^3$ is selected from O, $CH_2$, $N-NH_2$ or a linking group to a second N-oxide derivative of an opioid antagonist or a salt thereof when the dashed bond is present and is selected from OH, H, $OC_{1-8}$ alkyl, $OCOR^1$, $O(CH_2CH_2(CH_2)_yO)_xCH_3$ wherein x is 1-10 and y is 0-3, $N(R^1)_2$, $NR^1COR$, NHCOR, $CONR^1R$, CONHR, wherein R is a hydrocarbyl group, or a linking group to a second N-oxide derivative of an opioid antagonist or a salt thereof, when the dashed bond is absent and $R^4$ is selected from H, CONHR, $CONR^1R$, NHCOR, $NR^1COR$ and $C(OH)(R^1)_2$, wherein R is a hydrocarbyl group; or
$R^3$ and $R^4$, together with the carbon atoms to which they are attached form a heterocycle or a linking group to a second N-oxide derivative of an opioid antagonist or a salt thereof;
$R^5$ is selected from OH, $OC_{1-8}$ alkyl, $OCOR^1$, H and $CONH_2$;
$R^6$ and $R^7$ are each independently selected from H, OH and $OC_{1-8}$ alkyl or $R^6$ and $R^7$, together with the carbon atoms to which they are attached, form a dihydrofuran ring; and
each dashed bond in the cyclohexyl ring may be present or absent with the proviso that both may not be present.

Viewed from a further aspect, the present invention provides a pharmaceutical composition comprising a N-oxide derivative of an opioid antagonist of formula (I), or a salt thereof, an opioid agonist or salt thereof and an adhesive and/or a matrix forming polymer.

Viewed from a further aspect, the present invention provides a method of making a composition as hereinbefore defined, comprising mixing an N-oxide derivative of an opioid antagonist of formula (I) as hereinbefore described, or a salt thereof, an opioid agonist, or a salt thereof and an adhesive and/or a matrix forming polymer.

Viewed from a further aspect, the present invention provides a transdermal dosage form comprising a composition as hereinbefore described.

Viewed from a further aspect, the present invention provides a composition as hereinbefore defined for use in medicine.

Viewed from a further aspect, the present invention provides a composition as hereinbefore defined for use in the treatment of pain.

DEFINITIONS

As used herein the terms "tamper resistant" and "abuse deterrent" are interchangeable, unless otherwise

specified.

As used herein, the term "N-oxide" refers to an amine oxide or amine N-oxide. N-oxides comprise a N-O bond, along with three additional hydrogen and/or hydrocarbyl chains attached to the nitrogen.

As used herein, the term "antagonist" refers to compounds which bind to opioid receptors and thereby block agonists from binding to the receptors. As used herein the term encompasses full antagonists which do not activate the receptors at all as well as partial antagonists which produce a weak opioid agonist effect.

As used herein, the term "opioid antagonist" refers to a compound which comprises the core structure (A) wherein R is a hydrocarbyl group:

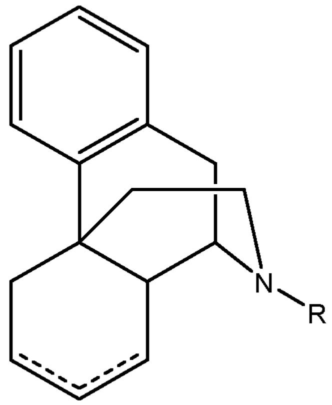

(A)

Preferred opioid antagonists comprise the core structure (B) wherein R is a hydrocarbyl group:

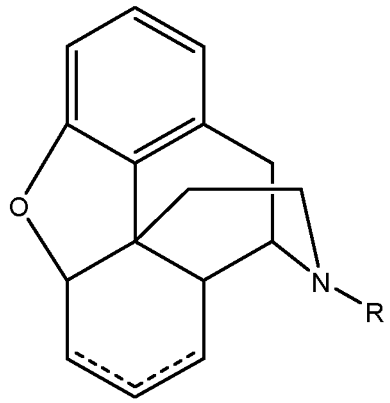

(B)

As used herein, the term "opioid agonist" refers to any natural, semi-synthetic or synthetic compound which binds to opioid receptors and activates the receptors to induce effects such as pain relief and sedation.

As used herein the term "alkyl" refers to saturated, straight chained, branched or cyclic groups. Alkyl groups may be substituted or unsubstituted.

As used herein the term "alkenyl" refers to straight chained, branched or cyclic group comprising a double bond. Alkenyl groups may be substituted or unsubstituted.

As used herein, the term "cycloalkyl" refers to a saturated or partially saturated mono- or bicyclic alkyl ring system containing 3 to 10 carbon atoms. Cycloalkyl groups may be substituted or unsubstituted.

As used herein, the term "aryl" refers to a group comprising at least one aromatic ring. The term aryl encompasses heteroaryl as well as fused ring systems wherein one or more aromatic ring is fused to a cycloalkyl ring. Aryl groups may be substituted or unsubstituted.

As used herein the term "hydrocarbyl" refers a univalent radical derived from a hydrocarbon group. A "hydrocarbon group" is a group which comprises carbon, hydrogen and optionally other atoms, e.g. halo atoms.

As used herein the term "halo" encompasses atoms selected from the group consisting of F, Cl, Br and I.

As used herein the term "heterocycle" refers to a cyclic group comprising at least one heteroatom selected from N, O and S. As used herein the term encompasses both aromatic and non-aromatic cyclic groups. Heterocycle groups may be substituted or unsubstituted.

As used herein the term "linking group" refers to a collection of atoms and/or bonds that connect together two or more other groups.

As used herein the term "transdermal delivery" refers to administration of compounds, e.g. opioid agonists, through the skin surface of an individual so that the agent passes through the skin tissue and into the individual's blood stream.

As used herein the term "transdermal delivery device" means any device that when contacted with a patient's skin, can transdermally deliver an analgesically effective amount of an opioid, or a pharmaceutically acceptable salt thereof, through the skin to the systemic circulation. The term "transdermal" is intended to include transmucosal administration, i.e., administration of the compound to the mucosal (e.g., sublingual, buccal, vaginal, rectal) surface of an individual so that it passes through the mucosal tissue and into the individual's blood stream.

As used herein the term "treatment of pain" encompasses the amelioration of pain or the cessation of pain in a patient.

As used herein the term "prevention of pain" encompasses the avoidance of the onset of pain in a patient.

## DETAILED DESCRIPTION OF INVENTION

The present invention relates to a pharmaceutical composition comprising a N-oxide derivative of an opioid antagonist or a salt thereof and an opioid agonist or salt thereof. The composition is designed for use in a transdermal delivery device. When the device is used as intended, i.e. for delivery of opioid agonists transdermally through the skin, the opioid agonist present in the pharmaceutical composition is delivered in the same way as if the N-oxide antagonist were not present to, for example, provide pain relief. Critically the N-oxide derivative of the opioid antagonist is not simultaneously delivered through the skin at all, or to any significant extent. This is because of the presence of the charged N-O bond in the N-oxide derivative which severely limits or prevents its passage through the skin. Thus, in normal operation, a transdermal delivery device comprising a composition of the present invention will solely provide opioid agonist and thereby pain relief.

If, however, the composition is removed or extracted from the transdermal delivery device for administration via a non-transdermal route, e.g. by oral or parenteral administration, then the N-oxide derivative of the opioid antagonist as well as the opioid agonist will be delivered into the blood stream. In the blood stream, the N-oxide derivative of the opioid antagonist is rapidly converted into the opioid antagonist and it antagonises the effect of the coadministered opioid agonist. In effect the opioid antagonist will inhibit the euphoric effect which would otherwise be achieved by administration of the opioid agonist. The composition of the present invention therefore provides an internal tamper resistance mechanism.

N-oxide derivatives of opioid antagonists are known compounds. US4722928, for example, discloses N-oxide derivatives of various compounds including narcotic antagonists. Naltrexone N-oxide and naloxone N-oxide are both specifically disclosed. US4722928 teaches that the oral bioavailabilty of N-oxide derivatives is better than that of the corresponding amino compound.

CH683005 also discloses N-oxide derivatives of various drugs including some opioid antagonists. CH683005 seems to teach that N-oxide opioid derivatives are stable in the blood and are present in excretion products.

Neither US4722928 nor CH683005 discloses the use of N-oxide derivatives of opioid antagonists in a transdermal delivery device. Moreover neither US4722928 nor CH683005 discloses the use of N-oxide derivatives of opioid antagonists as an abuse deterrent. Rather in both documents the N-oxide derivative is used as a prodrug.

In preferred compositions and transdermal delivery devices of the invention the N-oxide derivative is present in an amount sufficient to inhibit the euphoric effect of the opioid agonist. The N-oxide derivative of the opioid antagonist may completely or partially inhibit the euphoric effect of the opioid agonist, but preferably completely inhibits the euphoric effect. The amount of N-oxide derivative of opioid antagonist required to provide inhibition will depend on various factors including the identity of the opioid agonist, the amount of opioid agonist present and the identity of the opioid antagonist.

The N-oxide derivative of the opioid antagonist present in the composition and transdermal delivery device of the present invention comprises a core structure (A) wherein R is a hydrocarbyl group:

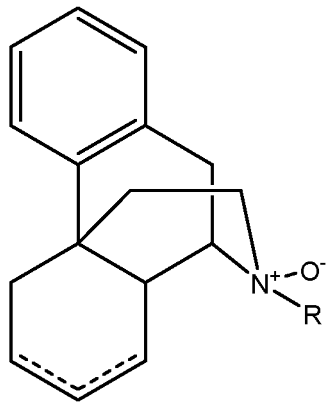

(A)

The N-oxide derivative of the opioid antagonist present in the composition and transdermal delivery device of the present invention comprises the core structure (B) wherein R is a hydrocarbyl group:

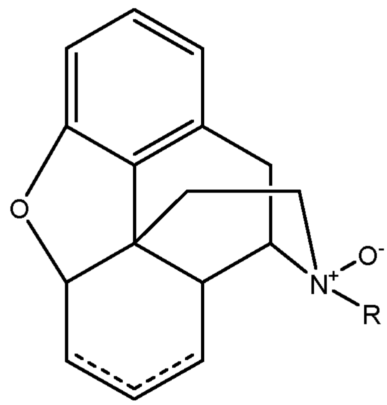

(B)

The composition and transdermal delivery device of the present invention comprises a N-oxide derivative of formula (I), or a salt thereof:

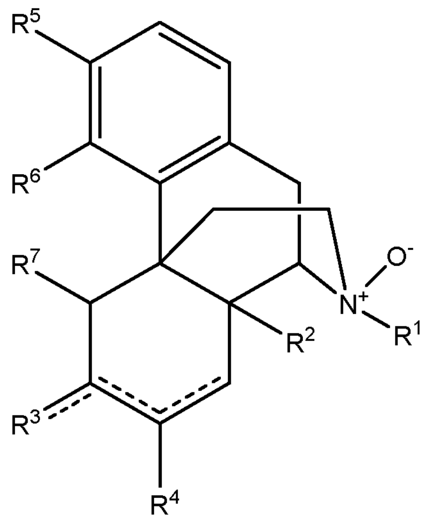

(I)

wherein

$R^1$ is selected from optionally substituted $C_{1-8}$ alkyl and optionally substituted $C_{2-8}$ alkenyl;
$R^2$ is selected from OH, H, $OC_{1-8}$ alkyl, NHCOR, $NR^1COR$, $CONR^1R$ and CONHR wherein R is a hydrocarbyl group

or $R^2$ forms a bridge to the carbon to which $R^3$ is attached;

$R^3$ is selected from O, $CH_2$, N-$NH_2$ or a linking group to a second N-oxide derivative of an opioid antagonist or a salt thereof when the dashed bond is present and is selected from OH, H, $OC_{1-8}$ alkyl, $OCOR^1$, $O(CH_2CH_2(CH_2)_yO)_xCH_3$ wherein x is 1-10 and y is 0-3, $N(R^1)_2$, $NR^1COR$, NHCOR, $CONR^1R$, CONHR, wherein R is a hydrocarbyl group, or a linking group to a second N-oxide derivative of an opioid antagonist or a salt thereof, when the dashed bond is absent and $R^4$ is selected from H, CONHR, $CONR^1R$, NHCOR, $NR^1COR$ and $C(OH)(R^1)_2$, wherein R is a hydrocarbyl group; or

$R^3$ and $R^4$, together with the carbon atoms to which they are attached form a heterocycle or a linking group to a second N-oxide derivative of an opioid antagonist or a salt thereof;

$R^5$ is selected from OH, $OC_{1-8}$ alkyl, $OCOR^1$, H and $CONH_2$;

$R^6$ and $R^7$ are each independently selected from H, OH and $OC_{1-8}$ alkyl or $R^6$ and $R^7$, together with the carbon atoms to which they are attached, form a dihydrofuran ring; and

each dashed bond in the cyclohexyl ring may be present or absent with the proviso that both may not be present.

Yet more preferably the composition and transdermal delivery device of the present invention comprises a N-oxide derivative of formula (II):

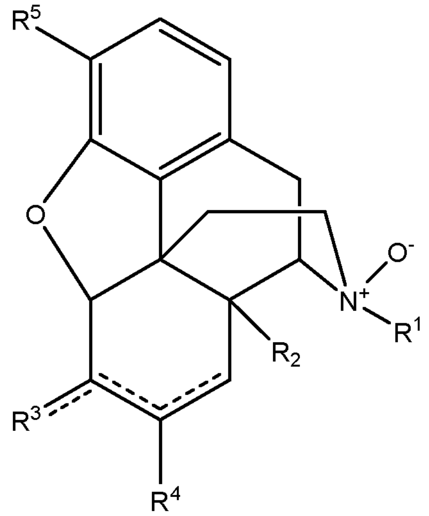

(II)

wherein each of $R^1$, $R^2$, $R^3$, $R^4$, $R^5$ and the dashed bonds are as hereinbefore defined.

As shown above in formulae (I) and (II), the cyclohexyl ring present in the N-oxide derivatives of opioid antagonists present in the composition and transdermal delivery device of the invention may be saturated or partially unsaturated. Preferably the cyclohexyl ring is saturated. Preferably therefore the N-oxide derivative of an opioid antagonist that is present in the composition and transdermal delivery device of the invention is of formula (IIIa):

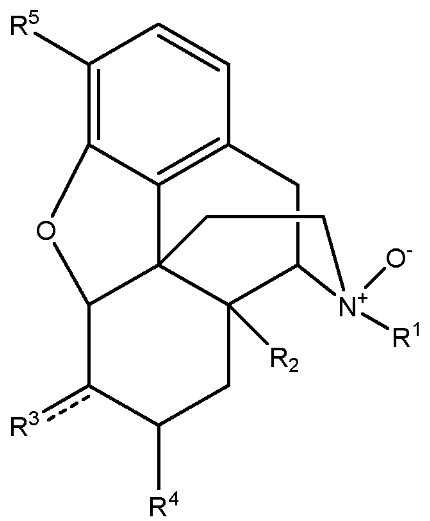

(IIIa)

wherein each of $R^1$, $R^2$, $R^3$, $R^4$ and $R^5$ are as hereinbefore defined.

If, however, the cyclohexyl ring is partially unsaturated, then preferably the N-oxide derivative of the opioid antagonist is of formula (IIIb) or (IIIc):

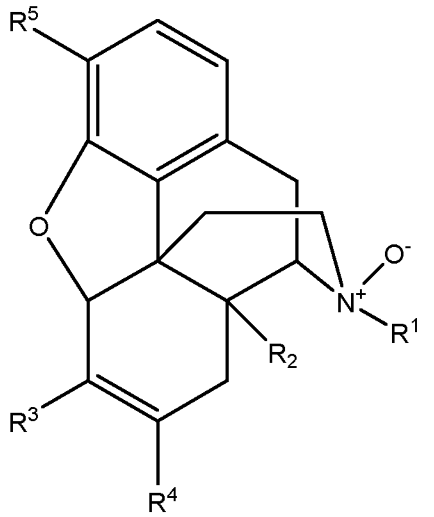

(IIIb)

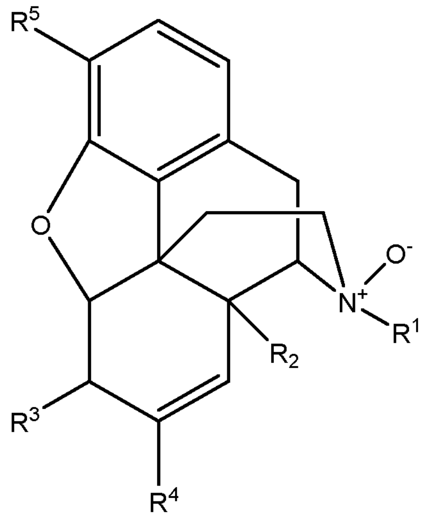

(IIIc)

wherein each of $R^1$, $R^2$ and $R^5$ are as hereinbefore defined; and $R^3$ is selected from OH, H, $C_{1-8}$ alkyl, $OCOR^1$, $O(CH_2CH_2(CH_2)_yO)_xCH_3$ wherein x is 1-10 and y is 0 to 3, $N(R^1)_2$, $NR^1COR$, NHCOR, $CONR^1R$, CONHR, wherein R is a hydrocarbyl group, or a linking group to a second N-oxide derivative of an opioid antagonist or a salt thereof; $R^4$ is selected from H, CONHR, $CONR^1R$, NHCOR, $NR^1COR$ and $C(OH)(R^1)_2$, wherein R is a hydrocarbyl group; or $R^3$ and $R^4$, together with the carbon atoms to which they are attached form a heterocycle or a linking group to a second N-oxide derivative of an opioid antagonist or a salt thereof.

The N-oxide derivative of an opioid antagonist present in the composition and transdermal delivery device of the invention may be a monomer or a dimer. Preferably the N-oxide derivative is a monomer and particularly preferably a monomer of formulae (I)-(III) and (V)-(VII) as herein defined. It can in some instances, however, be preferable for the N-oxide derivative to be a dimer. Dimers provide two molecules of antagonist per mole of compound which may be beneficial for use with strong opioid agonists. Additionally dimers have a relatively high molecular weight which means that they are even more resistant to transdermal delivery than their corresponding monomeric N-oxide compounds. Preferred dimeric N-oxide derivatives of opioid antagonists that are present in the compositions and transdermal delivery device of the invention are those of formula (IV):

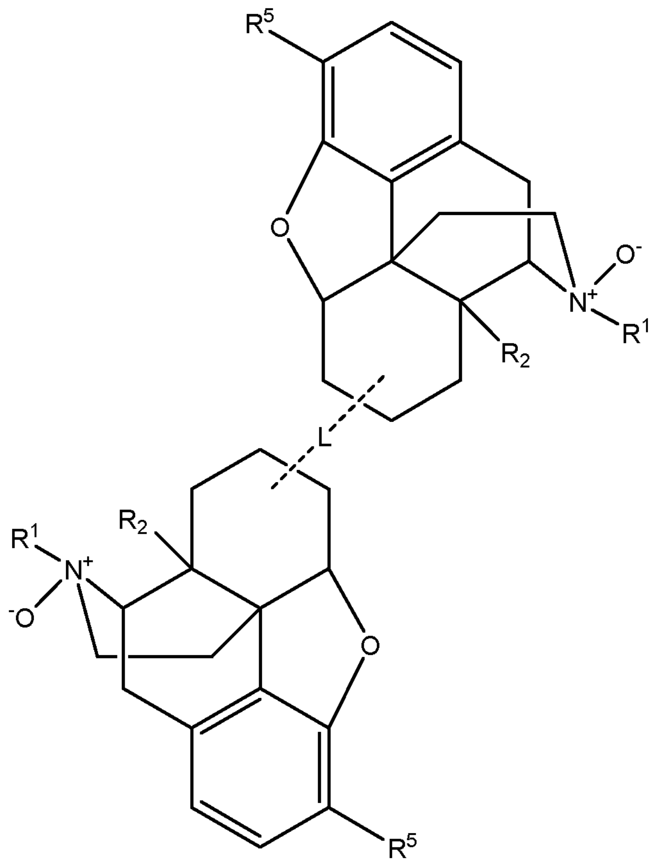

(IV)

wherein L is a linking group; and

each $R^1$, $R^2$ and $R^5$ are independently are as hereinbefore defined. Preferred linking groups include heterocycles. In this case, the heterocycle is preferably fused to both N-oxide derivatives as shown in formula (IVb) wherein H denotes heterocycle:

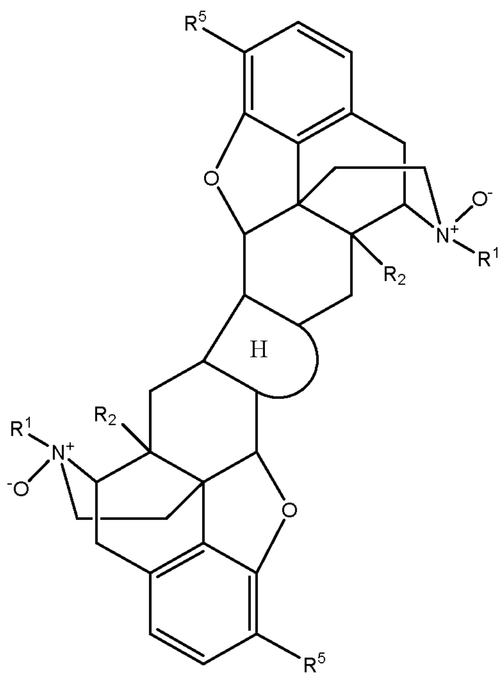

(IVb)

Preferred heterocycles comprise 5 or 6, and particularly 5, atoms including the atoms deriving from the two

fused cyclohexyl rings of the N-oxide derivatives. Further preferred heterocycles comprise at least one, e.g. one, nitrogen atom. Suitable heterocycles include furan, thiophene, pyrrole, pyrroline, oxazole, thiazole, imidazole, imidazolidine, pyrazole, pyrazoline, isoxazole and isothiazole. A preferred heterocycle linking group is pyrrole. Particularly preferred dimeric N-oxide derivatives of opioid antagonists that are present in the compositions and transdermal delivery devices of the invention are those of formula (IVc):

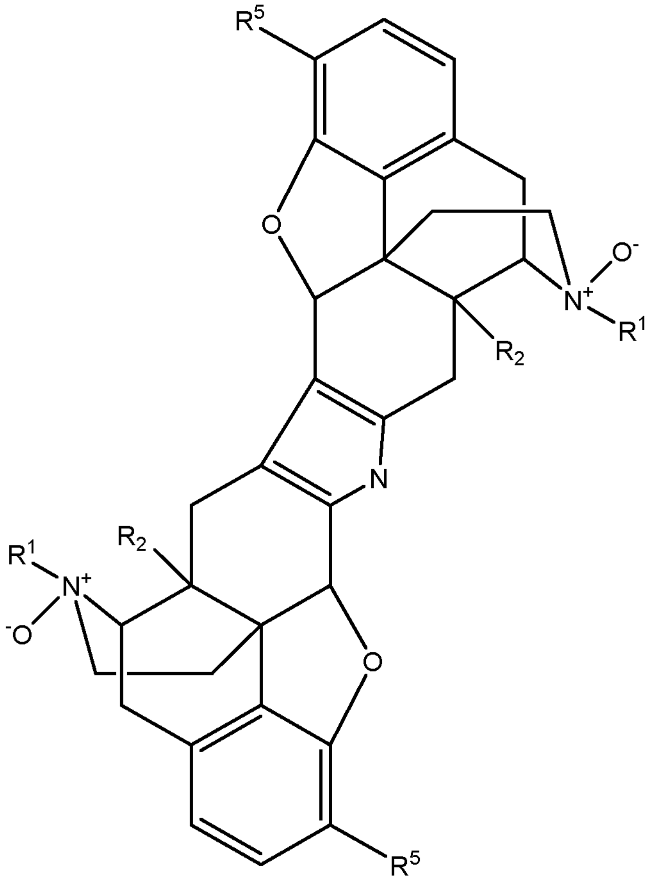

(IVc)

wherein each of $R^1$, $R^2$ and $R^5$ are as hereinbefore defined.

Particularly preferred compositions and transdermal delivery devices of the invention comprise a N-oxide derivative of formula (V):

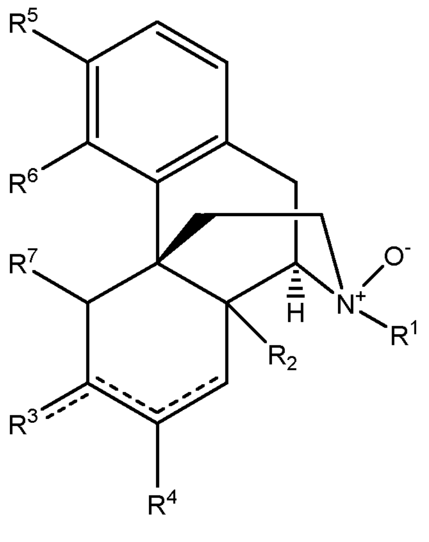

(V)

wherein each of $R^1$, $R^2$, $R^3$, $R^4$, $R^5$, $R^6$, $R^7$ and the dashed bonds are as hereinbefore defined.

Still further preferred compositions and transdermal delivery devices of the invention comprise a N-oxide derivative of formula (VI):

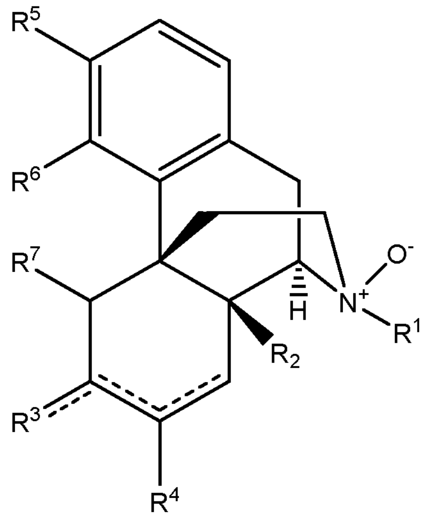

(VI)

wherein each of $R^1$, $R^2$, $R^3$, $R^4$, $R^5$, $R^6$, $R^7$ and the dashed bonds are as hereinbefore defined.

Yet further preferred compositions and transdermal delivery devices of the invention comprise a N-oxide derivative of formula (VII):

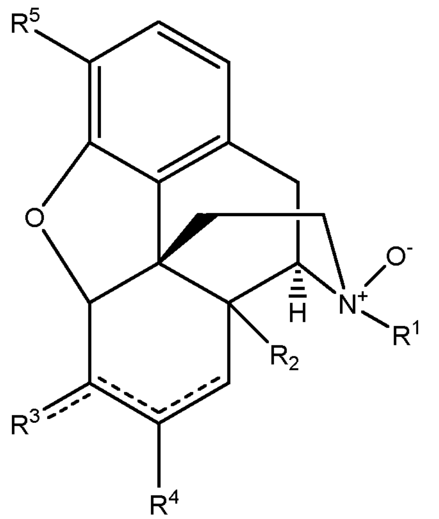

(VII)

wherein each of $R^1$, $R^2$, $R^3$, $R^4$, $R^5$ and the dashed bonds are as hereinbefore defined.

Still further preferred compositions and transdermal delivery devices of the invention comprise a N-oxide derivative of formula (VIII):

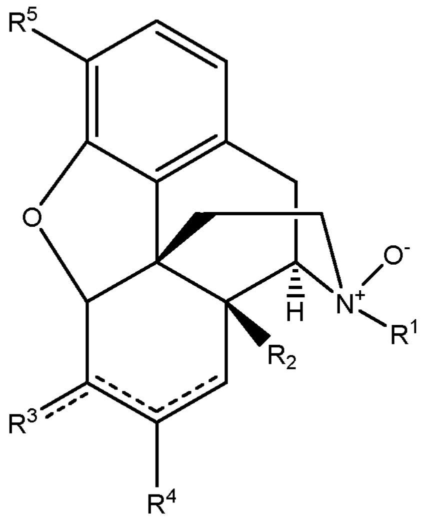

(VIII)

wherein each of $R^1$, $R^2$, $R^3$, $R^4$, $R^5$ and the dashed bonds are as hereinbefore defined.

Still further preferred compositions and transdermal delivery devices of the invention comprise a N-oxide derivative of formula (IX):

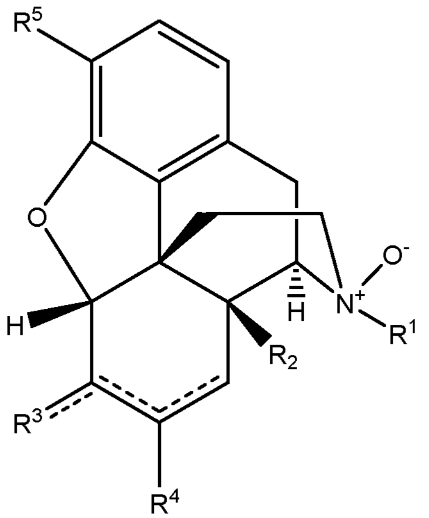

(IX)

wherein each of $R^1$, $R^2$, $R^3$, $R^4$, $R^5$ and the dashed bonds are as hereinbefore defined.

Yet further preferred compositions and transdermal delivery devices of the invention comprise a N-oxide derivative of formula (X):

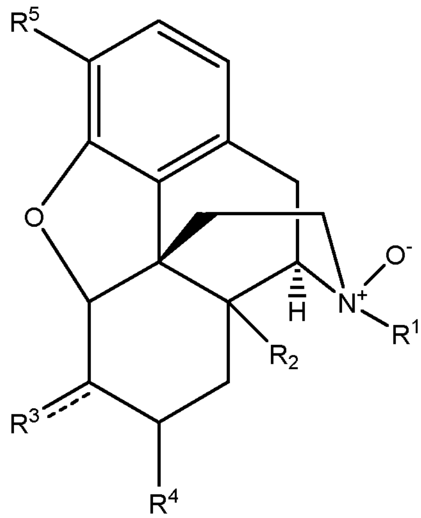

(X)

wherein each of $R^1$, $R^2$, $R^3$, $R^4$, and $R^5$ are as hereinbefore defined.

Yet further preferred compositions and transdermal delivery devices of the invention comprise a N-oxide derivative of formula (XI):

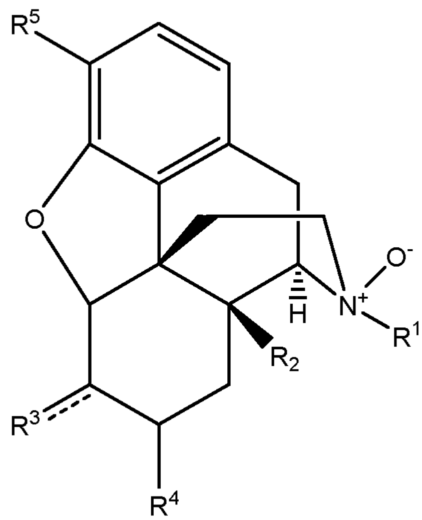

(XI)

wherein each of $R^1$, $R^2$, $R^3$, $R^4$, and $R^5$ are as hereinbefore defined.

Yet further preferred compositions and transdermal delivery devices of the invention comprise a N-oxide derivative of formula (XII):

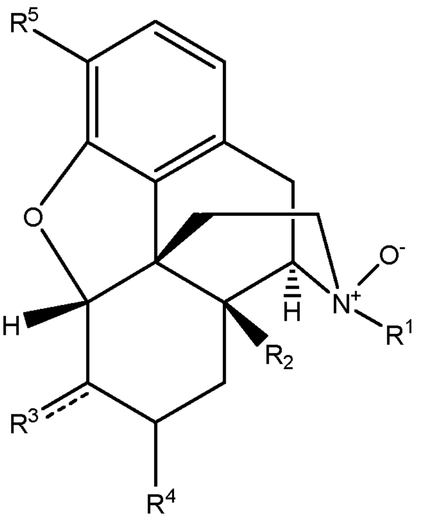

(XII)

wherein each of $R^1$, $R^2$, $R^3$, $R^4$ and $R^5$ are as hereinbefore defined.

In preferred compounds of formulae (I)-(XII), $R^5$ is selected from OH or $OC_{1-8}$ alkyl and particularly preferably OH.

In preferred compounds of formulae (I)-(XII), $R^2$ is selected from OH, H, $OC_{1-8}$ alkyl or $R^2$ forms a bridge to the carbon to which $R^3$ is attached. When $R^2$ forms a bridge, preferably an $-CH_2CH_2-$ bridge is formed. More preferably, however, $R^2$ is OH, H, $OC_{1-8}$ alkyl and still more preferably $R^2$ is OH.

In further preferred compounds of formulae (I)-(XII), $R^1$ is selected from substituted $C_{1-8}$ alkyl or unsubstituted $C_{2-8}$ alkenyl. Preferred alkyl groups are $C_{1-3}$ alkyl, e.g. methyl, ethyl or propyl and particularly methyl. Preferred substituents present on $R^1$ groups, and particularly $C_{1-8}$ alkyl groups, include $C_{3-8}$ cycloalkyl, $C_{6-12}$ aryl and halo (e.g. Cl, Br and F). Particularly preferred substituents are $C_{3-8}$ cycloalkyl and halo and especially $C_{3-8}$ cycloalkyl. Preferred cycloalkyl substituting groups are cyclopropyl and cyclobutyl, and particularly cyclopropyl. A particularly preferred $R^1$ group is $-CH_2$-cyclopropyl.

Preferred $C_{2-8}$ alkenyl groups are $C_{2-4}$ alkenyl, more preferably $C_2$ or $C_3$ alkenyl and still more preferably $C_3$ alkenyl. A further particularly preferred $R^1$ group is propen-1-yl, i.e. $-CH_2CH=CH_2$. Another preferred $R^1$ group is $-CH_2CH=C(CH_3)_2$.

In preferred compounds of formulae (I)-(III) and (V)-(XII) $R^4$ is selected from H or $C(OH)(R^1)_2$ or $R^3$ and $R^4$, together with the carbon atoms to which they are attached form a heterocycle. When $R^4$ is $C(OH)(R^1)_2$ each $R^1$ may be the same or different. Each $R^1$ is preferably $C_{1-8}$ alkyl and still more preferably methyl, ethyl, propyl or butyl. When $R^3$ and $R^4$, together with the carbon atoms to which they are attached form a heterocycle, the heterocycle is preferably optionally substituted indole (e.g. indole or 5'-guanidinoindole) or benzofuran. More preferably, however, $R^4$ is H.

In preferred compounds of formulae (I)-(III) and (V)-(XII), $R^3$ is selected from O, $CH_2$, $N-NH_2$ or a linking group to a second N-oxide derivative of an opioid antagonist or a salt thereof when the dashed bond is present and is selected from OH, H, $OC_{1-8}$ alkyl, $OCOR^1$, $O(CH_2CH_2(CH_2)yO)_xCH_3$ wherein x is 1-10 and y is 0-3, $N(R^1)_2$, $NR^1COR$, NHCOR, $CONR^1R$ and CONHR, wherein R is a hydrocarbyl group, or a linking group to a second N-oxide derivative of an opioid antagonist or a salt thereof, when the dashed bond is absent or $R^3$ and $R^4$, together with the carbon atoms to which they are attached form a heterocycle as set out above. In further preferred compounds of formulae (I)-(III) and (V)-(XII), $R^3$ is selected from O, $CH_2$, $N-NH_2$ when the dashed bond is present and is selected from OH, H, $OC_{1-8}$ alkyl, $OCOR^1$, $O(CH_2CH_2(CH_2)_y O)_xCH_3$ wherein x is 1-10 and y is 0-3, $N(R^1)_2$, $NR^1COR$, NHCOR, $CONR^1R$, CONHR, wherein R is a hydrocarbyl group, when the dashed bond is absent. In yet further preferred compounds of formulae (I)-(III) and (V)-(XII), $R^3$ is selected from O or $CH_2$, when the dashed bond is present, and is selected from OH or $OC_{1-8}$ alkyl, when the dashed bond is absent. In especially preferred compounds of formulae (I)-(III) and (V)-(XII), $R^3$ is O and the dashed bond is present.

In particularly preferred compositions and transdermal delivery devices of the invention, the N-oxide derivative is a derivative of an antagonist selected from Naloxone, Naloxol, Naloxegol, Naloxazone, Nalmefene, Nalbuphine, Nalmexone, Naltrexone, Naltrexol, Chlornaltrexamine, Clocinnamox, Nafurafine, Nalemedine, Naltrindole, 5'-Guanidinonaltrindole, Naltriben, Nalorphine, Diacetylnalorphine, Naloxonazine, Norbinaltorphimine, Binaltorphimine, Buprenorphine, Diprenorphine, Levellorphan, Cyprodime, Oxilorphan, Samidorphan, Cyclazocine, pharmaceutically acceptable salts thereof, and mixtures thereof. In still further preferred compositions and transdermal delivery devices of the invention, the N-oxide derivative is a derivative of an antagonist selected from Naloxone, Naloxol, Naloxegol, Naloxazone, Nalmefene, Naltrexone, Naltrexol, Chlornaltrexamine, Clocinnamox, Nafurafine, Nalemedine, Naltrindole, 5'-Guanidinonaltrindole, Naltriben, Nalorphine, Diacetylnalorphine, Naloxonazine, Norbinaltorphimine, Binaltorphimine, Levellorphan, Cyprodime, Oxilorphan, Samidorphan, Cyclazocine, pharmaceutically acceptable salts thereof, and mixtures thereof. The systematic name of each of these compounds is shown in the table below. The structure of each of these compounds is shown in Figure 1.

| | |
|---|---|
| Naloxone | (4R,4aS,7aR,12bS)-4a,9-dihydroxy-3-prop-2-enyl-2,4,5,6,7a,13-hexahydro-1H-4,12-methanobenzofuro[3,2-e]isoquinoline-7-one |
| 6-alpha naloxol | (4R,4aS,7S,7aR,12bS)-3-prop-2-enyl-1,2,4,5,6,7,7a,13-octahydro-4,12-methanobenzofuro[3,2-e]isoquinoline-4a,7,9-triol |
| Naloxegol | (4R,4aS,7S,7aR,12bS)-7-[2-[2-[2-[2-[2-[2-(2-methoxyethoxy)ethoxy]ethoxy]ethoxy]ethoxy]ethoxy]ethoxy]-3-prop-2-enyl-1,2,4,5,6,7,7a,13-octahydro-4,12-methanobenzofuro[3,2-e]isoquinoline-4a,9-diol |
| Naloxazone | (4R,4aS,7Z,7aR,12bS)-7-hydrazinylidene-3-prop-2-enyl-2,4,5,6,7a,13-hexahydro-1H-4,12-methanobenzofuro[3,2-e]isoquinoline-4a,9-diol |
| Nalmefene | (4R,4aS,7aS,12bS)-3-(cyclopropylmethyl)-7-methylidene-2,4,5,6,7a,13-hexahydro-1H-4,12-methanobenzofuro[3,2-e]isoquinoline-4a,9-diol |
| Nalbuphine | (4R,4aS,7S,7aR,12bS)-3-(cyclobutylmethyl)-1,2,4,5,6,7,7a,13-octahydro-4,12-methanobenzofuro[3,2-e]isoquinoline-4a,7,9-triol |
| Nalmexone | (4R,4aS,7aR,12bS)-4a,9-dihydroxy-3-(3-methylbut-2-enyl)-2,4,5,6,7a,13-hexahydro-1H-4,12-methanobenzofuro[3,2-e]isoquinoline-7-one |
| Naltrexone | (4R,4aS,7aR,12bS)-3-(cyclopropylmethyl)-4a,9-dihydroxy-2,4,5,6,7a,13-hexahydro-1H-4,12-methanobenzofuro[3,2-e]isoquinoline-7-one |
| 6-beta naltrexol | 3-(cyclopropylmethyl)-1,2,4,5,6,7,7a,13-octahydro-4,12-methanobenzofuro[3,2-e]isoquinoline-4a,7,9-triol |
| Chlornaltrexamine | (4R,4aS,7R,7aR,12bS)-7-[bis(2-chloroethyl)amino]-3-(cyclopropylmethyl)-1,2,4,5,6,7,7a,13-octahydro-4,12-methanobenzofuro[3,2-e]isoquinoline-4a,9-diol |
| Clocinnamox | (E)-N-[(4R,4aS,7aR,12bR)-3-(cyclopropylmethyl)-9-hydroxy-7-oxo-2,4,5,6,7a,13-hexahydro-1H-4,12-methanobenzofuro[3,2-e]isoquinoline-4a-yl]-3-(4-chlorophenyl)prop-2-enamide |
| Nalfurafine | (E)-N-[(4R,4aS,7R,7aR,12bS)-3-(cyclopropylmethyl)-4a,9-dihydroxy-1,2,4,5,6,7,7a,13-octahydro-4,12-methanobenzofuro[3,2-e]isoquinoline-7-yl]-3-(furan-3-yl)-N-methylprop-2-enamide |
| Naldemedine | (4R,4aS,7aR,12bS)-3-(cyclopropylmethyl)-4a,7,9-trihydroxy-N-[2-(3-phenyl-1,2,4-oxadiazol-5-yl)propan-2-yl]-1,2,4,5,7a,13-hexahydro-4,12-methanobenzofuro[3,2-e]isoquinoline-6-carboxamide |
| Naltrindole | 17-Cyclopropylmethyl-6,7-dehydro-4,5-epoxy-3,14-dihydroxy-6,7,2',3'-indolomorphinan |
| 5'-Guanidinonaltrindole | 5'-Guanidinyl-17-(cyclopropylmethyl)-6,7-dehydro-4,5a-epoxy-3, 14-dihydroxy-6,7-2',3'-indolomorphinan |
| Naltriben | 4,8-Methano-5H-bisbenzofuro(3,2-e:2',3'-g)isoquinoline-1,8a(9H)-diol, 7-(cyclopropylmethyl)-6,7,8,14b-tetrahydro-, (8R-(4bS*,8alpha,8beta,14bbeta))- |
| Nalporphine | (4R,4aR,7S,7aR,12bS)-3-prop-2-enyl-2,4,4a,7,7a,13-hexahydro-1H-4,12-methanobenzofuro[3,2-e]isoquinoline-7,9-diol |
| Diacetylnalorphine | [(4R,4aR,7S,7aR, 12bS)-9-acetyloxy-3-prop-2-enyl-2,4,4a,7,7a,13-hexahydro-1H-4,12-methanobenzofuro[3,2-e]isoquinoline-7-yl] acetate |
| Naloxonazine | (4R,4aS,7E,7aR,12bS)-7-[(E)-[(4R,4aS,7aR,12bS)-4a,9-dihydroxy-3-prop-2-enyl-2,4,5,6,7a,13-hexahydro-1H-4,12-methanobenzofuro[3,2-e]isoquinoline-7-ylidene]hydrazinylidene]-3-prop-2-enyl-2,4,5,6,7a,13-hexahydro-1H-4,12-methanobenzofuro[3,2-e]isoquinoline-4a,9-diol |
| Norbinaltorphimine | 17,17'-(dicyclopropylmethyl)-6,6',7,7'-6,6'-imino-7,7'-bimorphinan-3,4',14,14'-tetrol |

(continued)

| | |
|---|---|
| Binaltorphimine | (1*S*,2*S*,7*S*,8*S*, 12*R*,20*R*,24*R*,32*R*)-11,33-bis(cyclopropylmethyl)-22-methyl-19,25-dioxa-11,22,33-triazaundecacyclo[24.9.1.$1^{8,14}$.$0^{1,24}$.$0^{2,32}$.$0^{4,23}$.$0^{5,21}$.$0^{7,12}$.$0^{8,20}$.$0^{18,37}$.$0^{30,36}$] heptatriaconta-4(23),5(21),14(37),15,17,26(36),27,29-octaene-2,7,17,27-tetrol |
| Buprenorphine | (5α,6β,14β,18R)-17-(Cyclopropylmethyl)-18-[(2S)-2-hydroxy-3,3-dimethyl-2-butanyl]-6-methoxy-18,19-dihydro-4,5-epoxy-6,14-ethenomorphinan-3-ol |
| Diprenorphine | (5a,7a)-17-(Cyclopropylmethyl)- 4,5-epoxy-18,19-dihydro-3-hydroxy-6-methoxy-α,α-dimethyl-6,14-ethenomorphinan-7-methanol |
| Levallorphan | 17-Allylmorphinan-3-ol |
| Cyprodime | 17-(Cyclopropylmethyl)-4,14-dimethoxymorphinan-6-one |
| Oxilorphan | 17-(Cyclopropylmethyl)morphinan-3,14-diol |
| Samidorphan | 17-(Cyclopropylmethyl)-4,14-dihydroxy-6-oxomorphinan-3-carboxamide |
| Cyclazocine | 3-(Cyclopropylmethyl)-6,11-dimethyl-1,2,3,4,5,6-hexahydro-2,6-methano-3-benzazocin-8-ol |

In further preferred compositions and transdermal delivery devices of the invention the N-oxide derivative is a derivative of an antagonist selected from Naloxone, Naloxol, Naloxegol, Naloxazone, Nalmefene, Nalbuphine, Nalmexone, Naltrexone, Naltrexol, Chlornaltrexamine, Nafurafine, Naltriben, Nalorphine, Diacetylnalorphine, Levellorphan, Cyprodime, Oxilorphan, Samidorphan, pharmaceutically acceptable salts thereof, and mixtures thereof. In still further preferred compositions and transdermal delivery devices of the invention the N-oxide derivative is a derivative of an antagonist selected from Naloxone, Naloxol, Naloxegol, Naloxazone, Nalmefene, Nalbuphine, Nalmexone, Naltrexone, Naltrexol, Chlornaltrexamine, Nafurafine, Naltriben, Nalorphine, Diacetylnalorphine, pharmaceutically acceptable salts thereof, and mixtures thereof. In yet further preferred compositions and transdermal delivery devices of the invention, the N-oxide derivative is a derivative of an antagonist selected from Naloxone, Naltrexone, pharmaceutically acceptable salts thereof, and mixtures thereof. Any pharmaceutically acceptable salt that may be formed is included herein.

The compositions and transdermal delivery devices of the present invention also comprise an opioid agonist or a salt thereof. Preferably the opioid agonist is selected from alfentanil, allylprodine, alphaprodine, anileridine, benzylmorphine, bezitramide, buprenorphine, butorphanol, carfentanil, clonitazene, codeine, desomorphine, dextromoramide, dezocine, diampromide, diamorphone, dihydrocodeine, dihydromorphine, dihydromorphone, dihydroisomorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl, heroin, hydrocodone, hydromorphone, hydromorphodone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, pantopon, papaveretum, paregoric, pentazocine, phenadoxone, phendimetrazine, phendimetrazone, phenomorphan, phenazocine, phenoperidine, piminodine, piritramide, propheptazine, promedol, properidine, propoxyphene, propylhexedrine, sufentanil, tilidine, tramadol, pharmaceutically acceptable salts thereof, and mixtures thereof.

In preferred compositions and transdermal delivery devices of the invention the opioid agonist is selected from alfentanil, sufentanil, etorphine, dihydroetorphine, hydrocodone, morphine, hydromorphone, oxycodone, carfentanil, codeine, levorphanol, meperidine, methadone, oxymorphone, buprenorphine, fentanyl, dipipanone, heroin, tramadol, etorphine, dihydroetorphine, butorphanol, levorphanol, pharmaceutically acceptable salts thereof, and mixtures thereof. In still further preferred compositions and transdermal delivery devices of the invention the opioid agonist is selected from alfentanil, fentanyl, sufentanil, etorphine, dihydroetorphine, buprenorphine, pharmaceutically acceptable salts thereof, and mixtures thereof.

In preferred compositions, and particularly those designed for use in passive transdermal delivery devices, the opioid agonist is a free-base opioid, i.e. is not a pharmaceutically acceptable salt of the opioid. When opioid agonist salts are desired, however, any pharmaceutically acceptable salt may be employed. Preferred salts are those that retain the biological effectiveness and properties of the compounds of the present invention and are formed from suitable non-toxic organic or inorganic acids.

Salts of opioid agonists may be formed from an acid and the basic nitrogen group of an opioid. Suitable salts include sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (i.e. 1,1'-methylene-bis-(2-hydroxy-3-naphthoate)) salts. Salts of opioid agonists may also be formed from an opioid having an acidic functional group, such as a carboxylic acid or sulfonic acid functional group, and a pharmaceutically acceptable inorganic or organic base. Suitable bases include hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl, N-ethylamine; diethylamine; triethylamine; mono-, bis-, or tris- (2-hydroxy-lower alkyl amines), such as mono-, bis-, or tris- (2-hydroxyethyl) amine, 2-hydroxy-tert-butylamine, or tris- (hydroxymethyl) methylamine, N, N,-di-lower alkyl-N-(hydroxy lower alkyl) -amines, such as N, N,-dimethyl-N- (2-hydroxyethyl) amine, or tri-(2-hydroxyethyl) amine; N-methyl-D-glucamine; and amino acids such as arginine and lysine.

The N-oxide derivatives of opioid antagonists and opioid agonists present in the compositions and transdermal delivery devices of the present invention may exist in optically active or racemic forms by virtue of one or more asymmetric carbons atoms. The invention includes all such optically active or racemic forms. It is also to be understood that certain compounds, intermediates and/or starting materials may exist in tautomeric forms and that the invention also relates to any and all tautomeric forms of the compounds and their use.

The N-oxide derivatives of opioid antagonists and opioid agonists present in the compositions and transdermal delivery devices of the present invention may also be provided in the form of a derivative. As used herein, a derivative is a compound that is degradable in the body to produce a compound of the invention. Examples of typical derivatives include esters. Suitable ester forming groups for the hydroxy group often present in the compounds of the invention include $C_{1-6}$ alkanoyl, $C_{1-6}$ alkoxycarbonyl and arylalkanoyl (e.g. benzoyl).

The skilled man will be able to determine suitable molar ratios of N-oxide derivative to opioid agonist in the compositions and transdermal delivery devices of the invention. The exact ratios employed will depend on numerous factors including the type of opioid agonist, the type of opioid antagonist present in the N-oxide compond, their relative potentcies and half lives. At the ratios used the amount of antagonist present will generally be enough to inhibit the euphoric effect of the opioid agonist if the composition, or the transdermal delivery device in which it is present, is abused.

The amount of each of the N-oxide derivative of an opioid antagonist and the opioid agonist in the composition and transdermal delivery device will depend on the specific opioid agonist and N-oxide opioid antagonist present, the type of device or dosage form in which the composition will be used, the materials used to manufacture the device or dosage form, and the duration for which the opioid agonist will be delivered to the patient. The skilled man will be able to determine suitable amounts of N-oxide derivative and opioid agonist. Although the opioid agonist and the N-oxide derivative of the opioid antagonist in the composition may be separate or mixed together, in preferred compositions they are mixed together.

Preferred compositions of the invention further comprise a pharmaceutically acceptable excipient. Any conventional pharmaceutically acceptable excipient may be used. The compositon of the invention is preferably a liquid (e.g. solution or dispersion) or a polymer matrix. The precise excipients used will generally depend on the type of transdermal delivery device the compositions are designed for use with. The compositions of the invention may, for example, include adhesive and/or matrix forming polymer.

The present invention also relates to a method of making a composition as hereinbefore described, comprising mixing an N-oxide derivative of an opioid antagonist of formula (I), or a salt thereof and an opioid agonist, or a salt thereof. Any mixer conventional in the art may be used. The compositions of the invention may be formulated in any conventional manner with one or more physiologically acceptable carriers or diluents, according to techniques well known in the art. Suitable excipients (e.g. carriers and diluents) and other materials that can be used in compositions of the invention are well known to those skilled in the pharmaceutical arts, and depend on the particular tissue to which a given composition will be applied.

As mentioned above, the composition of the present invention is suitable for incorporation into a transdermal delivery device. Thus a transdermal delivery device of the present invention comprises a composition as hereinbefore described. The composition of the invention is such that the opioid agonist is delivered and little or none of the N-oxide derivative of the opioid antagonist is delivered, via the transdermal delivery device, to the patient if the device is used transdermally as intended. If, however, the composition of the invention is administered non-transdermally, e.g. parenterally or orally, then preferably both the opioid agonist and the N-oxide derivative of the opioid antagonist are delivered to the blood stream of the patient. In this case the N-oxide derivative of the opioid antagonist is converted to the opioid antagonist per se and the opioid antagonist inhibits the euphoric effect that would otherwise be achieved by the opioid agonist. The conversion of the N-oxide derivative to the opioid antagonist per se is believed to be facilitated by endogenous reductase enzymes either in the liver and/or blood. These tamper resistance and abuse deterrent properties are a consequence of the composition per se.

Thus preferably the composition and delivery device of the present invention allows for the transdermal administration of the opioid agonist, or a pharmaceutically acceptable salt thereof, but either (a) allows for the transdermal administration of only an amount of the N-oxide derivative of the opioid antagonist that is ineffective for inhibiting the

analgesic effect of the opioid agonist, or (b) does not allow the transdermal administration of the N-oxide derivative of the opioid antagonist. On the other hand, however, if the composition or delivery device of the present invention is used to deliver the opioid agonist via a route other than transdermal, and in particular oral or parenteral, then the N-oxide derivative of the opioid antagonist inhibits or minimises the euphoric effect of the opioid agonist.

The composition of the present invention is tamper resistant and any transdermal delivery device or dosage form into which the composition is incorporated is also tamper resistant in that if an abuser attempts to administer the composition via any route other than transdermally, e.g. orally or parentrally, to achieve an euphoric effect the abuser would simultaneously self-administer the N-oxide derivative of the opioid antagonist along with the opioid agonist.

For example, if an abuser tries to extract the opioid agonist from the composition by placing it in a solvent, then the N-oxide derivative of the opioid antagonist would also be extracted. If a mixture of the opioid agonist and the N-oxide derivative of the opioid antagonist is subsequently administered via a route other than the intended transdermal route (e.g. by injection), then the N-oxide derivative of the opioid antagonist will convert in the blood stream to the opioid antagonist per se and inhibit the euphoric effect of the opioid.

If the composition of the present invention is administered parenterally, then both of the opioid agonist and the N-oxide derivative of the opioid antagonist enter the blood stream. Thereafter the N-oxide derivative of the opioid antagonist converts to the opioid antagonist per se and has an inhibitory action on the euphoric effect that would otherwise be achieved via administration of the opioid agonist.

A transdermal dosage form is a unit that provides transdermal delivery. The use of compounds of formula (I) in transdermal dosage forms is advantageous because these compounds provide a tamper resistance mechanism. Thus preferred compositions and transdermal dosage forms, e.g. transdermal delivery devices, of the invention are tamper resistant. Transdermal dosage forms of the invention include sprays, ointments, salves, aerosols, creams, lotions, ointments, gels, solutions, powders, emulsions, suspensions, or other forms known to one of skill in the art.

More preferably, however, the transdermal dosage form is a transdermal delivery device. Any device conventional in the art for transdermally delivering a therapeutic agent to a patient can be used for the transdermal delivery of the composition of the invention and as the transdermal delivery device. For example, the transdermal delivery device can be a reservoir-type transdermal delivery device, a polymer-matrix type transdermal delivery device, or a drug-in-adhesive type transdermal delivery device. The transdermal delivery device is designed so that when contacted with the patient's skin, the opioid agonist, e.g. in a therapeutically effective amount, is transdermally administered to the patient. In contrast the N-oxide derivative of the opioid antagonist either remains in the transdermal delivery device and is not administered to the patient or is administered to the patient in an amount insufficient to inhibit the analgesic effect of the opioid agonist.

A reservoir-type transdermal delivery device preferably comprises a reservoir, usually a liquid, located between an impermeable backing film and a rate-controlling membrane that is covered with a pressure-sensitive adhesive skin-contacting layer. The reservoir, which may be a solution or a dispersion, contains the composition of the invention. The transdermal delivery device is preferably supported by the impermeable backing film and the adhesive surface is protected by a release liner. To administer the opioid agonist, the release liner is removed to expose the pressure- sensitive adhesive and the pressure-sensitive adhesive is contacted with the skin. The opioid agonist is permeable through the rate-controlling membrane, and penetrates through it and the adhesive, contacts the skin, and then penetrates the skin. The delivery rate of the opioid agonist is usually determined by the rate that the opioid agonist penetrates the rate-controlling membrane. In contrast to the opioid agonist, the N-oxide derivative of the opioid antagonist does not penetrate the rate-controlling membrane and/or the skin to any significant extent due to the presence of the charged N-O bond.

A variation of the reservoir-type transdermal delivery device is the polymer-matrix design. In the polymer-matrix design, the opioid agonist and the N-oxide derivative of the opioid antagonist are dispersed in a polymer matrix that controls the delivery rate of the opioid agonist. Preferably the polymer-matrix reservoir is supported on an impermeable backing layer. Rather than having a continuous adhesive layer, however, the polymer-matrix design preferably includes a peripheral ring of adhesive located around the edge of the matrix. A release liner preferably protects the adhesive surface and the surface of the polymer matrix. To administer the opioid agonist the release liner is removed to expose the polymer matrix and the ring of pressure-sensitive adhesive, and the device is contacted with the skin. The ring of adhesive holds the device against the skin so that the polymer matrix directly contacts the skin. When the polymer matrix is contacted with the skin, the opioid agonist diffuses out of the polymer matrix, contacts the patient's skin, and penetrates the skin. The delivery rate of the opioid agonist is usually determined by the rate of diffusion of the opioid out of the polymer matrix. The N-oxide derivative of the opioid antagonist, which may be present anywhere in the polymer matrix, on the other hand, either does not diffuse out of the polymer matrix and/or into the skin or, if it does, does so in an amount insufficient to inhibit the analgesic effect of the opioid agonist.

The drug-in-adhesive type transdermal delivery device comprises the opioid agonist and the N-oxide derivative of the opioid antagonist dispersed directly in a pressure-sensitive adhesive matrix. The adhesive matrix is preferably supported on the topside with an impermeable backing film and on the side that faces the skin with an impermeable release liner. To administer the opioid agonist the release liner is removed to expose the adhesive matrix, and the device

is contacted with the skin. The adhesive matrix functions to adhere the device to the skin and, typically, to control the delivery rate of the opioid agonist. Similar to the polymer-matrix design, the drug-in-adhesive design allows the opioid agonist to diffuse out of the adhesive matrix, contact the patient's skin, and penetrate the skin. The delivery rate of the opioid agonist is usually determined by the rate of diffusion of the opioid agonist out of the adhesive matrix. The delivery rate is such that an analgesically effective amount of the opioid agonist is delivered to the patient. In contrast the N-oxide derivative of the opioid antagonist, which may be present anywhere in the adhesive matrix, does not diffuse out of the adhesive matrix and into the skin or does so in an amount insufficient to inhibit the analgesic effect of the opioid agonist.

Any rate-controlling membrane known to those skilled in the art can be used in the transdermal delivery device of the invention. Suitable materials for the rate-controlling membranes include polyethylene; polypropylene; ethylene/propylene copolymers; ethylene/ethylacrylate copolymers; ethylene/vinyl acetate copolymers; polyacrylates; polymethacrylates; silicone elastomers; medical-grade polydimethylsiloxanes; neoprene rubber; polyisobutylene; chlorinated polyethylene; polyvinyl chloride; vinyl chloride-vinyl acetate copolymer; polymethacrylate polymer (hydrogel); polyvinylidene chloride; poly (ethylene terephthalate); butyl rubber; epichlorohydrin rubbers; ethylene-vinyl alcohol copolymer; ethylene-vinyloxyethanol copolymer; silicone copolymers, for example polysiloxane-polycarbonate copolymers, polysiloxane-polyethyleneoxidecopolymers, polysiloxane-polymethacrylate copolymers, polysiloxane-alkylene copolymers (e.g. polysiloxane-ethylene copolymers), polysiloxane-alkylenesilane copolymers (e.g. polysiloxaneethylenesilane copolymers); cellulose polymers, for example, methyl or ethyl cellulose, hydroxypropyl methyl cellulose, and cellulose esters; polycarbonates; polytetrafluoroethylene; starches; gelatin; natural and synthetic gums; any other natural or synthetic polymer or fiber; and combinations thereof.

The backing layer can be any suitable material that is impermeable to the contents of the reservoir compartment, the polymer matrix, or the adhesive matrix. The backing layer preferably serves as a protective cover and may also provide a support function. Suitable materials for backing films are well known to those skilled in the art and include occlusive polymers such as polyurethane, polyesters such as poly (ethylene phthalate), polyether amide, copolyester, polyisobutylene, polyesters, high and low density polyethylene, polypropylene, polyvinylchloride, metal foils, and metal foil laminates of suitable polymer films.

The backing layer can be any appropriate thickness which will provide the desired protective and support functions. A suitable thickness will be from about 10 to about 200 microns. Desirable materials and thickness will be apparent to the skilled man.

The polymer matrix preferably comprises a compound of formula (I), an active agent (e.g. opioid agonist) and a polymer. The polymer functions to provide a matrix in which the compound of formula (I) and active agent, e.g. opioid agonist, can be homogeneously distributed. Generally, the polymers present in the drug layer are biologically acceptable polymers capable of forming thin walls or coatings through which pharmaceuticals can pass at a controlled rate. Suitable materials for the polymer matrix are well known to those skilled in the art and include polyethylene; polypropylene; ethylene/propylene copolymers; ethylene/ethylacrylate copolymers; ethylene/vinyl acetate copolymers; silicone elastomers, especially the medical-grade polydimethylsiloxanes; neoprene rubber; polyisobutylene; chlorinated polyethylene; polyvinyl chloride; vinyl chloride-vinyl acetate copolymer; polymethacrylate polymer (hydrogel); polyvinylidene chloride; poly (ethylene terephthalate); butyl rubber; epichlorohydrin rubbers; ethylene-vinyl alcohol copolymer; ethylene-vinyloxyethanol copolymer; silicone copolymers, for example, polysiloxane-polycarbonate copolymers, polysiloxane-polyethyleneoxide copolymers, polysiloxane-polymethacrylate copolymers, polysiloxane-alkylene copolymers (e.g. polysiloxane-ethylene copolymers), polysiloxane-alkylenesilane copolymers (e.g. polysiloxaneethylenesilane copolymers); cellulose polymers, for example methyl or ethyl cellulose, hydroxypropyl methyl cellulose, and cellulose esters; polycarbonates; polytetrafluoroethylene; and combinations thereof. In one embodiment, the polymer matrix has a glass transition temperature below room temperature. The polymer can, but need not necessarily, have a degree of crystallinity at room temperature.

Cross-linking monomeric units or sites can be incorporated into the polymers. For example, cross-linking monomers can be incorporated into polyacrylate polymers. The cross-linking monomers may, for example, provide sites for cross-linking the polymer matrix after dispersing the opioid agonist, and the N-oxide derivative of the opioid antagonist, into the polymer. Known cross-linking monomers for polyacrylate polymers include, for example, polymethacrylic esters of polyols such as butylene diacrylate, butylene dimethacrylate and trimethylol propane trimethacrylate. Other monomers that provide cross-linking sites include allyl acrylate, allyl methacrylate and diallyl maleate.

The polymer matrix can be any appropriate thickness which will provide the desired amount of compound of formula (I) and active agent, e.g. opioid agonist, and deliver the active agent transdermally at such a rate that a therapeutic effect is achieved. Desirable materials and thickness will be apparent to the skilled man.

Suitable materials for the pressure-sensitive adhesive matrix are well known to those skilled in the art and include, for example, polyisobutylenes, polysiloxanes, polyacrylate copolymers (polyacrylic esters), natural rubber/karaya gum-based adhesives, hydrogels, hydrophilic polymers, and polyurethanes. The adhesive may further include modifying monomers, tackifiers, plasticizers, fillers, waxes, oils, and other additives to impart the desired adhesive properties.

The adhesive layer can be any appropriate thickness which will provide the necessary adhesion to the patient's skin. Desirable materials and thickness will be apparent to the skilled man.

Preferred transdermal delivery devices also comprise a removable protective layer or release liner. The removable protective layer is removed prior to application, and preferably comprises the same materials used for the production of the backing layer described above provided that they are rendered removable, for example, by a silicone treatment. Other removable protective layers are, for example, polytetrafluoroethylene, treated paper, allophane and polyvinyl chloride. Preferably, the removable protective layer is in contact with the adhesive layer and provides a convenient means of maintaining the integrity of the adhesive layer until the desired time of application.

The removable protective layer can be any appropriate thickness which will provide the necessary protection to the adhesive layer prior to application. Desirable materials and thickness will be apparent to the skilled man.

The transdermal delivery device may optionally include one or more penetration enhancers, which increase the rate at which the opioid agonist penetrates through the patient's skin. Preferably the penetration enhancer does not enhance the penetration of the N-oxide derivative of the opioid antagonist. Preferably the penetration enhancer penetrates the rate-controlling membrane or diffuses out of the polymer matrix or adhesive matrix so that it can contact the patient's skin and improve penetration of the opioid agonist through the patient's skin. Suitable penetration enhancers for use in the transdermal delivery devices and compositions of the invention include, for example, $C_{2-4}$ alcohols, e.g. ethanol and isopropanol, polyethylene glycol monolaurate, polyethylene glycol-3-lauramide, dimethyl lauramide, sorbitan trioleate, fatty acids, esters of fatty acids having from about 10 to about 20 carbon atoms, monoglycerides or mixtures of monoglycerides of fatty acids having a total monoesters content of at least 51% where the monoesters are those with from 10 to 20 carbon atoms, and mixtures of mono-, di- and tri-glycerides of fatty acids. Suitable fatty acids include, for example, lauric acid, myristic acid, stearic acid, oleic acid, linoleic acid and palmitic acid. Monoglyceride permeation enhancers include, for instance, glycerol monooleate, glycerol monolaurate, and glycerol monolinoleate.

The composition may optionally further comprise one or more additives conventionally used in compositions designed for use in transdermal delivery devices. For example, the composition may also include one or more preservatives or bacteriostatic agents, e.g. methyl hydroxybenzoate, propyl hydroxybenzoate, chlorocresol, benzalkonium chlorides; or other active ingredients such as antimicrobial agents, particularly antibiotics; anesthetics; other analgesics; and antipruritic agents.

Generally, the size of the device of can vary from about 1 $cm^2$ to greater than 200 $cm^2$ and typically are between about 5-50 $cm^2$. Methods for manufacturing transdermal delivery devices are well known to those skilled in the art.

The skilled man may readily determine an appropriate amount of the composition of the invention to include in a transdermal delivery device. The total amount of opioid agonist provided is generally that sufficient to provide analgesia. The total amount of opioid agonist administered to a patient in a dose will vary depending on numerous factors including the nature of the opioid agonist, the weight of the patient, the severity of the pain, the nature of other therapeutic agents being administered etc.

The total amount of N-oxide derivative of opioid antagonist provided is generally that sufficient to inhibit or minimise the opioid agonist induced euphoric effect that would otherwise be achieved ift he composition were to be administered by a route other than transdermal, particularly orally or parentally. The total amount of N-oxide derivative of opioid antagonist will vary depending on various factors including the nature of the opioid agonist present, the nature of the N-oxide derivative of opioid antagonist, the weight of the patient, the overall treatment regimen and so on.

Preferred transdermal delivery devices of the present invention release active agent, e.g. opioid agonist, in a controlled release rate. The precise release profile can be altered by, for example, varying the size of the device, its composition and/or the concentration of opioid agonist present. Further preferred transdermal delivery devices release essentially no compound of formula (I) when used transdermally. Preferably less than 10 % wt, more preferably less than 5 % wt and still more preferably less than 1 % wt of the compound of formula (I) originally present in the device is delivered transdermally when a system of the present invention is applied to the skin for 72-168 hours, e.g. 168 hours.

Preferred compositions and transdermal delivery devices, of the present invention are tamper resistant. A s used herein, the term "tamper resistant" refers to compositions and transdermal delivery devices that do not provide an abuser who extracts their contents with solvent and injects the resulting solution with an euphorogenic effect.

The present invention further relates to a pharmaceutical composition comprising an N-oxide derivative of an opioid antagonist, or a salt thereof, and an opioid agonist, or a salt thereof, as hereinbefore described for use in medicine, and particularly for use in the treatment of pain.

Methods of treating a subject in need of pain relief comprise administering to the subject a pharmaceutical composition as hereinbefore described. A preferred method for treating or preventing pain in a patient is by transdermally administering to the patient in need thereof an analgesically effective amount of a composition as hereinbefore described, with the transdermal-delivery device of the invention. Methods for treating or preventing pain in a patient comprise contacting the skin of a patient in need thereof with a transdermal delivery device as hereinbefore described. Preferably the composition present in the devices of the invention comprises an analgesically effective amount of an opioid agonist, or a pharmaceutically acceptable salt thereof, and a N-oxide derivative of opioid antagonist, or a pharmaceutically

acceptable salt thereof, in an amount sufficient to inhibit the euphoric effect of the opioid, wherein the contacting is fora n amount of time sufficient to treat or prevent pain. Preferably the patient is human.

The pain treated or prevented as hereinbefore described may be acute pain or chronic pain. For example, the pain may be cancer pain, central pain, labor pain, myocardial infarction pain, pancreatic pain, colic pain, post-operative pain, headache pain, muscle pain and bone pain.

In preferred methods of the invention the composition, preferably via a transdermal delivery device, is contacted with the skin of the patient, and the opioid agonist is released and becomes absorbed through the skin of the patient. Once absorbed, the opioid agonist enters into the patient's circulatory system providing an analgesically effective amount of the opioid agonist. Preferably the transdermal delivery device is contacted with a patient's skin for from about 12 h to about 2 weeks and more preferably from about 24 h to about 1 week. In another embodiment, the transdermal delivery device is contacted with a patient's skin for from about 3 days to about 1 week.

DESCRIPTION OF THE FIGURES

Figure 1 shows the structures of preferred N-oxide derivatives of opioid antagonists present in preferred compositions and transdermal delivery devices of the present invention;
Figure 2 is a schematic of a preferred reservoir type transdermal delivery device of the invention;
Figure 3 is a schematic of a preferred polymer-matrix transdermal delivery device of the present invention
Figure 4 is a schematic of a preferred drug-in-adhesive transdermal delivery device of the present invention;
Figure 5 is a graph showing the in vitro skin permeation of an opioid agonist from a prototype transdermal patch comprising an opioid agonist and naloxone N-oxide;
Figure 6 is a graph showing the mean concentration of Naloxone in human plasma over 2 h at 37 °C (10 ng/ml of Naloxone N-oxide);
Figure 7 is a graph showing the mean concentration of Naloxone N-oxide in human plasma over 2 h at 37 °C (10 ng/ml of Naloxone N-oxide);
Figure 8 is a graph showing the mean concentration of Naloxone in human plasma over 2 h at 37 °C (50 ng/ml of Naloxone N-oxide);
Figure 9 is a graph showing the mean concentration of Naloxone N-oxide in human plasma over 2 h at 37 °C (50 ng/ml of Naloxone N-oxide);
Figure 10 is a graph showing the mean concentration of Naloxone in human plasma over 2 h at 37 °C (250 ng/ml of Naloxone N-oxide);
Figure 11 is a graph showing the mean concentration of Naloxone N-oxide in human plasma over 2 h at 37 °C (250 ng/ml of Naloxone N-oxide);
Figure 12 is a graph showing the mean concentration of Naloxone in human whole blood over 2 h at 37 °C (10 ng/ml of Naloxone N-oxide);
Figure 13 is a graph showing the mean concentration of Naloxone N-oxide in human whole blood over 2 h at 37 °C (10 ng/ml of Naloxone N-oxide);
Figure 14 is a graph showing the mean concentration of Naloxone in human whole blood over 2 h at 37 °C (50 ng/ml of Naloxone N-oxide);
Figure 15 is a graph showing the mean concentration of Naloxone N-oxide in human whole blood over 2 h at 37 °C (50 ng/ml of Naloxone N-oxide);
Figure 16 is a graph showing the mean concentration of Naloxone in human whole blood over 2 h at 37 °C (250 ng/ml of Naloxone N-oxide); and
Figure 17 is a graph showing the mean concentration of Naloxone N-oxide in human whole blood over 2 h at 37 °C (250 ng/ml of Naloxone N-oxide)

DETAILED DESCRIPTION OF THE FIGURES

Figure 2 depicts a preferred reservoir-type transdermal delivery device of the invention. The transdermal delivery device 10 comprises a reservoir 11, typically in the form of a solution or a dispersion 12, having dispersed therein a composition as hereinbefore described, i.e. a composition comprising an opioid agonist, or a pharmaceutically acceptable salt thereof, 13 and a N-oxide derivative of an opioid antagonist, or a pharmaceutically acceptable salt thereof, 14.

The reservoir 11 is disposed between an impermeable backing film 15, a rate-controlling membrane 16, and a pressure-sensitive adhesive 17. A release liner 18 is applied to the pressure-sensitive adhesive layer 17, and is removed prior to use. In one preferred embodiment, the opioid agonist and the N-oxide derivative of the opioid antagonist are uniformly dispersed throughout the reservoir.

Figure 3 depicts a preferred polymer-matrix transdermal delivery device of the invention. The transdermal delivery device 20 comprises a reservoir 21 in the form of a polymer matrix 22, having dispersed therein a composition as hereinbefore described, i.e. a composition comprising an opioid agonist, or a pharmaceutically acceptable salt thereof, 23 and a N-oxide derivative of an opioid antagonist, or a pharmaceutically acceptable salt thereof, 24. Preferably the opioid agonist and the N-oxide derivative of the opioid antagonist are uniformly dispersed throughout the polymer matrix. The polymer matrix 21 is supported on an impermeable backing layer 25 and has a peripheral ring of adhesive 26 located around the edge of the patch. A release liner 28 is applied to the peripheral ring of adhesive 26 and polymer matrix 22 and is removed prior to use.

Figure 4 depicts a preferred drug-in-adhesive transdermal delivery device of the invention. The transdermal delivery device 30 comprises an adhesive matrix 31 having dispersed therethrough a composition as hereinbefore described, i.e. a composition comprising an opioid agonist, or a pharmaceutically acceptable salt thereof, 32 and a N-oxide derivative of an opioid antagonist, or a pharmaceutically acceptable salt thereof, 33. Preferably the opioid agonist and the N-oxide derivative of the opioid antagonist are uniformly dispersed throughout the adhesive matrix. The adhesive matrix 31 is supported on an impermeable backing layer 34 and has an impermeable release liner 35 on the side that faces the skin which is removed prior to use.

EXAMPLES

Determination of $pK_a$ and logP of naloxone free base and naloxone N-oxide

$pK_a$ analysis was performed using a pH-metric method.

The sample was titrated in a pH-metric triple titration from pH 2.0-12.0 at concentrations of 1.6-1.2 mM for naloxone free base and 1.7-1.4 mM for naloxone N-oxide under aqueous conditions. No precipitation or degradation was observed and two pKas were determined from the potentiometric data.

Log P was determined using the potentiometric (pH-metric method).

The sample was titrated in various ratios of octanol/water in two titrations covering the pH range 2.0-12.0 at concentrations of 1.9-1.1 mM. The shift of the aqueous pKas in the presence of octanol was used to determine the log P of the neutral species.

The results are shown in the table below.

| Naloxone free base | | | | |
|---|---|---|---|---|
| pKa | Type | T/°C | Ionic environment | Method |
| 8.07±0.01 | Base | 25.0 | 0.15 M KCl | pH-metric |
| 9.15±0.01 | Acid | 25.0 | 0.15 M KCl | pH-metric |
| | | | | |
| LogP | Species | T/°C | Ionic environment | Method |
| 1.97±0.01 | Neutral | 25.0 | 0.15 M KCl | pH-metric |
| **Naloxone N-oxide** | | | | |
| pKa | Type | T/°C | Ionic environment | Method |
| 2.96±0.01 | Base | 25.0 | 0.15 M KCl | pH-metric |
| 8.55±0.01 | Acid | 25.0 | 0.15 M KCl | pH-metric |
| | | | | |
| LogP | Species | T/°C | Ionic environment | Method |
| -0.42±0.01 | Neutral | 25.0 | 0.15 M KCl | pH-metric |

A logP value of around 3 is generally considered to be desirable for a compound which is to be delivered by passive transdermal delivery. Naloxone free base has a logP value of 1.97 and therefore has the potential to cross the skin. Naloxone N-oxide, however, has a logP value of -0.42 which indicates that it cannot be delivered by passive

transdermal delivery through the skin.

Determination of the intrinsic in vitro skin permeation of Naloxone and Naloxone N-oxide

The determination of in vitro skin permeation was performed with saturated solutions of the respective antagonist species. Therefore an excess of the antagonist was weighed in phosphate buffer of pH 5.0 and stirred overnight. pH was monitored and, if necessary, adjusted. The cells were incubated at 32°C. Solutions were filtrated through a syringe filter (1μm PTFE) prior to application into the diffusion cells. Concentrations of the solutions were determined.

Diffusion cells (made of glass) with a vertical application and a volume of 5 ml were used. The human skin used for the analysis came from an aesthetic operation. Belly skin from female donors was supplied from plastic surgery. After arrival, skin was visually checked whether it was without any scars and stretch marks. A layer of 200-400 μm was cut with a dermatome. The permeation area of 0.82 $cm^2$ was punched out of the skin.

The diffusion cell consists of a donor chamber and a receptor compartment, the skin is fixed between both. The sampling and volume replacement were executed via an autosampler. Further details of the set up are in the table below.

| | |
|---|---|
| Cell | 5 mL Franz cell, vertical; permeation area 0.82 $cm^2$, n=7 per variant plus blanks |
| Skin | Human split-thickness skin (200-400 μm thick) prepared using a dermatome; sex female; body site belly |
| Testing volume | 5 ml |
| Revolutions | Magnetic stirring bars 2 x 5 mm |
| Sampling time | 6, 12, 18, 24, 36, 48, 60 and 72 hours |
| Sampling volume | 4.5 ml |
| Replaced volume | 4.5 ml |
| Temperature | 32 °C |
| Stirring speed | 350 rpm |
| Acceptor medium | Phosphate buffer pH5.0; 1.36 g $KH_2PO_4$ were dissolved in 500 ml Milli-Q water. The pH value is adjusted to 5.0 ± 0.05 with phosphoric acid or potassium hydroxide solution. |
| Donor solution or Transdermal patch | Saturated solution of the antagonist in Phosphate buffer pH 5.0: (1.36 g $KH_2PO_4$ were dissolved in 500 ml Milli-Q water. The pH value is adjusted to 5.0 ± 0.05 with phosphoric acid or potassium hydroxide solution).<br>Place the TDS on the skin and press it for a short time of 10 s. Apply patch adhered to skin on the Franz cell, which is then placed in the incubator for the permeation. At each sampling time, a sample was taken by an autosampler and the same volume was replaced. Aliquots of the sample solution were transferred into HPLC vials for analyses. |
| Materials/reagents | Binder KBS. Prosense AutoPlus/Maximiser. Franz cell. Pipette. Deratome, HPLC - Dionex Ultimate, glass vials with caps |

The determination of naloxone and naloxone N-oxide in the acceptor medium of the in vitro skin permeations samples (receptor phase) was done by HPLC with UV-detection. The concentration of the saturated solutions and permeated antagonist concentration in the receptor medium is shown in the table below.

| Antagonist | Concentration saturated solution c [mg/ml] | Mass API from donor in acceptor mean value [μg] | Relative standard deviation [%] |
|---|---|---|---|
| Naloxone | 4.46 | 38.37<br>(n=5) | 45.70 |
| Naloxone N-oxide | 3.96 | ND<br>(n=5) | - |
| ND = none detected<br>Naloxone N-oxide did not permeate through the skin. | | | |

Prototype transdermal patch comprising naloxone N-oxide and an opioid agonist

Naloxone N-oxide was incorporated into a prototype transdermal patch along with an opioid agonist. The ratio of naloxone N-oxide to the opioid agonist in the patch was 1:1, 3:1 or 5:1 by weight. Batches of patches were prepared by weighing the appropriate amounts of the opioid agonist, Naloxone N-oxide and dry patch matrix of poly(meth)acrylate and dissolving in ethylacetate. The matrix solvent system was added and stirred for 30 minutes on a magnetic stirrer to yield a homogenous mixture. After mixing, the drug/polymer mixture was hand cast onto a release liner, Loparex Prime Liner FL 2000. Casting was carried out with a casting knife of variable width to achieve a target dry area weight matrix of 55 g/m$^2$. The cast was then dried at room temperature for 10 minutes, transferred to a convection oven and dried at 70 °C for 15 minutes and at 100 °C for 5 minutes. Finally the dried casts were hand-laminated with an occlusive backing, Scotchpak 9738, and patches were cut out of the laminate with 0.82 cm$^2$ cutting dies for skin permeation and 5 cm$^2$ for stability experiments.

Patches, containing the opioid agonist, drug loading 4.5% (no Naloxone N-oxide), were also prepared using an identical method.

The in vitro skin permeation of opioid agonist from each of the patches was tested using a Franz cell as described above. The results are presented in Figure 5. The results show that the opioid agonist permeates skin in the presence of various ratios of naloxone N-oxide. It was also confirmed that no detectable levels of N-oxide permeated through the skin regardless of the ratio.

The stability of each of the patches over 6 days at 60 °C was also tested. The results are summarised in the table below and show that the Naloxone N-oxide does not impact on the stability of the patch.

| Patch | Time point | Sum impurities [%] |
|---|---|---|
| Opioid agonist only 4.5 % API | Initial | 1.19 |
| | 6 days/60 °C | 1.34 |
| Opioid agonist, 4.5 % Naloxone N-oxide, 13.5 % | Initial | 0.97 |
| | 6 days/60 °C | 1.50 |
| Opioid agonist, 4.5 % Naloxone N-oxide, 4.5 % | Initial | 0.93 |
| | 6 days/60 °C | 1.43 |
| Opioid agonist, 4.5 % Naloxone N-oxide, 22.5 % | Initial | 0.93 |
| | 6 days/60 °C | 1.02 |

Determination of stability of Naloxone and Naloxone N-oxide in human plasma and blood samples

The purpose of this experiment was the measurement of Naloxone and Naloxone-N-oxide concentrations in human plasma by LC-MS/MS. The objective was to assess in-vitro stability of Naloxone-N-oxide in human plasma and blood.

Analytical procedures followed were based on those outlined in the "Guideline on Bioanalytical Method Validation", EMA, CHMP, EWP, July 2011 and "Reflection paper for laboratories that perform the analysis or evaluation of clinical trial samples, 28 February 2012, EMA/INS/GCP/532137/2010" with reference to the "Guidance for Industry, Bioanalytical Method Validation" recommendations issued by the U.S. Department of Health and Human Services, Food and Drug Administration, Center for Drug Evaluation and Research (CDER), Center for Veterinary Medicine (CVM), May 2001, BP.

Reference Standards

| | Naloxone | Naloxone-N-oxide | Naloxone-D5 |
|---|---|---|---|
| Supplier | Cerilliant | Aptuit | Cerilliant |
| Lot Number | FN093012-03 | 94048-01 | FN093012-02 |
| Storage Conditions | -20°C | RT, Desiccated | -20°C |
| Molecular Formula (free base) | $C_{19}H_{21}NO_4$ | $C_{19}H_{21}NO_5$ | $C_{19}H_{16}NO_4D_5$ |
| Molecular Weight (free base) | 327.38 | 343.37 | 332.41 |

(continued)

| | Naloxone | Naloxone-N-oxide | Naloxone-D5 |
|---|---|---|---|
| Water content (%) | 2.99% | 0.2% | 0% (None Detected) |
| Purity (%) | Certified 100μg/mL solution | 97.82% | Certified 100μg/mL solution |
| 1 mg compound weighed equals | N/A | 0.980 | N/A |
| Physical Appearance | Solution | White Solid | Solution |

Control blank human plasma and blood containing K2EDTA as an anti-coagulant, was obtained from Clinical Trials Laboratory Service or BAS volunteers and was stored at nominally -20°C and +4°C, respectively, when not in use.

The mass spectrometer used was an Applied Biosystems API5000 with the following settings:

| | |
|---|---|
| Ionisation/Interface | Positive TurboIonSpray |
| Source temperature | +650 °C |
| GS1 | 50 psi |
| GS2 | 50 psi |
| Curtain gas setting | 30 psi |
| Collision gas setting | 12 psi |
| Ionspray voltage | 1000 eV |

Procedure

Concentrations of Naloxone and Naloxone-N-oxide in human plasma were measured by LC-MS/MS after protein precipitation extraction over the calibration range of 0.1-25 ng/mL and 0.5-500 ng/mL respectively. The purpose of the assay was to assess the stability of the Naloxone-N-oxide in human plasma and whole blood. Stability samples of Naloxone-N-oxide (n=3 for each time point and concentration) at 10, 50 and 250 ng/mL, were incubated at +37°C. Plasma and plasma generated from whole blood were analysed post centrifugation at t=0, 10, 20, 30, 45, 60 and 120 minutes for both Naloxone-N-oxide and Naloxone.

Naloxone in human plasma was proven stable at +22°C and -20°C for up to 24 hours and 156 days respectively.

Data Acquisition and Processing

All instrument control, data collection, peak area integration and storage was performed using Analyst (version 1.5.2). Peak areas were then imported into Watson LIMS (version 7.2.0.02) for regression and quantification. The mass spectrometer response (peak area ratio of analyte to internal standard) of each calibration standard was calculated by Watson LIMS and plotted against the nominal (prepared) concentration. A weighted ($1/x^2$) least squares linear regression analysis was used to calculate an equation of the calibration line. Concentrations of Naloxone and Naloxone-N-oxide in the plasma samples were back calculated from the calibration lines to 3 significant figures.

Acceptability of Analytical Batches and Reanalysis

All results quoted are from batches which met the following criteria:

$$\text{RE (relative error) (\%)} = \frac{\text{calculated concentration} - \text{nominal concentration}}{\text{nominal concentration}} \times 100$$

- At least 75% of calibration standards within +15% RE (within +20% RE at the lower limit of quantification (LLOQ)) of their target concentrations.
- At least two-thirds of the QC samples within +15% RE of their respective target values, with at least 50% at each concentration.

Assay Performance

All reported batches met the acceptance criteria described above.

Stability samples

The results obtained for all study samples are presented in Table 1 to Table 12 and plotted results shown in Figure 6 to Figure 17. The lower limits of quantification (LLOQ) for Naloxone and Naloxone-N-oxide in human plasma were 0.1 and 0.5 ng/mL respectively. Any concentrations below this level are reported as below the limit of quantification (BLQ). Samples with initial concentrations above the upper limit of quantification (25 and 500 mg/mL) were re-injected with a reduced injection volume (alongside QC High with reduced injection volume) and sample concentrations interpolated against a calibration curve injected with normal injection volume.

Plasma samples

At t=0 the amount of Naloxone present in the Naloxone-N-oxide spiked plasma samples was comparable to the amount present in the Naloxone-N-oxide spiking solutions. Over the 2 hour time course there was a small, but insignificant amount of conversion of the Naloxone-N-oxide to Naloxone in the plasma, however the stability samples analysed indicted stability of Naloxone-N-oxide in plasma.

Blood sample

The Naloxone-N-oxide spiked whole blood samples were centrifuged and resulting plasma analysed. At t=0 there was a high amount of Naloxone present compared to plasma suggesting an instant release of Naloxone (during the plasma generation step). The combined t=0 concentrations of Naloxone-N-oxide and Naloxone is greater than the theoretical spiked amount of Naloxone-N-oxide. The greater than theoretical maximum value is likely due to a partitioning effect of Naloxone-N-oxide in blood, i.e. blood spiked with Naloxone-N-oxide will take a while to equilibrate between the plasma and whole blood cell volume, more compound being freely available in the plasma than blood cells. Over the 2 hour time course there was a large decrease in Naloxone-N-oxide in whole blood with an increase in Naloxone over the same period. This shows that Naloxone N-oxide rapidly converts to Naloxone in whole blood.

Table 1: Concentrations of Naloxone in human plasma samples (10 ng/mL)

| Minutes | 0 | 10 | 20 | 30 | 45 | 60 | 120 |
|---|---|---|---|---|---|---|---|
| Replicate 1 | 0.351 | 0.323 | 0.388 | 0.383 | 0.499 | 0.525 | 0.610 |
| Replicate 2 | 0.330 | 0.384 | 0.365 | 0.451 | 0.437 | 0.535 | 0.644 |
| Replicate 3 | 0.345 | 0.370 | 0.401 | 0.443 | 0.448 | 0.473 | 0.659 |
| | | | | | | | |
| Mean | 0.342 | 0.359 | 0.385 | 0.426 | 0.461 | 0.511 | 0.638 |
| S.D. | 0.0108 | 0.0320 | 0.0182 | 0.0372 | 0.0331 | 0.0333 | 0.0251 |
| %CV | 3.2 | 8.9 | 4.7 | 8.7 | 7.2 | 6.5 | 3.9 |
| n | 3 | 3 | 3 | 3 | 3 | 3 | 3 |

Table 2: Concentrations of Naloxone N-oxide in human plasma samples (10 ng/mL)

| Minutes | 0 | 10 | 20 | 30 | 45 | 60 | 120 |
|---|---|---|---|---|---|---|---|
| Replicate 1 | 9.93 | 9.61 | 10.6 | 9.37 | 10.7 | 10.3 | 10.4 |
| Replicate 2 | 9.87 | 10.4 | 10.3 | 10.1 | 10.0 | 10.6 | 10.1 |
| Replicate 3 | 10.2 | 10.7 | 9.93 | 10.4 | 10.4 | 10.1 | 10.4 |
| | | | | | | | |
| Mean | 10.0 | 10.2 | 10.3 | 9.96 | 10.4 | 10.3 | 10.3 |

(continued)

| | | | | | | | |
|---|---|---|---|---|---|---|---|
| S.D. | 0.176 | 0.563 | 0.336 | 0.530 | 0.351 | 0.252 | 0.173 |
| %CV | 1.8 | 5.5 | 3.3 | 5.3 | 3.4 | 2.4 | 1.7 |
| n | 3 | 3 | 3 | 3 | 3 | 3 | 3 |

Table 3: Concentrations of Naloxone in human plasma samples (50 ng/mL)

| Minutes | 0 | 10 | 20 | 30 | 45 | 60 | 120 |
|---|---|---|---|---|---|---|---|
| Replicate 1 | 1.74 | 1.62 | 1.85 | 1.96 | 2.00 | 2.50 | 3.06 |
| Replicate 2 | 1.55 | 1.89 | 1.77 | 1.87 | 2.15 | 2.36 | 2.95 |
| Replicate 3 | 1.38 | 1.82 | 1.74 | 1.87 | 2.24 | 2.34 | 3.17 |
| | | | | | | | |
| Mean | 1.56 | 1.78 | 1.79 | 1.90 | 2.13 | 2.40 | 3.06 |
| S.D. | 0.180 | 0.140 | 0.0569 | 0.0520 | 0.121 | 0.0872 | 0.110 |
| %CV | 11.5 | 7.9 | 3.2 | 2.7 | 5.7 | 3.6 | 3.6 |
| n | 3 | 3 | 3 | 3 | 3 | 3 | 3 |

Table 4: Concentrations of Naloxone N-oxide in human plasma samples (50 ng/mL)

| Minutes | 0 | 10 | 20 | 30 | 45 | 60 | 120 |
|---|---|---|---|---|---|---|---|
| Replicate 1 | 48.5 | 43.4 | 50.1 | 46.9 | 47.6 | 50.0 | 47.6 |
| Replicate 2 | 45.6 | 48.7 | 47.1 | 44.1 | 49.1 | 46.0 | 43.9 |
| Replicate 3 | 40.5 | 45.8 | 48.1 | 47.9 | 45.4 | 48.1 | 46.8 |
| | | | | | | | |
| Mean | 44.9 | 46.0 | 48.4 | 46.3 | 47.4 | 48.0 | 46.1 |
| S.D. | 4.05 | 2.65 | 1.53 | 1.97 | 1.86 | 2.00 | 1.95 |
| %CV | 9.0 | 5.8 | 3.2 | 4.3 | 3.9 | 4.2 | 4.2 |
| n | 3 | 3 | 3 | 3 | 3 | 3 | 3 |

Table 5: Concentrations of Naloxone in human plasma samples (250 ng/mL)

| Minutes | 0 | 10 | 20 | 30 | 45 | 60 | 120 |
|---|---|---|---|---|---|---|---|
| Replicate 1 | 9.47 | 8.44 | 8.98 | 9.92 | 10.3 | 12.5 | 15.7 |
| Replicate 2 | 8.69 | 8.87 | 9.12 | 9.98 | 10.4 | 11.7 | 15.9 |
| Replicate 3 | 8.54 | 8.86 | 9.53 | 9.88 | 10.8 | 12.0 | 14.2 |
| | | | | | | | |
| Mean | 8.90 | 8.72 | 9.21 | 9.93 | 10.5 | 12.1 | 15.3 |
| S.D. | 0.499 | 0.245 | 0.286 | 0.0503 | 0.265 | 0.404 | 0.929 |
| %CV | 5.6 | 2.8 | 3.1 | 0.5 | 2.5 | 3.3 | 6.1 |
| n | 3 | 3 | 3 | 3 | 3 | 3 | 3 |

Table 6: Concentrations of Naloxone N-oxide in human plasma samples (250 ng/mL)

| Minutes | 0 | 10 | 20 | 30 | 45 | 60 | 120 |
|---|---|---|---|---|---|---|---|
| Replicate 1 | 236 | 218 | 228 | 231 | 225 | 242 | 239 |
| Replicate 2 | 233 | 234 | 241 | 229 | 228 | 229 | 226 |
| Replicate 3 | 229 | 223 | 226 | 226 | 230 | 221 | 211 |
| | | | | | | | |
| Mean | 233 | 225 | 232 | 229 | 228 | 231 | 225 |
| S.D. | 3.51 | 8.19 | 8.14 | 2.52 | 2.52 | 10.6 | 14.0 |
| %CV | 1.5 | 3.6 | 3.5 | 1.1 | 1.1 | 4.6 | 6.2 |
| n | 3 | 3 | 3 | 3 | 3 | 3 | 3 |

Table 7: Concentrations of Naloxone in human whole blood samples (10 ng/mL)

| Minutes | 0 | 10 | 20 | 30 | 45 | 60 | 120 |
|---|---|---|---|---|---|---|---|
| Replicate 1 | 4.52 | 3.79 | 4.62 | 5.42 | 5.94 | 6.61 | 7.26 |
| Replicate 2 | 4.36 | 3.47 | 4.21 | 5.39 | 6.28 | 6.40 | 7.34 |
| Replicate 3 | 5.11 | 3.32 | 4.33 | 4.70 | 5.94 | 6.66 | 6.98 |
| | | | | | | | |
| Mean | 4.66 | 3.53 | 4.39 | 5.17 | 6.05 | 6.56 | 7.19 |
| S.D. | 0.395 | 0.240 | 0.211 | 0.407 | 0.196 | 0.138 | 0.189 |
| %CV | 8.5 | 6.8 | 4.8 | 7.9 | 3.2 | 2.1 | 2.6 |
| n | 3 | 3 | 3 | 3 | 3 | 3 | 3 |

Table 8: Concentrations of Naloxone N-oxide in human whole blood samples (10 ng/mL)

| Minutes | 0 | 10 | 20 | 30 | 45 | 60 | 120 |
|---|---|---|---|---|---|---|---|
| Replicate 1 | 7.95 | 5.94 | 4.70 | 2.77 | 2.18 | 1.79 | 1.55 |
| Replicate 2 | 7.69 | 6.11 | 4.15 | 3.14 | 2.27 | 1.81 | 1.57 |
| Replicate 3 | 6.70 | 6.01 | 4.75 | 3.47 | 2.01 | 2.02 | 1.41 |
| | | | | | | | |
| Mean | 7.45 | 6.02 | 4.53 | 3.13 | 2.15 | 1.87 | 1.51 |
| S.D. | 0.660 | 0.0854 | 0.333 | 0.350 | 0.132 | 0.127 | 0.0872 |
| %CV | 8.9 | 1.4 | 7.4 | 11.2 | 6.1 | 6.8 | 5.8 |
| n | 3 | 3 | 3 | 3 | 3 | 3 | 3 |

Table 9: Concentrations of Naloxone in human whole blood samples (50 ng/mL)

| Minutes | 0 | 10 | 20 | 30 | 45 | 60 | 120 |
|---|---|---|---|---|---|---|---|
| Replicate 1 | 15.8 | 19.2 | 20.5 | 25.4 | 24.0 | 27.1 | 29.6 |
| Replicate 2 | 21.4 | 19.6 | 21.4 | 25.1 | 23.2 | 25.2 | 31.5 |
| Replicate 3 | 16.7 | 18.4 | 19.6 | 23.3 | 21.9 | 26.2 | 31.7 |

(continued)

| | | | | | | | |
|---|---|---|---|---|---|---|---|
| | | | | | | | |
| Mean | 18.0 | 19.1 | 20.5 | 24.6 | 23.0 | 26.2 | 30.9 |
| S.D. | 3.01 | 0.611 | 0.900 | 1.14 | 1.06 | 0.950 | 1.16 |
| %CV | 16.7 | 3.2 | 4.4 | 4.6 | 4.6 | 3.6 | 3.8 |
| n | 3 | 3 | 3 | 3 | 3 | 3 | 3 |

Table 10: Concentrations of Naloxone N-oxide in human whole blood samples (50 ng/mL)

| Minutes | 0 | 10 | 20 | 30 | 45 | 60 | 120 |
|---|---|---|---|---|---|---|---|
| Replicate 1 | 48.9 | 26.0 | 20.3 | 15.9 | 14.1 | 10.7 | 3.99 |
| Replicate 2 | 41.3 | 25.0 | 21.1 | 15.2 | 14.1 | 10.6 | 4.51 |
| Replicate 3 | 46.0 | 26.8 | 19.9 | 16.9 | 15.1 | 10.0 | 4.22 |
| | | | | | | | |
| Mean | 45.4 | 25.9 | 20.4 | 16.0 | 14.4 | 10.4 | 4.24 |
| S.D. | 3.84 | 0.902 | 0.611 | 0.854 | 0.577 | 0.379 | 0.261 |
| %CV | 8.5 | 3.5 | 3.0 | 5.3 | 4.0 | 3.6 | 6.2 |
| n | 3 | 3 | 3 | 3 | 3 | 3 | 3 |

Table 11: Concentrations of Naloxone in human whole blood samples (250 ng/mL)

| Minutes | 0 | 10 | 20 | 30 | 45 | 60 | 120 |
|---|---|---|---|---|---|---|---|
| Replicate 1 | 62.1 | 64.8 | 81.1 | 88.2 | 112 | 104 | 127 |
| Replicate 2 | 60.3 | 75.5 | 85.3 | 93.3 | 108 | 108 | 124 |
| Replicate 3 | 61.1 | 62.4 | 84.1 | 87.8 | 112 | 100 | 129 |
| | | | | | | | |
| Mean | 61.2 | 67.6 | 83.5 | 89.8 | 111 | 104 | 127 |
| S.D. | 0.902 | 6.97 | 2.16 | 3.07 | 2.31 | 4.00 | 2.52 |
| %CV | 1.5 | 10.3 | 2.6 | 3.4 | 2.1 | 3.8 | 2.0 |
| n | 3 | 3 | 3 | 3 | 3 | 3 | 3 |

Table 12: Concentrations of Naloxone N-oxide in human whole blood samples (250 ng/mL)

| Minutes | 0 | 10 | 20 | 30 | 45 | 60 | 120 |
|---|---|---|---|---|---|---|---|
| Replicate 1 | 245 | 135 | 107 | 99.0 | 78.2 | 73.9 | 46.8 |
| Replicate 2 | 254 | 143 | 111 | 96.8 | 80.8 | 65.5 | 52.8 |
| Replicate 3 | 265 | 144 | 115 | 97.1 | 85.3 | 67.7 | 48.3 |
| | | | | | | | |
| Mean | 255 | 141 | 111 | 97.6 | 81.4 | 69.0 | 49.3 |
| S.D. | 10.0 | 4.93 | 4.00 | 1.19 | 3.59 | 4.36 | 3.12 |
| %CV | 3.9 | 3.5 | 3.6 | 1.2 | 4.4 | 6.3 | 6.3 |
| n | 3 | 3 | 3 | 3 | 3 | 3 | 3 |

## Claims

1. A transdermal delivery device comprising a pharmaceutical composition, wherein said composition comprises a N-oxide derivative of an opioid antagonist of formula (I), or a salt thereof, and an opioid agonist or salt thereof:

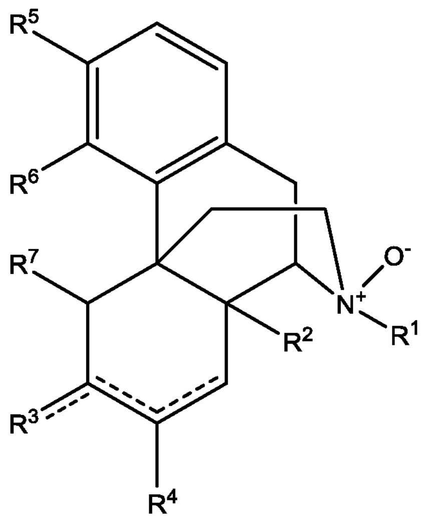

(I)

wherein

$R^1$ is selected from optionally substituted $C_{1-8}$ alkyl and optionally substituted $C_{2-8}$ alkenyl;
$R^2$ is selected from OH, H, $OC_{1-8}$ alkyl, NHCOR, $NR^1COR$, $CONR^1R$ and CONHR wherein R is a hydrocarbyl group or $R^2$ forms a bridge to the carbon to which $R^3$ is attached;
$R^3$ is selected from O, $CH_2$, $N\text{-}NH_2$ or a linking group to a second N-oxide derivative of an opioid antagonist or a salt thereof when the dashed bond is present and is selected from OH, H, $OC_{1-8}$ alkyl, $OCOR^1$, $O(CH_2CH_2(CH_2)_yO)_xCH_3$ wherein x is 1-10 and y is 0-3, $N(R^1)_2$, $NR^1COR$, NHCOR, $CONR^1R$, CONHR, wherein R is a hydrocarbyl group, or a linking group to a second N-oxide derivative of an opioid antagonist or a salt thereof, when the dashed bond is absent and $R^4$ is selected from H, CONHR, $CONR^1R$, NHCOR, $NR^1COR$ and $C(OH)(R^1)_2$, wherein R is a hydrocarbyl group; or
$R^3$ and $R^4$, together with the carbon atoms to which they are attached form a heterocycle or a linking group to a second N-oxide derivative of an opioid antagonist or a salt thereof;
$R^5$ is selected from OH, $OC_{1-8}$ alkyl, $OCOR^1$, H and $CONH_2$;
$R^6$ and $R^7$ are each independently selected from H, OH and $OC_{1-8}$ alkyl or $R^6$ and $R^7$, together with the carbon atoms to which they are attached, form a dihydrofuran ring; and
each dashed bond in the cyclohexyl ring may be present or absent with the proviso that both may not be present.

2. A device as claimed in claim 1, wherein said N-oxide derivative is of formula (II) or (IIIa):

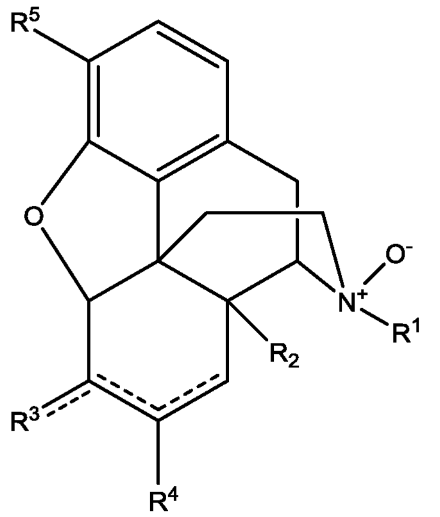

(II)

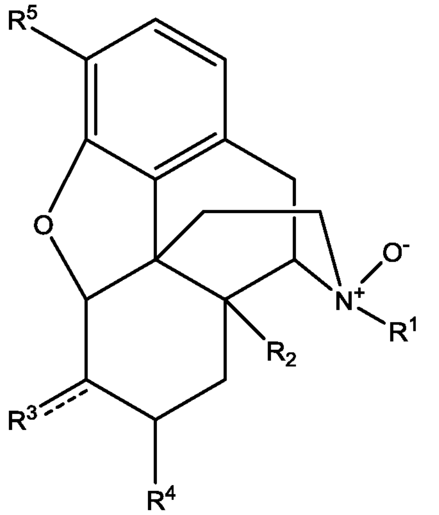

(IIIa)

wherein each of $R^1$, $R^2$, $R^3$, $R^4$ and $R^5$ and the dashed bonds are as defined in claim 1, or said N-oxide derivative is of formula (IIIb) or (IIIc):

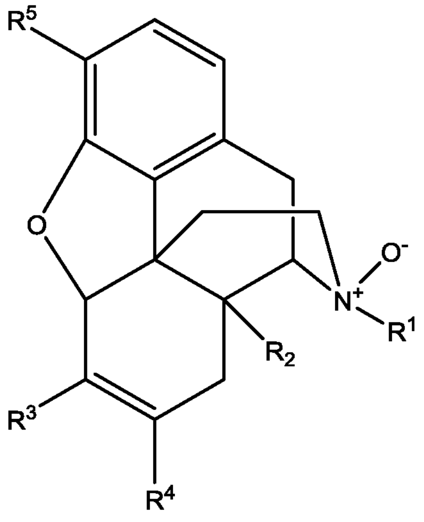

(IIIb)

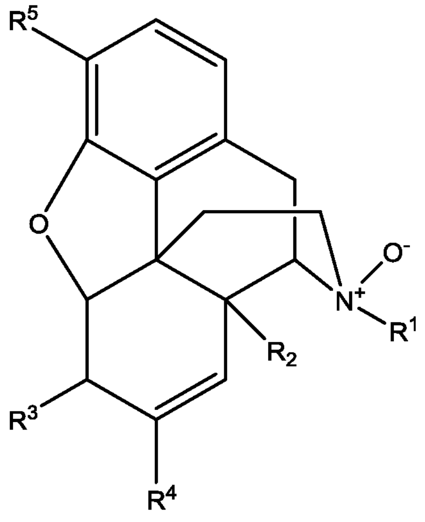

(IIIc)

wherein each of $R^1$, $R^2$ and $R^5$ are as defined in claim 1; and

$R^3$ is selected from OH, H, $OC_{1-8}$ alkyl, $OCOR^1$, $O(CH_2CH_2(CH_2)_yO)_xCH_3$ wherein x is 1-10 and y is 0 to 3 $N(R^1)_2$, $NR^1COR$, NHCOR, $CONR^1R$, CONHR, wherein R is a hydrocarbyl group, or a linking group to a second N-oxide derivative of an opioid antagonist or a salt thereof; $R^4$ is selected from H, CONHR, $CONR^1NR$, NHCOR, $NR^1COR$ and $C(OH)(R^1)_2$, wherein R is a hydrocarbyl group; or

$R^3$ and $R^4$, together with the carbon atoms to which they are attached form a heterocycle or a linking group to a second N-oxide derivative of an opioid antagonist or a salt thereof

or said N-oxide derivative is of formula (IV):

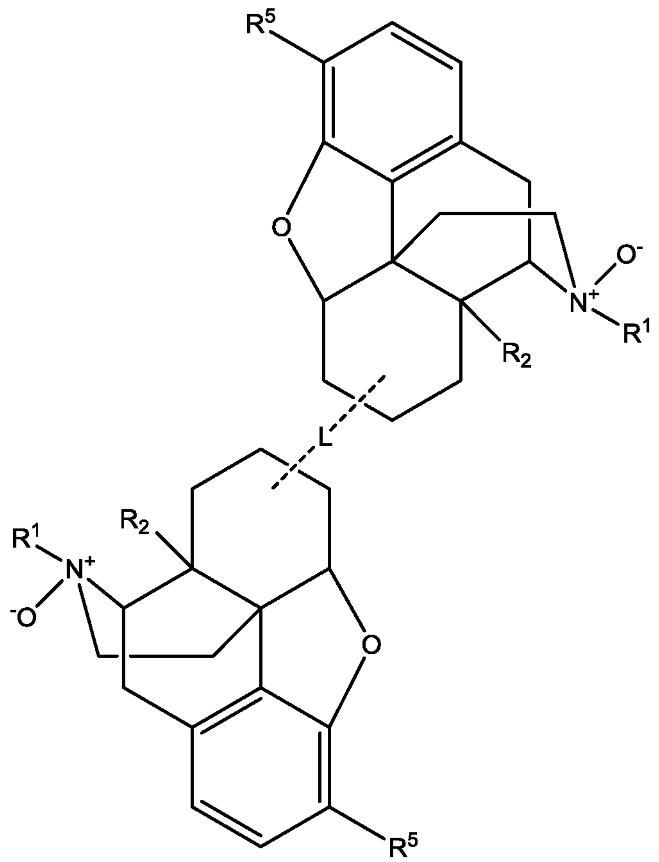

(IV)

wherein L is a linking group; and

each $R^1$, $R^2$ and $R^5$ are independently as defined as in claim 1.

**3.** A device as claimed in claim 1 or 2, wherein said N-oxide derivative is of formula (V) or (VI):

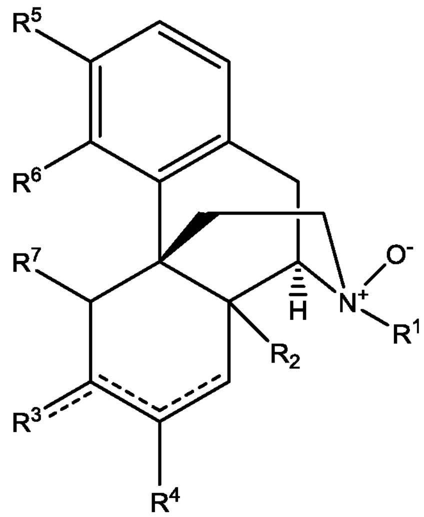

(V)

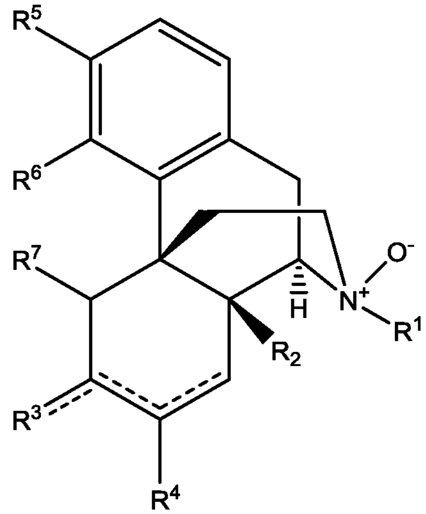

(VI)

wherein each of $R^1$, $R^2$, $R^3$, $R^4$, $R^5$, $R^6$, $R^7$ and the dashed bonds are as defined in claim 1.

**4.** A device as claimed in any one of claims 1 to 3, wherein said N-oxide derivative is of formula (VII), (VIII), (IX), (X), (XI) or (XII):

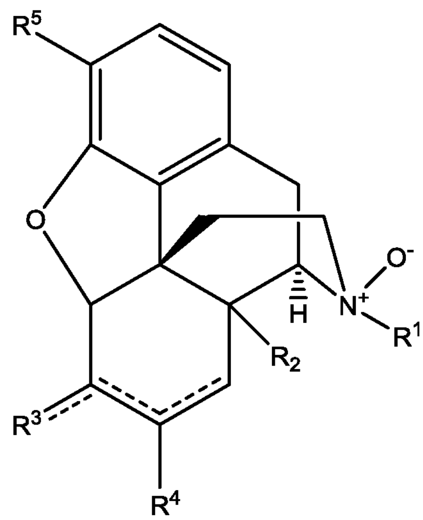

(VII)

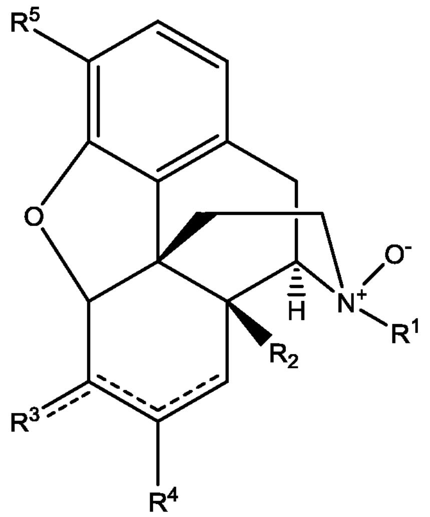

(VIII)

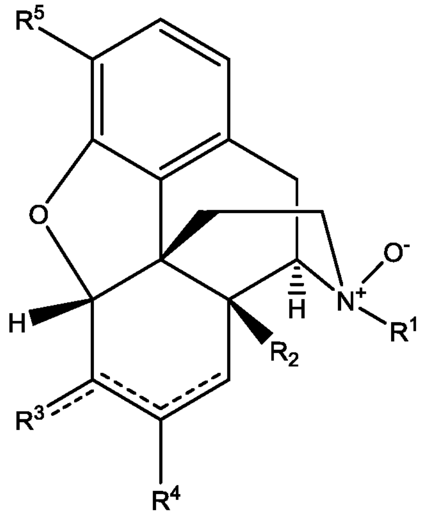

(IX)

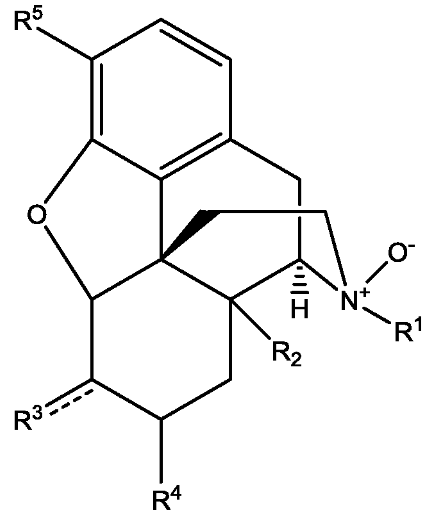

(X)

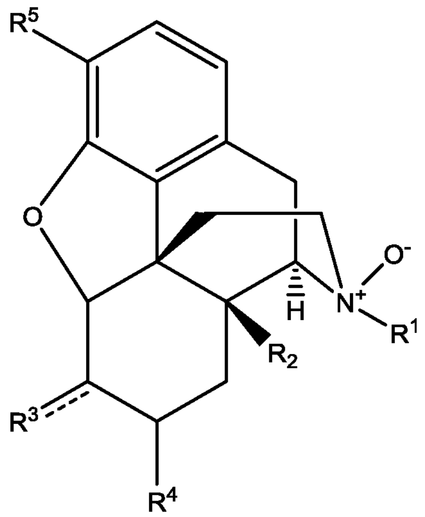

(XI)

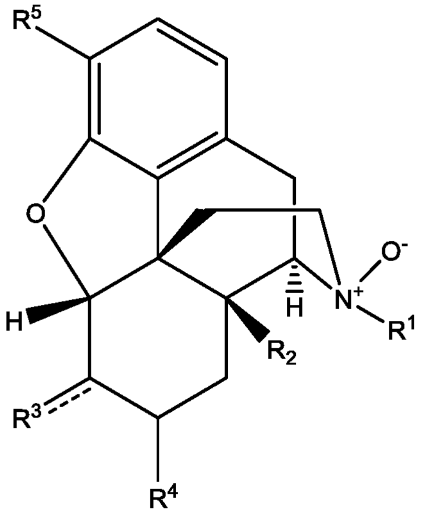

(XII)

wherein each of $R^1$, $R^2$, $R^3$, $R^4$, and $R^5$ and the dashed bonds are as defined in claim 1.

5. A device as claimed in any preceding claim, wherein in said N-oxide derivative of formulae (I)-(XII) $R^5$ is OH.

6. A device as claimed in any preceding claim, wherein in said N-oxide derivative of formulae (I)-(III) and (V)-(XII) $R^4$ is H.

7. A device as claimed in any preceding claim, wherein in said N-oxide derivative of formulae (I)-(XII) $R^2$ is OH.

8. A device as claimed in any preceding claim, wherein in said N-oxide derivative of formulae (I)-(III) and (V)-(XII), $R^3$ is selected from O, $CH_2$, N-$NH_2$ when the dashed bond is present and is selected from OH, H, $C_{1-8}$ alkyl, $OCOR^1$, $O(CH_2CH_2(CH_2)_yO)_xCH_3$ wherein x is 1-10 and y is 0-3, $N(R^1)_2$, $NR^1COR$, NHCOR, $CONR^1R$ and CONHR, wherein R is a hydrocarbyl group, when the dashed bond is absent and preferably $R^3$ is selected from O or $CH_2$, when the dashed bond is present, and is selected from OH or $OC_{1-8}$ alkyl, when the dashed bond is absent and still more preferably $R^3$ is O and the dashed bond is present.

9. A device as claimed in claim 1, wherein said N-oxide derivative is a derivative of an antagonist selected from Naloxone, Naloxol, Naloxegol, Naloxazone, Nalmefene, Nalbuphine, Nalmexone, Naltrexone, Naltrexol, Chlornaltrexamine, Clocinnamox, Nafurafine, Nalemedine, Naltrindole, 5'-Guanidinonaltrindole, Naltriben, Nalorphine, Diacetylnalorphine, Naloxonazine, Norbinaltorphimine, Binaltorphimine, Buprenorphine, Diprenorphine, Levellorphan, Cyprodime, Oxilorphan, Samidorphan and Cyclazocine and preferably said N-oxide derivative is a derivative of an antagonist selected from Naloxone and Naltrexone.

**10.** A device as claimed in any preceding claim, wherein said opioid agonist is selected from alfentanil, allylprodine, alphaprodine, anileridine, benzylmorphine, bezitramide, buprenorphine, butorphanol, carfentanil, clonitazene, codeine, desomorphine, dextromoramide, dezocine, diampromide, diamorphone, dihydrocodeine, dihydromorphine, dihydromorphone, dihydroisomorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl, heroin, hydrocodone, hydromorphone, hydromorphodone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, pantopon, papaveretum, paregoric, pentazocine, phenadoxone, phendimetrazine, phendimetrazone, phenomorphan, phenazocine, phenoperidine, piminodine, piritramide, propheptazine, promedol, properidine, propoxyphene, propylhexedrine, sufentanil, tilidine, tramadol, and mixtures thereof.

**11.** A device as claimed in any one of claims 1 to 10, which is a reservoir-type transdermal delivery device, a polymer-matrix type transdermal delivery device or a drug-in-adhesive type transdermal delivery device, preferably a drug-in-adhesive type transdermal delivery device.

**12.** A pharmaceutical composition comprising:

(i) a N-oxide derivative of an opioid antagonist of formula (I), or a salt thereof, preferably a N-oxide derivative of an opioid antagonist of formula (I) as defined in any one of claims 2 to 9;
(ii) an opioid agonist or salt thereof, preferably an opioid agonist as defined in claim 10; and
(iii) an adhesive and/or a matrix-forming polymer,

wherein said compound of formula (I) is:

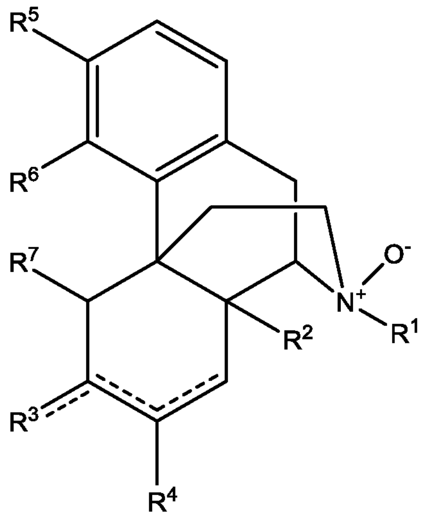

(I)

wherein

$R^1$ is selected from optionally substituted $C_{1-8}$ alkyl and optionally substituted $C_{2-8}$ alkenyl;
$R^2$ is selected from OH, H, $OC_{1-8}$ alkyl, NHCOR, $NR^1COR$, $CONR^1R$ and CONHR wherein R is a hydrocarbyl group or $R^2$ forms a bridge to the carbon to which $R^3$ is attached;
$R^3$ is selected from O, $CH_2$, $N-NH_2$ or a linking group to a second N-oxide derivative of an opioid antagonist or a salt thereof when the dashed bond is present and is selected from OH, H, $OC_{1-8}$ alkyl, $OCOR^1$, $O(CH_2CH_2(CH_2)_yO)_xCH_3$ wherein x is 1-10 and y is 0-3, $N(R^1)_2$, $NR^1COR$, NHCOR, $CONR^1R$, CONHR, wherein R is a hydrocarbyl group, or a linking group to a second N-oxide derivative of an opioid antagonist or a salt thereof, when the dashed bond is absent and $R^4$ is selected from H, CONHR, $CONR^1R$, NHCOR, $NR^1COR$ and $C(OH)(R^1)_2$, wherein R is a hydrocarbyl group; or
$R^3$ and $R^4$, together with the carbon atoms to which they are attached form a heterocycle or a linking group to a second N-oxide derivative of an opioid antagonist or a salt thereof;
$R^5$ is selected from OH, $OC_{1-8}$ alkyl, $OCOR^1$, H and $CONH_2$;

$R^6$ and $R^7$ are each independently selected from H, OH and $OC_{1-8}$ alkyl or $R^6$ and $R^7$, together with the carbon atoms to which they are attached, form a dihydrofuran ring; and
each dashed bond in the cyclohexyl ring may be present or absent with the proviso that both may not be present.

**13.** A transdermal dosage form comprising a composition as claimed in claim 12.

**14.** A method of making a composition as claimed in claim 12, comprising mixing an N-oxide derivative of an opioid antagonist of formula (I) as defined in any one of claims 1 to 9, or a salt thereof, an opioid agonist, or a salt thereof and an adhesive and/or a matrix forming polymer to form said composition.

**15.** A pharmaceutical composition as claimed in claim 12 for use in medicine, preferably for use in the treatment, e.g. transdermal treatment, of pain.

## Patentansprüche

**1.** Transdermale Zuführungsvorrichtung, umfassend eine pharmazeutische Zusammensetzung, wobei die Zusammensetzung ein N-Oxid-Derivat eines Opioidantagonisten der Formel (I) oder ein Salz davon und einen Opioidagonisten oder ein Salz davon umfasst:

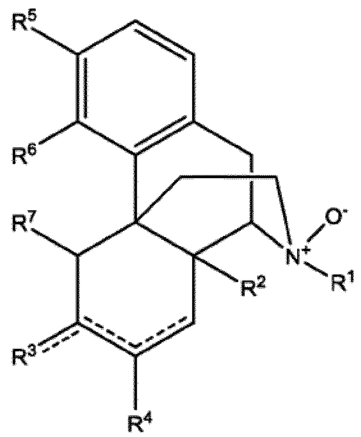

(I)

wobei

$R^1$ aus wahlweise substituiertem $C_{1-8}$-Alkyl und wahlweise substituiertem $C_{2-8}$-Alkenyl ausgewählt ist;
$R^2$ aus OH, H, $OC_{1-8}$-Alkyl, NHCOR, $NR^1COR$, $CONR^1R$ und CONHR ausgewählt ist, wobei R eine Hydrocarbylgruppe ist oder $R^2$ eine Brücke zu dem Kohlenstoff, an das $R^3$ angefügt ist, bildet;
$R^3$ aus O, $CH_2$, $N\text{-}NH_2$ oder einer verbindenden Gruppe zu einem zweiten N-Oxid-Derivat eines Opioidantagonisten oder einem Salz davon ausgewählt ist, wenn die gestrichelte Bindung vorhanden ist, und aus OH, H, $OC_{1-8}$-Alkyl, $OCOR^1$, $O(CH_2CH_2(CH_2)_yO)_xCH_3$, wobei x 1-10 ist und y 0- 3 ist, $N(R^1)_2$, $NR^1$ COR, NHCOR, $CONR^1R$, CONHR, wobei R eine Hydrocarbylgruppe ist, oder eine verbindende Gruppe zu einem zweiten N-Oxid-Derivat eines Opioidantagonisten oder ein Salz davon ist, wenn die gestrichelte Bindung nicht vorhanden ist, und $R^4$ aus H, CONHR, $CONR^1R$, NHCOR, $NR^1COR$ und $C(OH)(R^1)_2$, wobei R eine Hydrocarbylgruppe ist, ausgewählt ist; oder
$R^3$ und $R^4$, zusammen mit den Kohlenstoffatomen, an die sie angefügt sind, einen Heterocyclus oder eine verbindende Gruppe zu einem zweiten N-Oxid-Derivat eines Opioidantagonisten oder einem Salz davon bilden;
$R^5$ aus OH, $OC_{1-8}$-Alkyl, $OCOR^1$, H und $CONH_2$ ausgewählt ist;
$R^6$ und $R^7$ jeweils unabhängig aus H, OH und $OC_{1-8}$-Alkyl ausgewählt sind oder $R^6$ und $R^7$, zusammen mit den Kohlenstoffatomen, an die sie angefügt sind, einen Dihydrofuranring bilden; und
jede gestrichelte Bindung in dem Cyclohexylring vorhanden oder nicht vorhanden sein kann, mit der Maßgabe, dass nicht beide vorhanden sein können.

**2.** Vorrichtung nach Anspruch 1, wobei das N-Oxid-Derivat der Formel (II) oder (IIIa) entspricht:

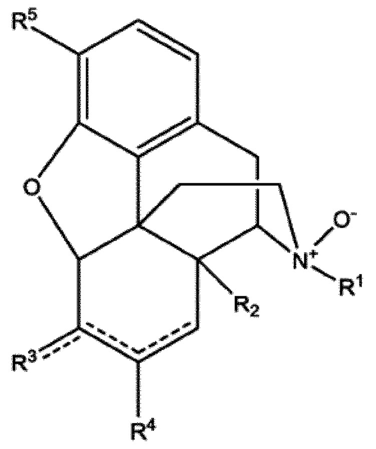

(II)

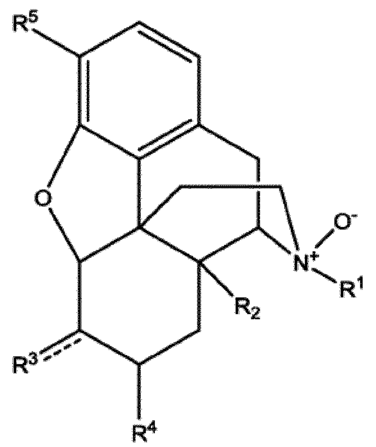

(IIIa)

wobei jedes von $R^1$, $R^2$, $R^3$, $R^4$ und $R^5$ und die gestrichelten Bindungen sind, wie in Anspruch 1 definiert, oder das N-Oxid-Derivat der Formel (IIIb) oder (IIIc) entspricht:

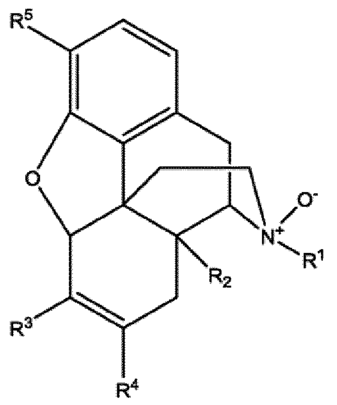

(IIIb)

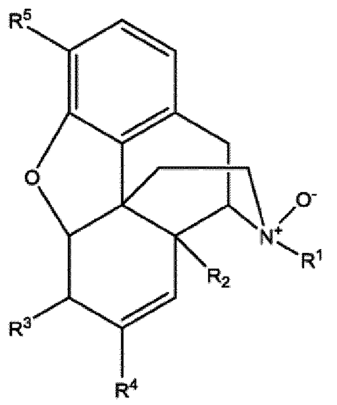

(IIIc)

wobei jedes von $R^1$, $R^2$ und $R^5$ sind, wie in Anspruch 1 definiert; und

$R^3$ aus OH, H, $OC_{1-8}$-Alkyl, $OCOR^1$, $O(CH_2CH_2(CH_2)_yO)_xCH_3$, wobei x 1-10 ist und y 0- 3 ist, $N(R^1)_2$, $NR^1$ COR, NHCOR, $CONR^1R$, CONHR, wobei R eine Hydrocarbylgruppe ist, oder eine verbindende Gruppe zu einem zweiten N-Oxid-Derivat eines Opioidantagonisten oder ein Salz davon ist; $R^4$ aus H, CONHR, $CONR^1R$, NHCOR, $NR^1COR$ und $C(OH)(R^1)_2$, wobei R eine Hydrocarbylgruppe ist, ausgewählt ist; oder

$R^3$ und $R^4$, zusammen mit den Kohlenstoffatomen, an die sie angefügt sind, einen Heterocyclus oder eine verbindende Gruppe zu einem zweiten N-Oxid-Derivat eines Opioidantagonisten oder ein Salz davon bilden; oder das N-Oxid-Derivat der Formel (IV) entspricht:

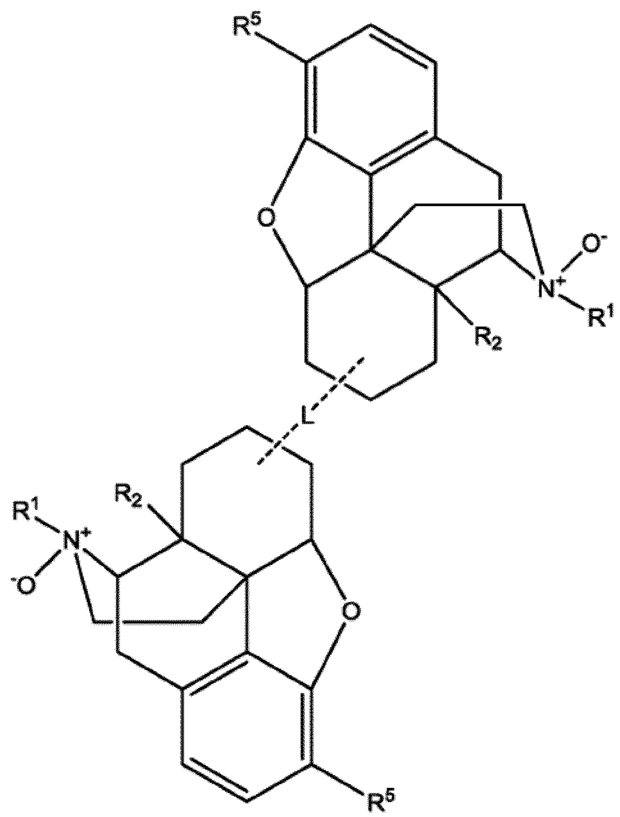

(IV)

wobei L eine verbindende Gruppe ist; und
jedes $R^1$, $R^2$ und $R^5$ unabhängig sind, wie in Anspruch 1 definiert.

**3.** Vorrichtung nach Anspruch 1 oder 2, wobei das N-Oxid-Derivat der Formel (V) oder (VI) entspricht:

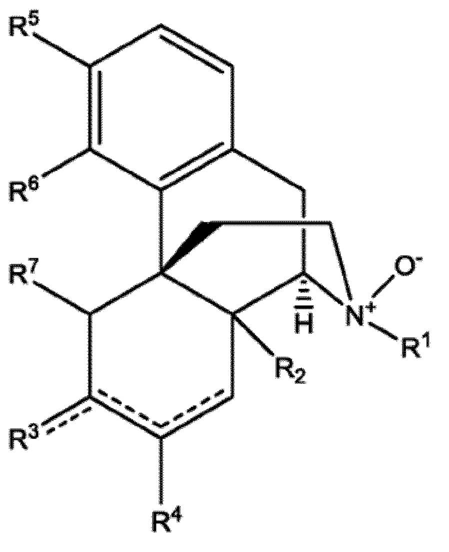

(V)

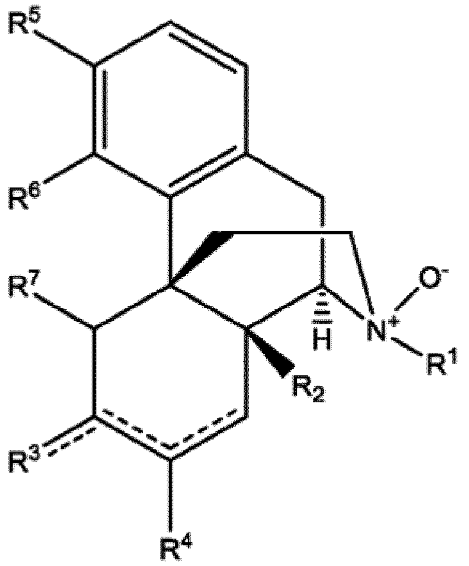

(VI)

wobei jedes von $R^1$, $R^2$, $R^3$, $R^4$, $R^5$, $R^6$, $R^7$ und die gestrichelten Bindungen sind, wie in Anspruch 1 definiert.

**4.** Vorrichtung nach einem der Ansprüche 1 bis 3, wobei das N-Oxid-Derivat der Formel (VII), (VIII), (IX), (X), (XI) oder (XII) entspricht:

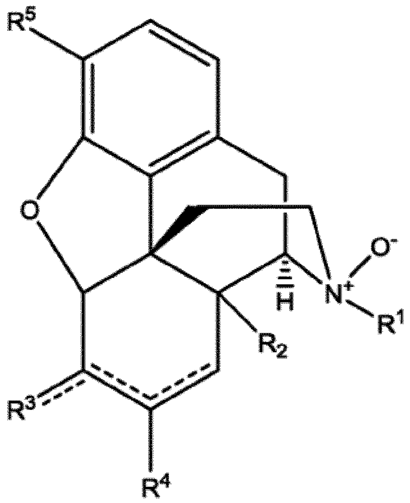

(VII)

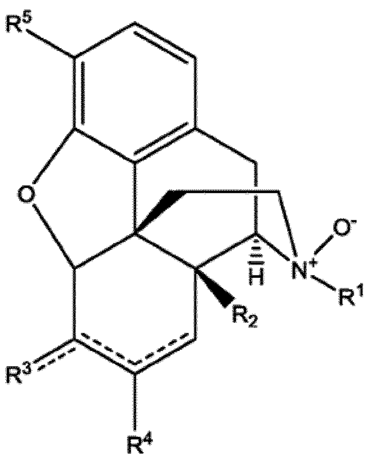

(VIII)

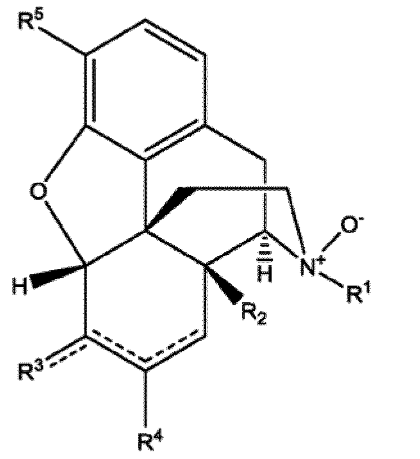

(IX)

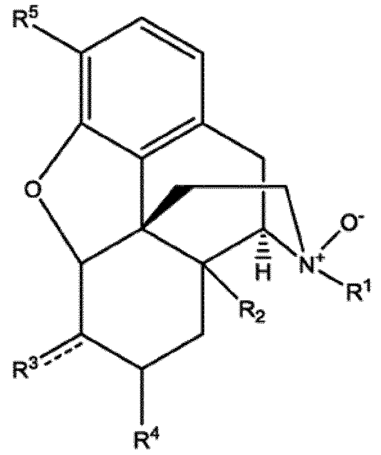

(X)

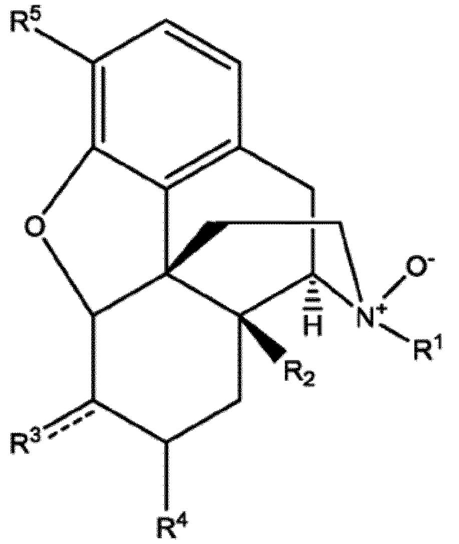

(XI)

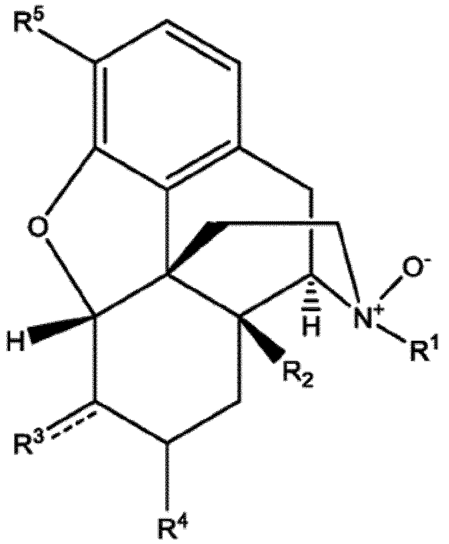

(XII)

wobei jedes von $R^1$, $R^2$, $R^3$, $R^4$ und $R^5$ und die gestrichelten Bindungen sind, wie in Anspruch 1 definiert.

**5.** Vorrichtung nach einem der vorstehenden Ansprüche, wobei $R^5$ in dem N-Oxid-Derivat der Formeln (I)-(XII) OH ist.

**6.** Vorrichtung nach einem der vorstehenden Ansprüche, wobei $R^4$ in dem N-Oxid-Derivat der Formeln (I)-(III) und (V)-(XII) H ist.

**7.** Vorrichtung nach einem der vorstehenden Ansprüche, wobei $R^2$ in dem N-Oxid-Derivat der Formeln (I)-(XII) OH ist.

**8.** Vorrichtung nach einem der vorstehenden Ansprüche, wobei $R^3$ in dem N-Oxid-Derivat der Formeln (I)-(III) und (V)-(XII) aus O, $CH_2$, N-$NH_2$ ausgewählt ist, wenn die gestrichelte Bindung vorhanden ist, und aus OH, H, $C_{1-8}$-Alkyl, $OCOR^1$, $O(CH_2CH_2(CH_2)_yO)_xCH_3$, wobei x 1-10 ist und y 0-3 ist, $N(R^1)_2$, $NR^1COR$, NHCOR, $CONR^1R$ und CONHR, wobei R eine Hydrocarbylgruppe ist, ausgewählt ist, wenn die gestrichelte Bindung nicht vorhanden ist, und $R^3$ vorzugsweise aus O oder $CH_2$ ausgewählt ist, wenn die gestrichelte Bindung vorhanden ist, und aus OH oder $OC_{1-8}$-Alkyl ausgewählt ist, wenn die gestrichelte Bindung nicht vorhanden ist, und noch stärker bevorzugt $R^3$ O ist und die gestrichelte Bindung vorhanden ist.

**9.** Vorrichtung nach Anspruch 1, wobei das N-Oxid-Derivat ein Derivat eines Antagonisten ist, ausgewählt aus Naloxon, Naloxol, Naloxegol, Naloxazon, Nalmefen, Nalbuphin, Nalmexon, Naltrexon, Naltrexol, Chlornaltrexamin, Clocinnamox, Nafurafin, Naldemedin, Naltrindol, 5'-Guanidinonaltrindol, Naltriben, Nalorphin, Diacetylnalorphin, Naloxonazin, Norbinaltorphimin, Binaltorphimin, Buprenorphin, Diprenorphin, Levellorphan, Cyprodim, Oxilorphan, Samidorphan und Cyclazocin, und das N-Oxid-Derivat vorzugsweise ein Derivat eines Antagonisten ist, ausgewählt aus Naloxon und Naltrexon.

**10.** Vorrichtung nach einem der vorstehenden Ansprüche, wobei der Opioidagonist aus Alfentanil, Allylprodin, Alphaprodin, Anileridin, Benzylmorphin, Bezitramid, Buprenorphin, Butorphanol, Carfentanil, Clonitazen, Codein, Desomorphin, Dextromoramid, Dezocin, Diampromid, Diamorphon, Dihydrocodein, Dihydromorphin, Dihydromorphon, Dihydroisomorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Etorphin, Dihydroetorphin, Fentanyl, Heroin, Hydrocodon, Hydromorphon, Hydromorphodon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Nalorphin, Nalbuphen, Normorphin, Norpipanon, Opium, Oxycodon, Oxymorphon, Pantopon, Papaveretum, schmerzstillenden Mitteln, Pentazocin, Phenadoxon, Phendimetrazin, Phendimetrazon, Phenomorphan, Phenazocin, Phenoperidin, Piminodin, Piritramid, Propheptazin, Promedol, Properidin, Propoxyphen, Propylhexedrin, Sufentanil, Tilidin, Tramadol und Mischungen davon ausgewählt ist.

**11.** Vorrichtung nach einem der Ansprüche 1 bis 10, die eine transdermale Zuführungsvorrichtung in Reservoirausführung, eine transdermale Zuführungsvorrichtung in Polymermatrixausführung oder eine transdermale Zuführungsvorrichtung in Arzneimittel-in-Haftmittel-Ausführung, vorzugsweise eine transdermale Zuführungsvorrichtung in Arzneimittel-in-Haftmittel-Ausführung ist.

**12.** Pharmazeutische Zusammensetzung, umfassend:

(i) ein N-Oxid-Derivat eines Opioidantagonisten der Formel (I) oder ein Salz davon, vorzugsweise ein N-Oxid-Derivat eines Opioidantagonisten der Formel (I), wie in einem der Ansprüche 2 bis 9 definiert;
(ii) ein Opioidagonist oder Salz davon, vorzugsweise ein Opioidagonist, wie in Anspruch 10 definiert; und
(iii) ein Haftmittel und/oder ein matrixbildendes Polymer,

wobei die Verbindung der Formel (I) ist:

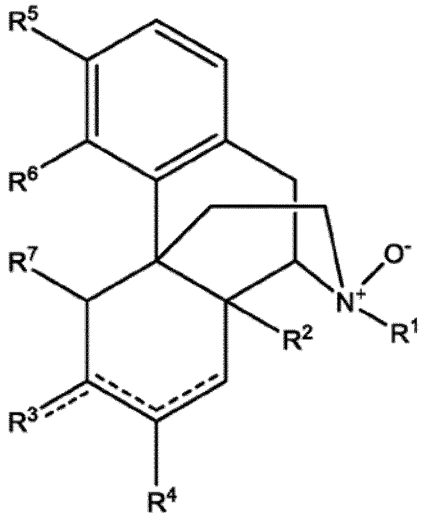

(I)

wobei

$R^1$ aus wahlweise substituiertem $C_{1-8}$-Alkyl und wahlweise substituiertem $C_{2-8}$-Alkenyl ausgewählt ist;
$R^2$ aus OH, H, $OC_{1-8}$-Alkyl, NHCOR, $NR^1COR$, $CONR^1R$ und CONHR ausgewählt ist, wobei R eine Hydrocarbylgruppe ist, oder $R^2$ eine Brücke zu dem Kohlenstoff, an das $R^3$ angefügt ist, bildet;
$R^3$ aus O, $CH_2$, $N\text{-}NH_2$ oder einer verbindenden Gruppe zu einem zweiten N-Oxid-Derivat eines Opioidantagonisten oder einem Salz davon ausgewählt ist, wenn die gestrichelte Bindung vorhanden ist, und aus OH, H, $OC_{1-8}$-Alkyl, $OCOR^1$, $O(CH_2CH_2(CH_2)_yO)_xCH_3$, wobei x 1-10 ist und y 0-3 ist, $N(R^1)_2$, $NR^1COR$, NHCOR, $CONR^1R$, CONHR, wobei R eine Hydrocarbylgruppe ist, oder eine verbindende Gruppe zu einem zweiten N-Oxid-Derivat eines Opioidantagonisten oder ein Salz davon ist, wenn die gestrichelte Bindung nicht vorhanden ist, und $R^4$ aus H, CONHR, $CONR^1R$, NHCOR, $NR^1COR$ und $C(OH)(R^1)_2$, wobei R eine Hydrocarbylgruppe ist, ausgewählt ist; oder
$R^3$ und $R^4$, zusammen mit den Kohlenstoffatomen, an die sie angefügt sind, einen Heterocyclus oder eine verbindende Gruppe zu einem zweiten N-Oxid-Derivat eines Opioidantagonisten oder ein Salz davon bilden;
$R^5$ aus OH, $OC_{1-8}$-Alkyl, $OCOR^1$, H und $CONH_2$ ausgewählt ist;
$R^6$ und $R^7$ jeweils unabhängig aus H, OH und $OC_{1-8}$-Alkyl ausgewählt sind oder $R^6$ und $R^7$, zusammen mit den Kohlenstoffatomen, an die sie angefügt sind, einen Dihydrofuranring bilden; und
jede gestrichelte Bindung in dem Cyclohexylring vorhanden oder nicht vorhanden sein kann, mit der Maßgabe, dass nicht beide vorhanden sein können.

**13.** Transdermale Dosierungsform, umfassend eine Zusammensetzung nach Anspruch 12.

**14.** Verfahren zum Herstellen einer Zusammensetzung nach Anspruch 12, umfassend Mischen eines N-Oxid-Derivats eines Opioidantagonisten der Formel (I), wie in einem der Ansprüche 1 bis 9 definiert oder eines Salzes davon, eines Opioidagonisten oder eines Salzes davon und eines Haftmittels und/oder eines matrixbildenden Polymers, um die Zusammensetzung zu bilden.

**15.** Pharmazeutische Zusammensetzung nach Anspruch 12 zur Verwendung in der Medizin, vorzugsweise zur Verwendung bei der Behandlung, z. B. transdermalen Behandlung, von Schmerzen.

## Revendications

1. Dispositif d'administration transdermique comprenant une composition pharmaceutique, dans lequel ladite composition comprend un dérivé de N-oxyde d'un antagoniste d'opioïde de la formule (I), ou un sel de celui-ci, et un agoniste d'opioïde ou un sel de celui-ci :

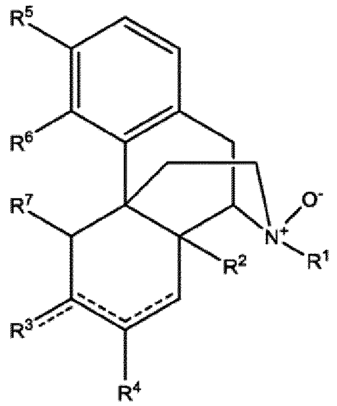

(I)

dans lequel:

$R^1$ est choisi parmi: $C_{1-8}$ alkyle optionnellement substitué et $C_{2-8}$ alcényle optionnellement substitué;
$R^2$ est choisi parmi: OH, H, $OC_{1-8}$ alkyle, NHCOR, $NR^1COR$, $CONR^1R$ et CONHR, où R est un groupe hydrocarbyle, ou $R^2$ forme un pont avec le carbone auquel $R^3$ est attaché;
$R^3$ est choisi parmi: O, $CH_2$, $N\text{-}NH_2$ ou un groupe liant à un deuxième dérivé de N-oxyde d'un antagoniste d'opioïde ou un sel de celui-ci lorsque la liaison en tirets est présente et il est choisi parmi: OH, H, $OC_{1-8}$ alkyle, $OCOR^1$, $O(CH_2CH_2(CH_2)_yO)_xCH_3$ où x est 1-10 et y est 0-3, $N(R^1)_2$, $NR^1COR$, NHCOR, $CONR^1R$, CONHR, où R est un groupe hydrocarbyle, ou un groupe liant à un deuxième dérivé de N-oxyde d'un antagoniste d'opioïde ou un sel de celui-ci, lorsque la liaison en tirets est absente et $R^4$ est choisi parmi: H, CONHR, $CONR^1R$, NHCOR, $NR^1COR$ et $C(OH)(R^1)_2$, où R est un groupe hydrocarbyle; ou
$R^3$ et $R^4$, ensemble avec les atomes de carbone auxquels ils sont attachés, forment un hétérocycle ou un groupe liant à un deuxième dérivé de N-oxyde d'un antagoniste d'opioïde ou un sel de celui-ci ;
$R^5$ est choisi parmi: OH, $OC_{1-8}$ alkyle, $OCOR^1$, H et $CONH_2$;
$R^6$ et $R^7$ sont chacun indépendamment choisis parmi: H, OH et $OC_{1-8}$ alkyle, ou $R^6$ et $R^7$, ensemble avec les atomes de carbone auxquels ils sont attachés, forment un cycle de dihydrofurane; et
chaque liaison en tirets dans le cycle de cyclohexyle peut être présente ou absente sous réserve que les deux ne puissent pas être présentes.

2. Dispositif selon la revendication 1, dans lequel ledit dérivé de N-oxyde est de la formule (II) ou (IIIa):

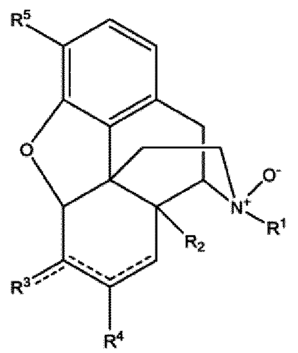

(II)

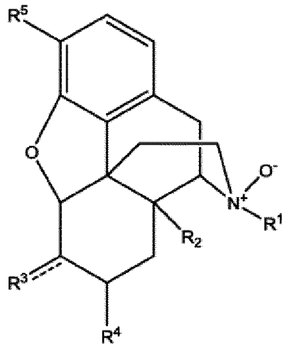

(IIIa)

dans lequel chacun de $R^1$, $R^2$, $R^3$, $R^4$ et $R^5$ et les liaisons en tirets sont tels que définis dans la revendication 1, ou ledit dérivé de N-oxyde est de la formule (IIIb) ou (IIIc):

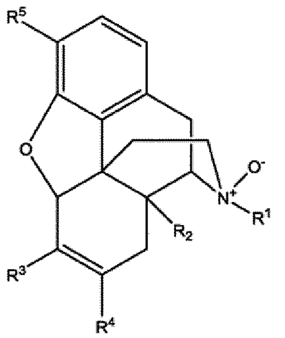

(IIIb)

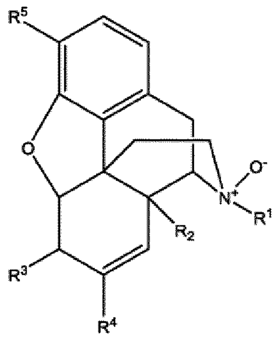

(IIIc)

dans lequel $R^1$, $R^2$ et $R^5$ sont chacun tels que définis dans la revendication 1; et
$R^3$ est choisi parmi: OH, H, $OC_{1-8}$ alkyle, $OCOR^1$, $O(CH_2CH_2(CH_2)_yO)_xCH_3$ où x est 1-10 et y est 0 à 3, $N(R^1)_2$, $NR^1COR$, NHCOR, $CONR^1R$, CONHR, où R est un groupe hydrocarbyle, ou un groupe liant à un deuxième dérivé de N-oxyde d'un antagoniste d'opioïde ou un sel de celui-ci ; $R^4$ est choisi parmi: H, CONHR, $CONR^1R$, NHCOR, $NR^1COR$ et $C(OH)(R^1)_2$, où R est un groupe hydrocarbyle; ou
$R^3$ et $R^4$, ensemble avec les atomes de carbone auxquels ils sont attachés, forment un hétérocycle ou un groupe liant à un deuxième dérivé de N-oxyde d'un antagoniste d'opioïde ou un sel de celui-ci,

ou ledit dérivé de N-oxyde est de la formule (IV):

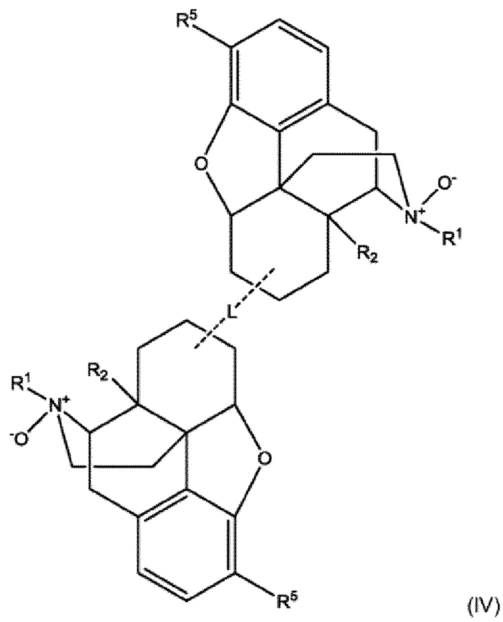

(IV)

dans lequel L est un groupe liant; et
$R^1$, $R^2$ et $R^5$ sont chacun indépendamment tels que définis dans la revendication 1.

**3.** Dispositif selon la revendication 1 ou 2, dans lequel ledit dérivé de N-oxyde est de la formule (V) ou (VI):

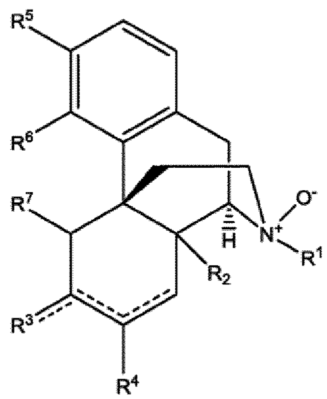

(V)

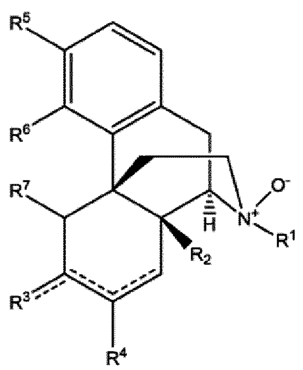

(VI)

dans lequel chacun de $R^1$, $R^2$, $R^3$, $R^4$, $R^5$, $R^6$, $R^7$ et les liaisons en tirets sont tels que définis dans la revendication 1.

**4.** Dispositif selon l'une quelconque des revendications 1 à 3, dans lequel ledit dérivé de N-oxyde est de la formule (VII), (VIII), (IX), (X), (XI) ou (XII):

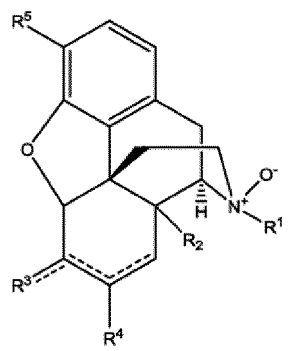

(VII)

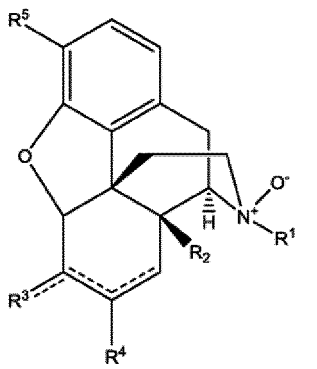

(VIII)

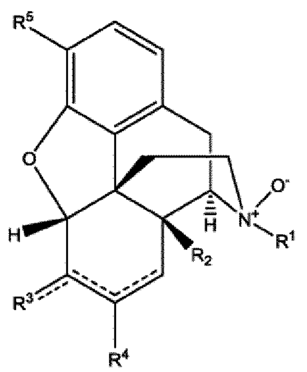

(IX)

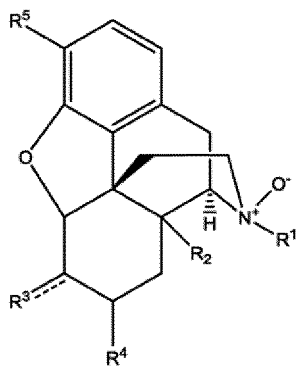

(X)

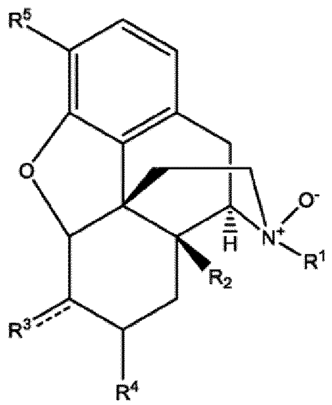

(XI)

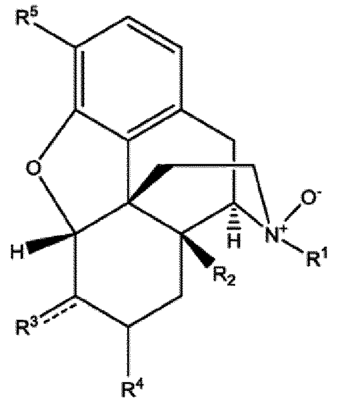

(XII)

dans lequel chacun de $R^1$, $R^2$, $R^3$, $R^4$ et $R^5$ et les liaisons en tirets sont tels que définis dans la revendication 1.

**5.** Dispositif selon l'une quelconque des revendications précédentes, dans lequel dans ledit dérivé de N-oxyde des formules (I)-(XII), $R^5$ est OH.

**6.** Dispositif selon l'une quelconque des revendications précédentes, dans lequel dans ledit dérivé de N-oxyde des formules (I)-(III) et (V)-(XII), $R^4$ est H.

**7.** Dispositif selon l'une quelconque des revendications précédentes, dans lequel dans ledit dérivé de N-oxyde des formules (I)-(XII), $R^2$ est OH.

**8.** Dispositif selon l'une quelconque des revendications précédentes, dans lequel dans ledit dérivé de N-oxyde des formules (I)-(III) et (V)-(XII), $R^3$ est choisi parmi: O, $CH_2$, N-$NH_2$ lorsque la liaison en tirets est présente et il est choisi parmi: OH, H, $C_{1-8}$ alkyle, $OCOR^1$, $O(CH_2CH_2(CH_2)_yO)_xCH_3$ où x est 1-10 et y est 0-3, $NCR^1)_2$, $NR^1COR$, NHCOR, $CONR^1R$, CONHR, où R est un groupe hydrocarbyle, lorsque la liaison en tirets est absente et de préférence $R^3$ est choisi parmi: O ou $CH_2$, lorsque la liaison en tirets est présente et il est choisi parmi: OH ou $OC_{1-8}$ alkyle, lorsque la liaison en tirets est absente et encore plus préférablement $R^3$ est O et la liaison en tirets est présente.

**9.** Dispositif selon la revendication 1, dans lequel ledit dérivé de N-oxyde est un dérivé d'un antagoniste choisi parmi: Naloxone, Naloxol, Naloxégol, Naloxazone, Nalméfène, Nalbuphine, Nalmexone, Naltrexone, Naltrexol, Chornaltrexamine, Clocinnamox, Nafurafine, Naldémédine, Naltrindole, 5'-Guanidinonaltrindole, Naltriben, Nalorphine, Diacétylnalorphine, Naloxonazine, Norbinaltorphimine, Binaltorphimine, Buprénorphine, Diprénorphine, Lévellorphan, Cyprodime, Oxilorphan, Samidorphan et Cyclazocine et de préférence ledit dérivé de N-oxyde est un dérivé d'un antagoniste choisi parmi: Naloxone et Naltrexone.

**10.** Dispositif selon l'une quelconque des revendications précédentes, dans lequel ledit agoniste d'opioïde est choisi parmi: alfentanil, allylprodine, alphaprodine, anileridine, benzylmorphine, bézitramide, buprénorphine, butorphanol, carfentanil, clonitazène, codéine, désomorphine, dextromoramide, dezocine, diampromide, diamorphone, dihydrocodéine, dihydromorphine, dihydromorphone, dihydroisomorphine, diménoxadol, dimépheptanol, diméthylthiambutène, butyrate de dioxaphétyle, dipipanone, eptazocine, éthoheptazine, éthylméthylthiambutène, éthylmorphine, étonitazène, étorphine, dihydroétorphine, fentanyl, héroïne, hydrocodone, hydromorphone, hydromorphodone, hydroxypéthidine, isométhadone, kétobémidone, lévorphanol, lévophénacylmorphan, lofentanil, mépéridine, meptazinol, métazocine, méthadone, métopon, morphine, myrophine, narcéine, nicomorphine, norlévorphanol, norméthadone, nalorphine, nalbuphine, normorphine, norpipanone, opium, oxycodone, oxymorphone, pantopon, papaveretum, élixir parégorique, pentazocine, phénadoxone, phendimétrazine, phendimétrazone, phénomorphan, phénazocine, phénopéridine, piminodine, piritramide, propheptazine, promédol, propéridine, propoxyphène, propylhexédrine, sufentanil, tilidine, tramadol et des mélanges de ceux-ci.

**11.** Dispositif selon l'une quelconque des revendications 1 à 10, qui est un dispositif d'administration transdermique de type réservoir, un dispositif d'administration transdermique de type matrice de polymère ou un dispositif d'administration transdermique de type médicament-dans-adhésif, de préférence un dispositif d'administration transdermique de type médicament-dans-adhésif.

**12.** Composition pharmaceutique comprenant:

(i) un dérivé de N-oxyde d'un antagoniste d'opioïde de la formule (I), ou un sel de celui-ci, de préférence un dérivé de N-oxyde d'un antagoniste d'opioïde de la formule (I) tel que défini dans l'une quelconque des revendications 2 à 9;
(ii) un agoniste d'opioïde ou un sel de celui-ci, de préférence un agoniste d'opioïde tel que défini dans la revendication 10; et
(iii) un adhésif et/ou un polymère formant une matrice,

où ledit composé de la formule (I) est:

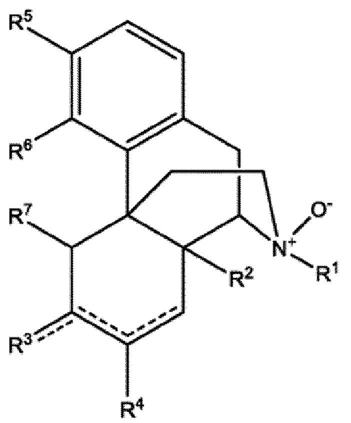

(I)

dans lequel:

$R^1$ est choisi parmi: $C_{1-8}$ alkyle optionnellement substitué et $C_{2-8}$ alcényle optionnellement substitué;
$R^2$ est choisi parmi: OH, H, $OC_{1-8}$ alkyle, NHCOR, $NR^1COR$, $CONR^1R$ et CONHR, où R est un groupe hydrocarbyle, ou $R^2$ forme un pont avec le carbone auquel $R^3$ est attaché;
$R^3$ est choisi parmi: O, $CH_2$, $N\text{-}NH_2$ ou un groupe liant à un deuxième dérivé de N-oxyde d'un antagoniste d'opioïde ou un sel de celui-ci lorsque la liaison en tirets est présente et il est choisi parmi: OH, H, $OC_{1-8}$ alkyle, $OCOR^1$, $O(CH_2CH_2(CH_2)_yO)_xCH_3$ où x est 1-10 et y est 0-3, $N(R^1)_2$, $NR^1COR$, NHCOR, $CONR^1R$, CONHR, où R est un groupe hydrocarbyle, ou un groupe liant à un deuxième dérivé de N-oxyde d'un antagoniste d'opioïde ou un sel de celui-ci, lorsque la liaison en tirets est absente et $R^4$ est choisi parmi: H, CONHR, $CONR^1R$, NHCOR, $NR^1COR$ et $C(OH)(R^1)_2$, où R est un groupe hydrocarbyle; ou
$R^3$ et $R^4$, ensemble avec les atomes de carbone auxquels ils sont attachés, forment un hétérocycle ou un groupe liant à un deuxième dérivé de N-oxyde d'un antagoniste d'opioïde ou un sel de celui-ci ;
$R^5$ est choisi parmi: OH, $OC_{1-8}$ alkyle, $OCOR^1$, H et $CONH_2$;
$R^6$ et $R^7$ sont chacun indépendamment choisis parmi: H, OH et $OC_{1-8}$ alkyle, ou $R^6$ et $R^7$, ensemble avec les atomes de carbone auxquels ils sont attachés, forment un cycle de dihydrofurane; et
chaque liaison en tirets dans le cycle de cyclohexyle peut être présente ou absente sous réserve que les deux

ne puissent pas être présentes.

**13.** Forme galénique transdermique comprenant une composition selon la revendication 12.

**14.** Méthode pour la fabrication d'une composition selon la revendication 12, comprenant le mélange d'un dérivé de N-oxyde d'un antagoniste d'opioïde de la formule (I) tel que défini dans l'une quelconque des revendications 1 à 9, ou un sel de celui-ci, d'un agoniste d'opioïde, ou un sel de celui-ci, et d'un adhésif et/ou d'un polymère formant une matrice pour former ladite composition.

**15.** Composition pharmaceutique selon la revendication 12 pour une utilisation en médecine, de préférence pour une utilisation dans le traitement, par ex. traitement transdermique, d'une douleur.

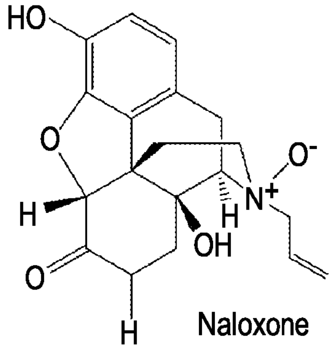

Naloxone

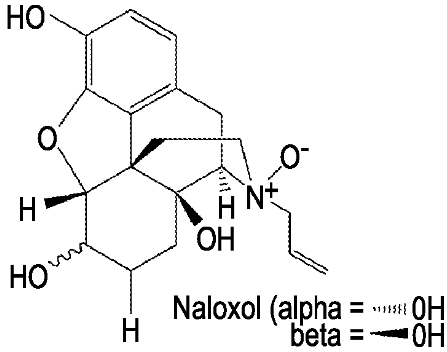

Naloxol (alpha = OH
beta = OH

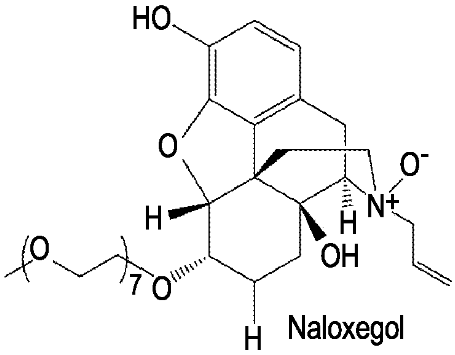

Naloxegol

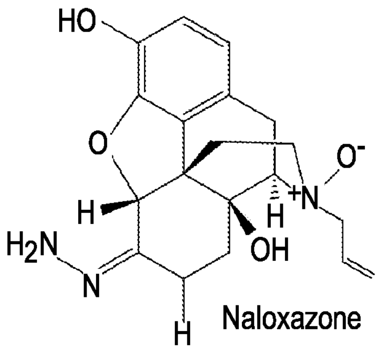

Naloxazone

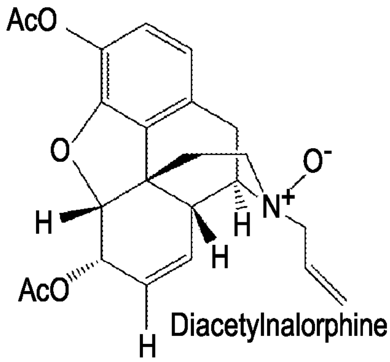

Diacetylnalorphine

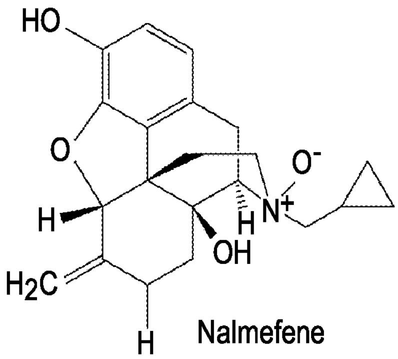

Nalmefene

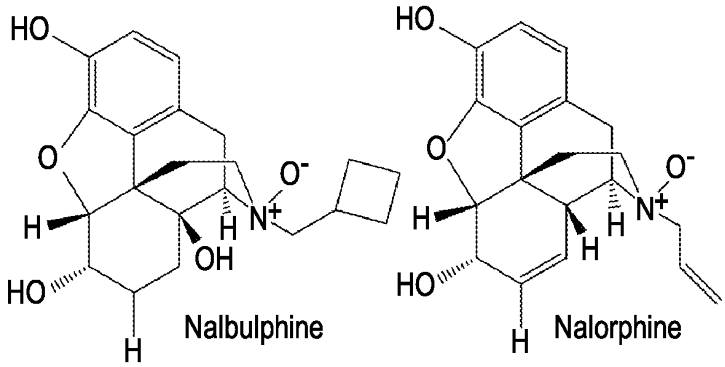

Nalbulphine

Nalorphine

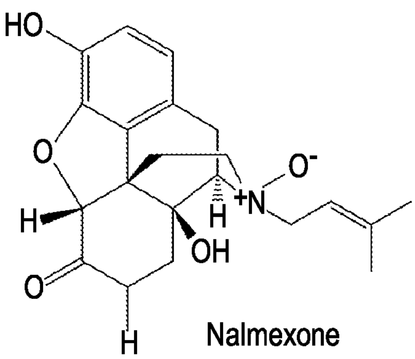

Nalmexone

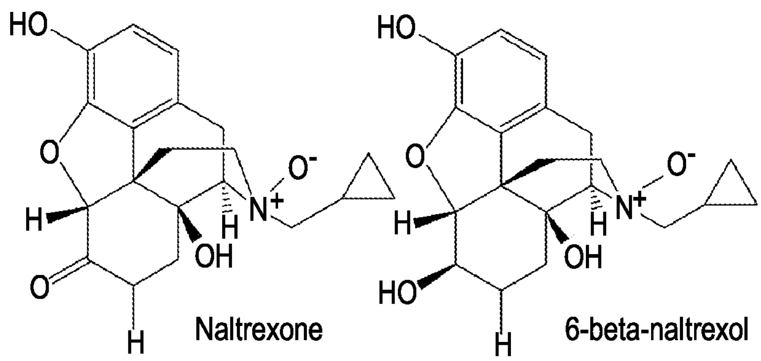

Naltrexone

6-beta-naltrexol

Figure 1

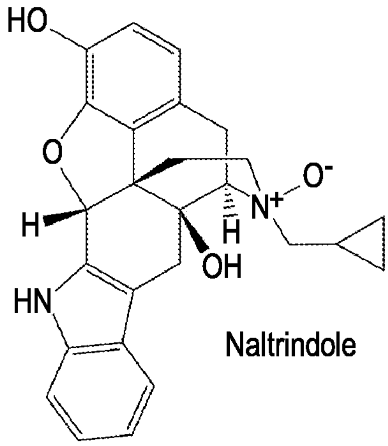

Naltrindole

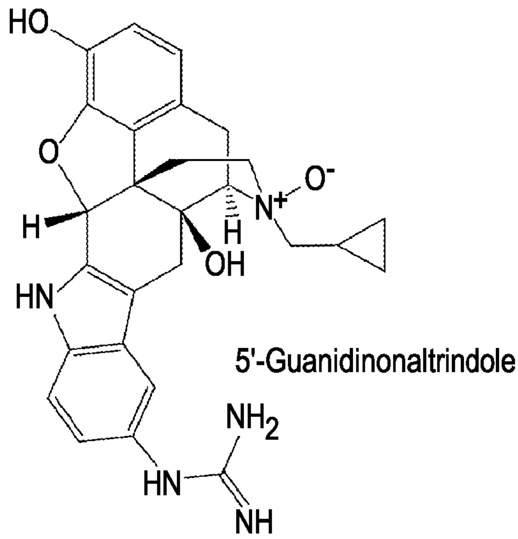

5'-Guanidinonaltrindole

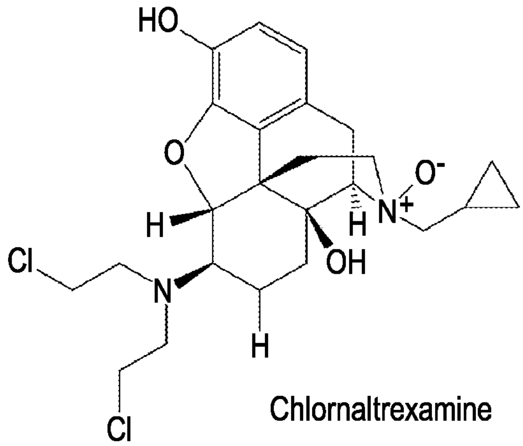

Chlornaltrexamine

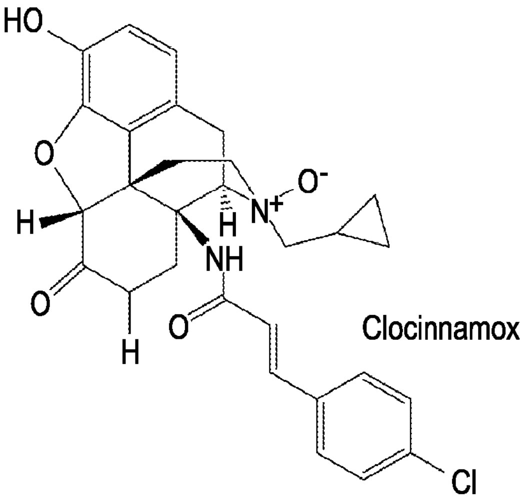

Clocinnamox

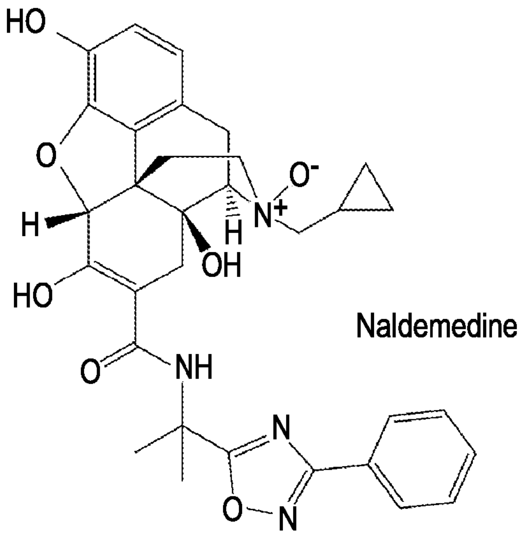

Naldemedine

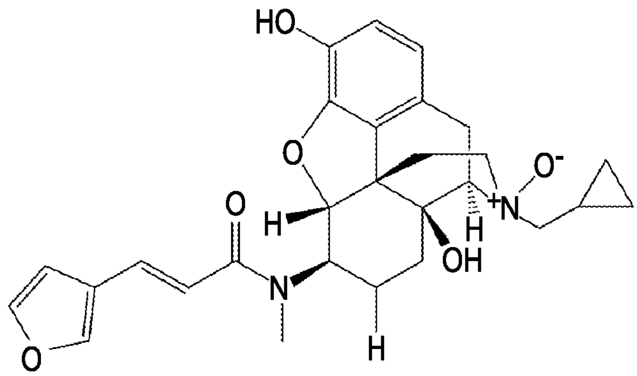

Nafurafine

Figure 1 (continued)

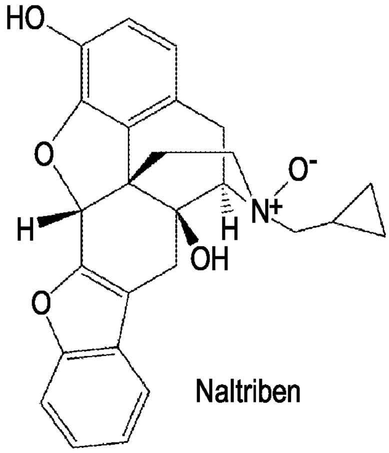

Naltriben

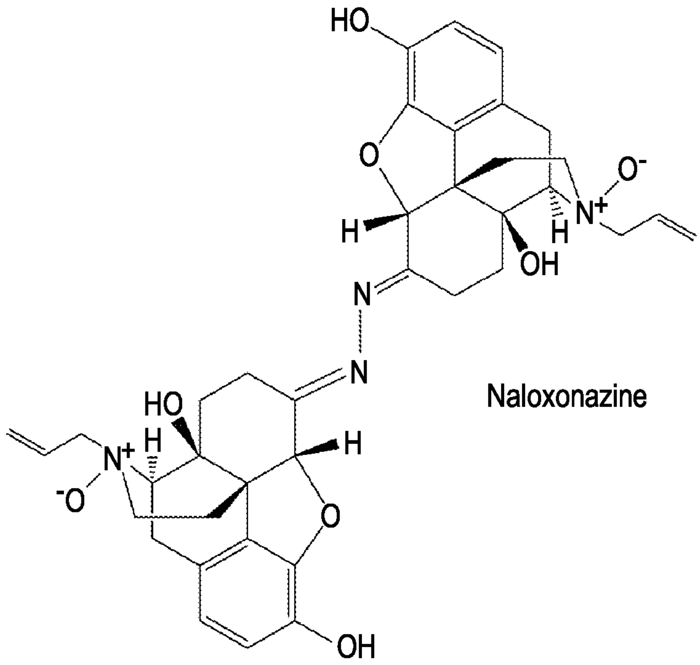

Naloxonazine

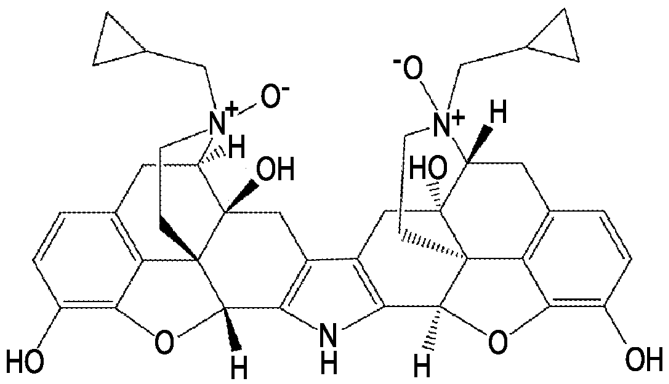

Norbinaltorphimine

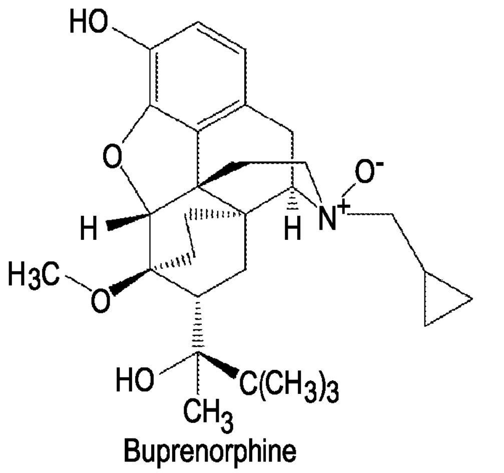

Buprenorphine

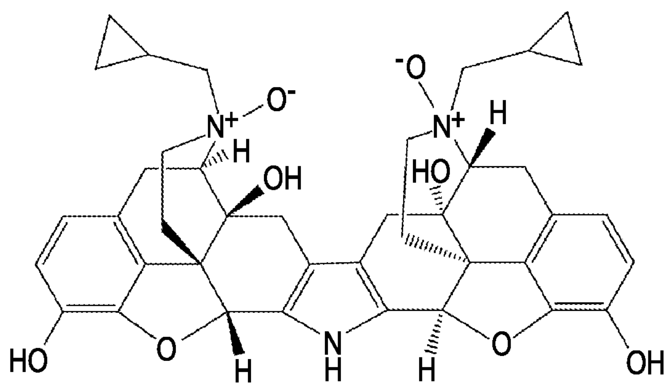

Binaltorphimine

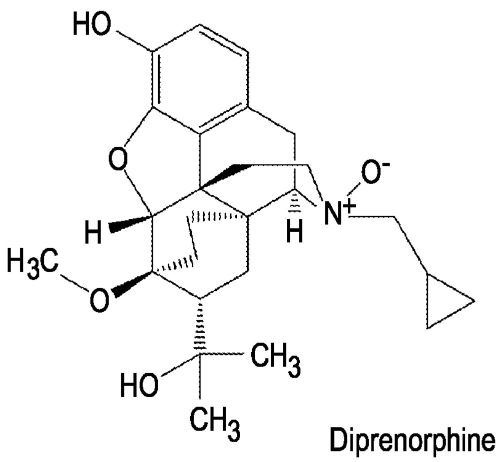

Diprenorphine

Figure 1 (continued)

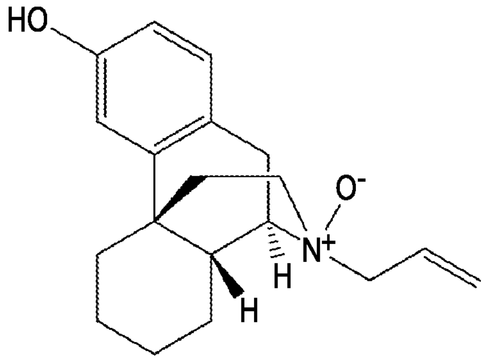

Levallorphan

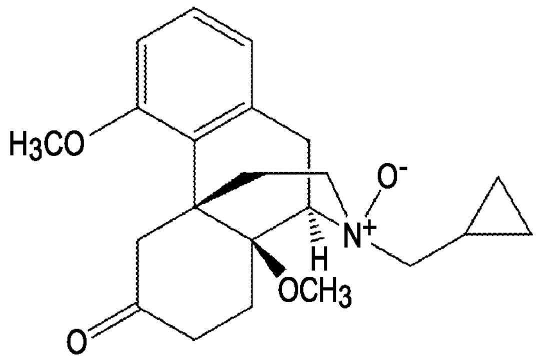

Cyprodime

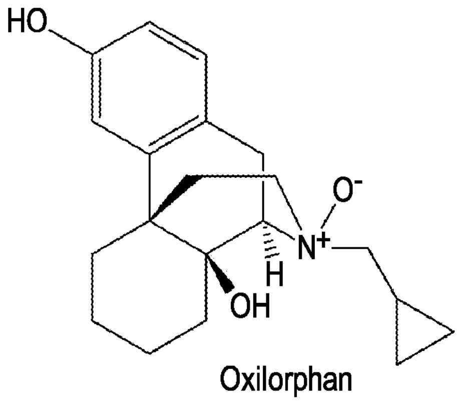

Oxilorphan

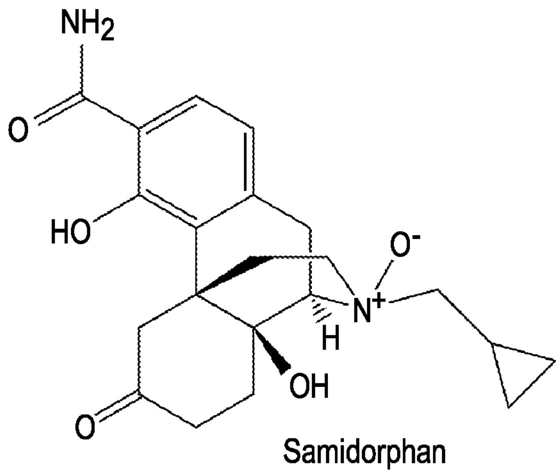

Samidorphan

Figure 1 (continued)

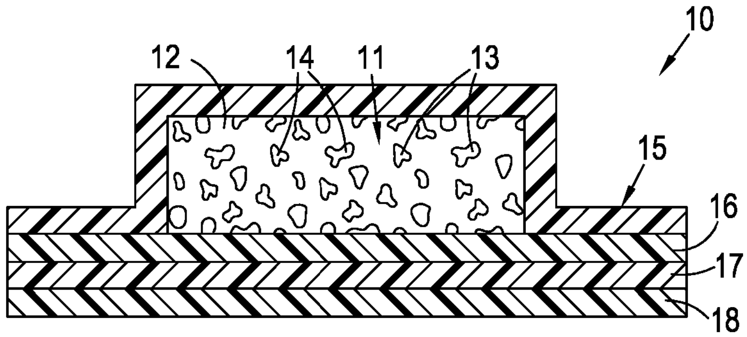


Figure 2

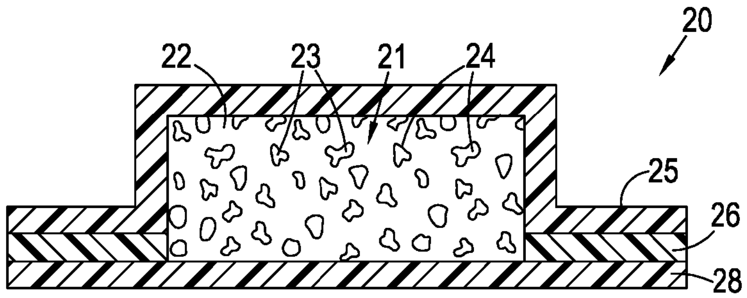


Figure 3

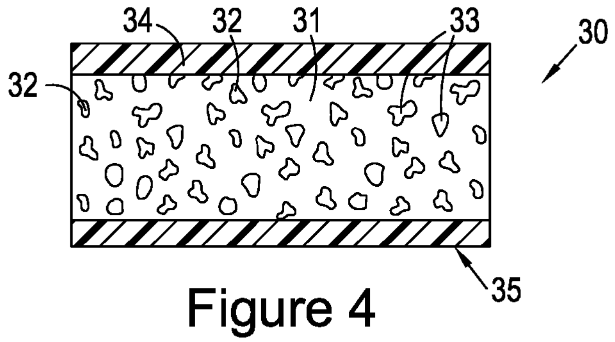


Figure 4

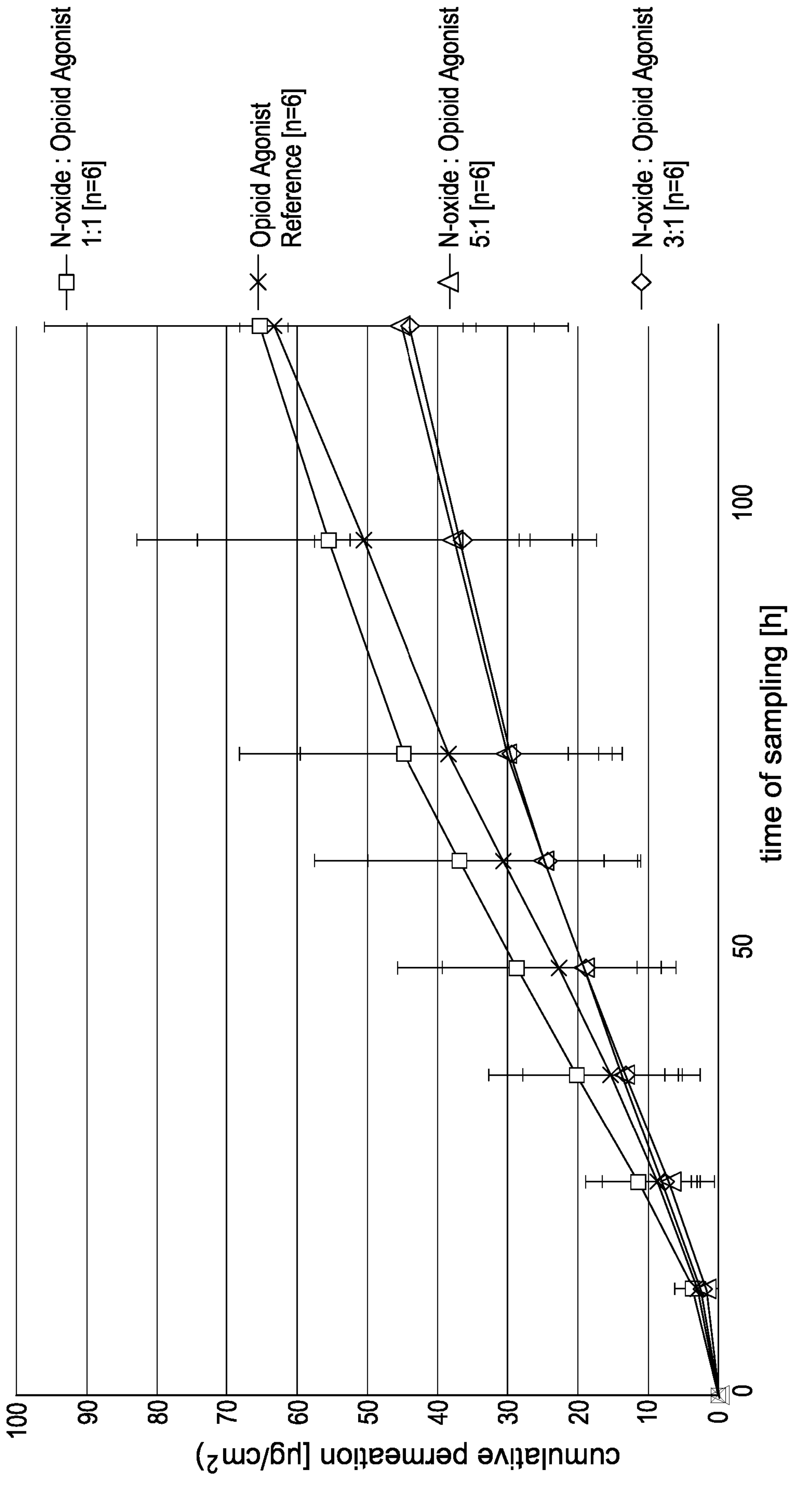
cumulative permeation [µg/cm²]
time of sampling [h]
0
10
20
30
40
50
60
70
80
90
100
0
50
100
N-oxide : Opioid Agonist 1:1 [n=6]
Opioid Agonist Reference [n=6]
N-oxide : Opioid Agonist 5:1 [n=6]
N-oxide : Opioid Agonist 3:1 [n=6]

Figure 5

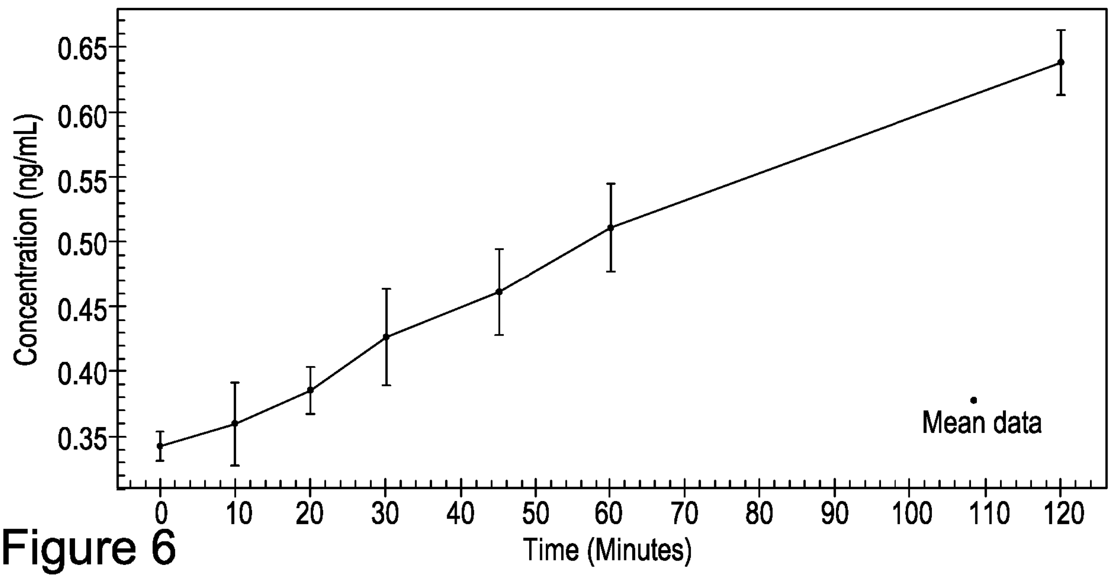


Figure 6

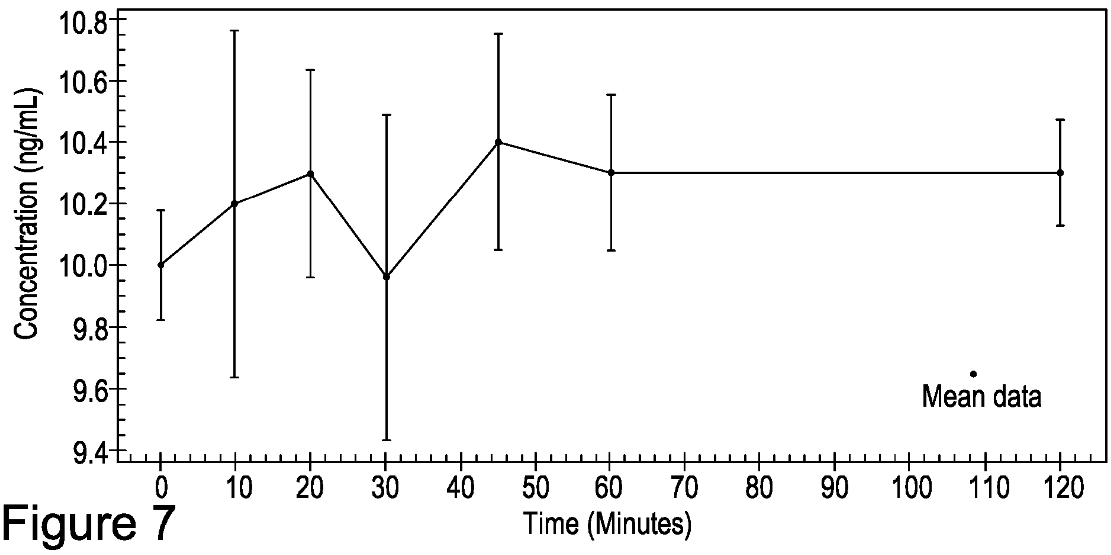


Figure 7

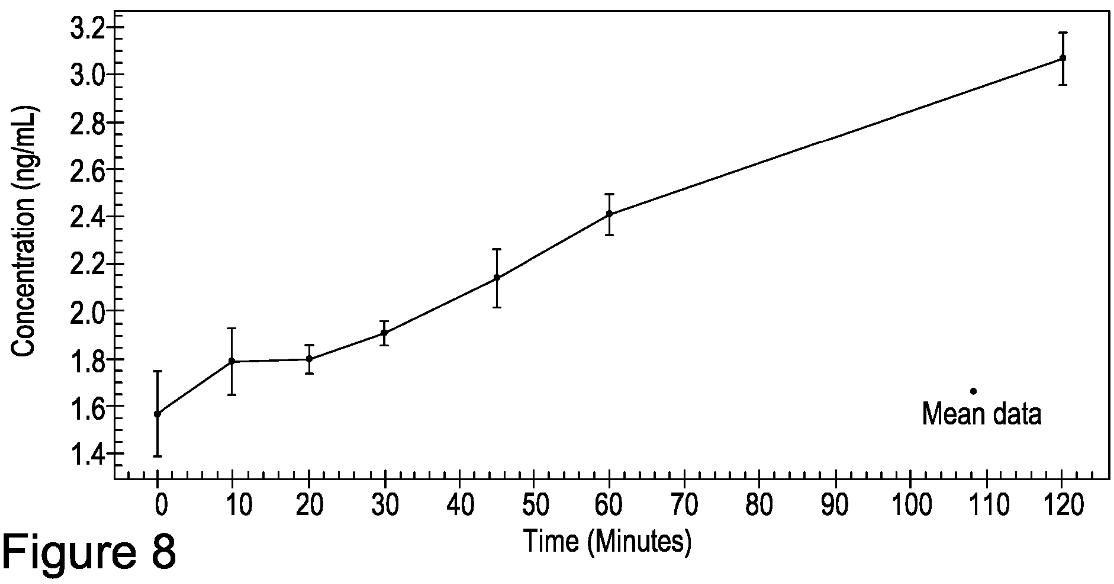


Figure 8

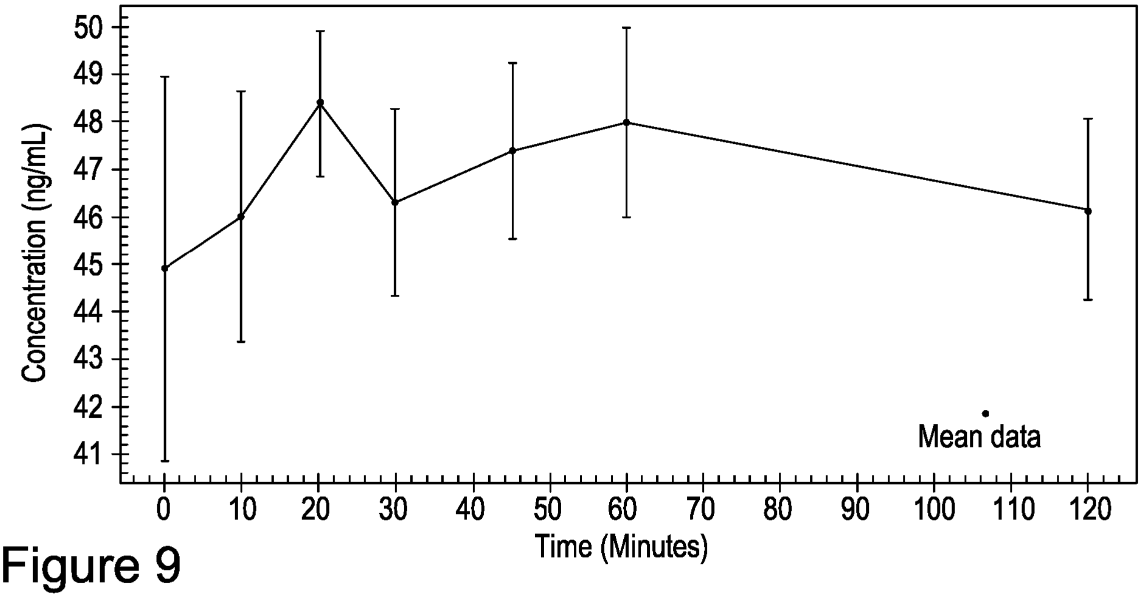


Figure 9

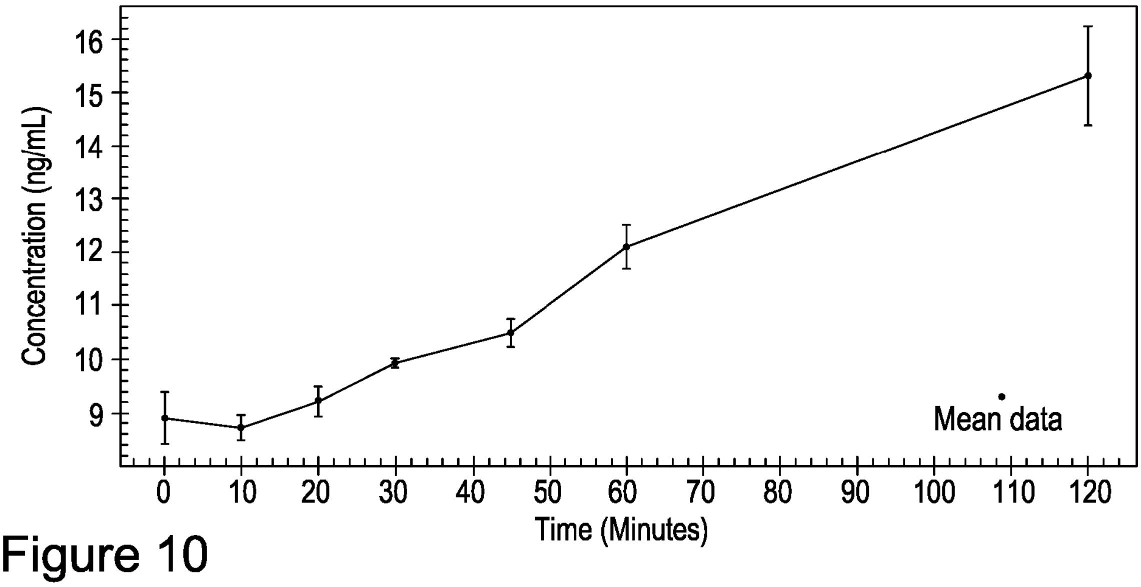


Figure 10

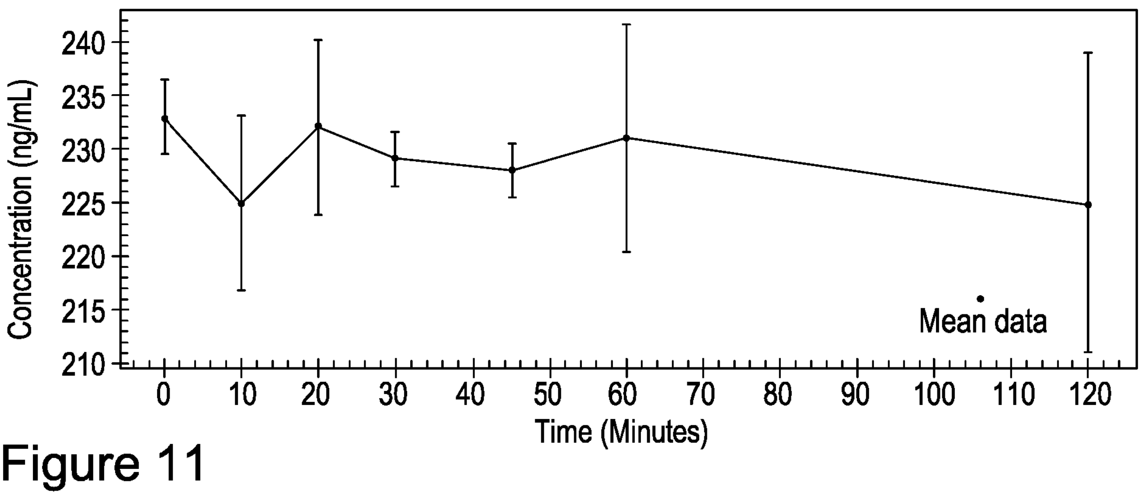


Figure 11

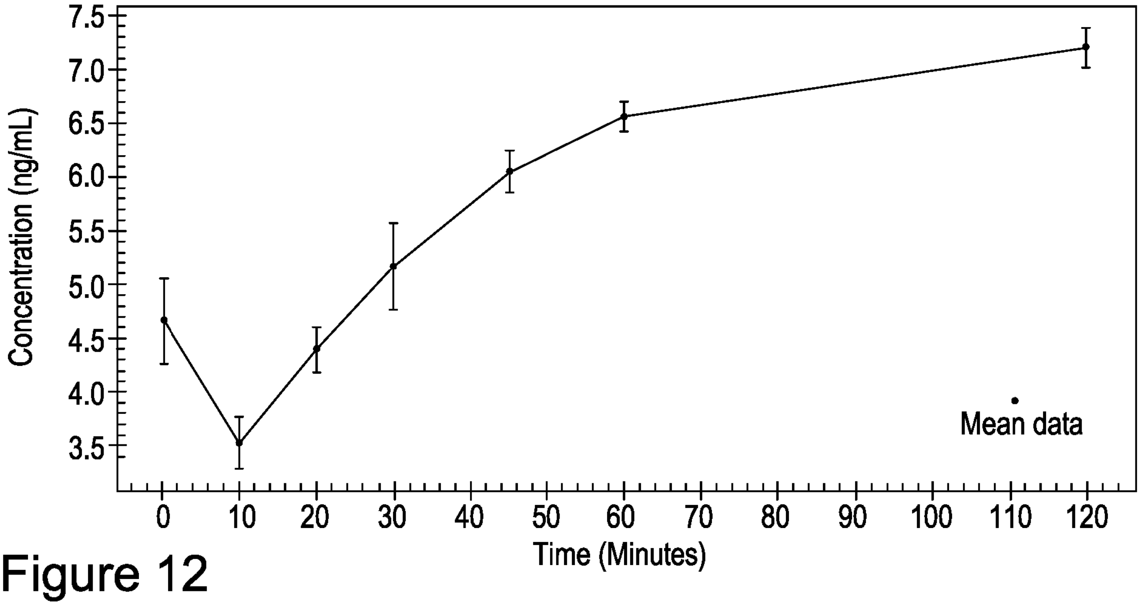


Figure 12

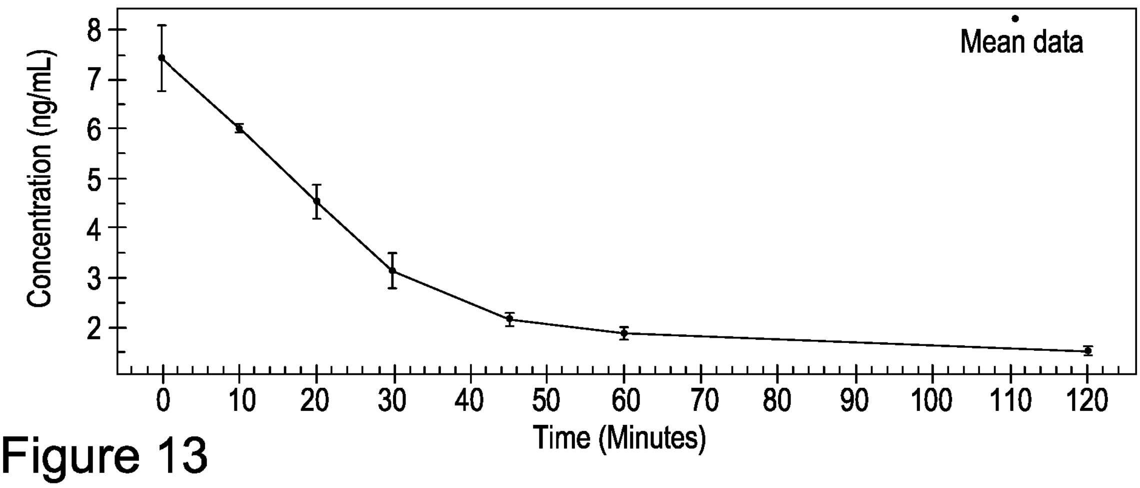


Figure 13

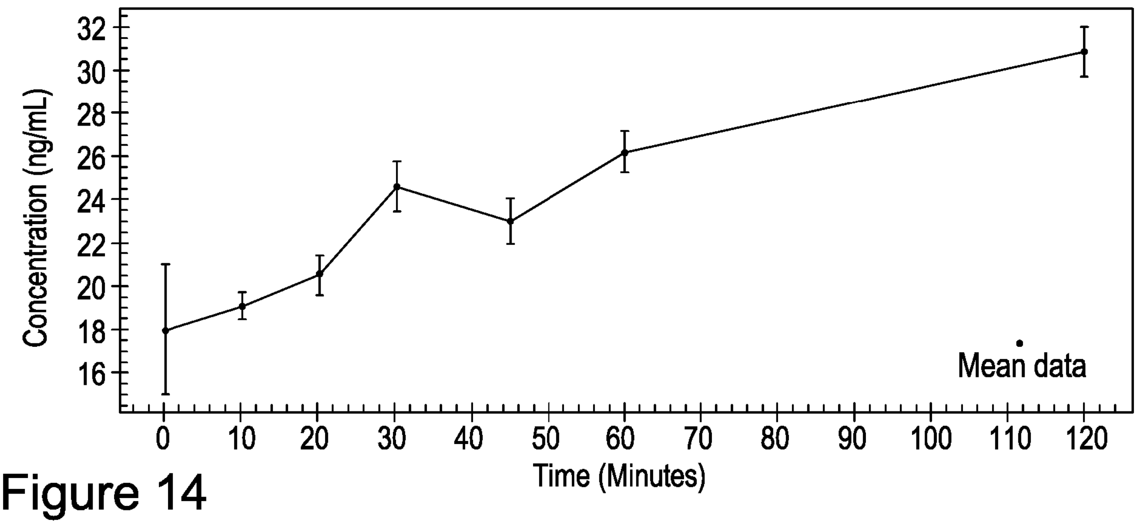


Figure 14

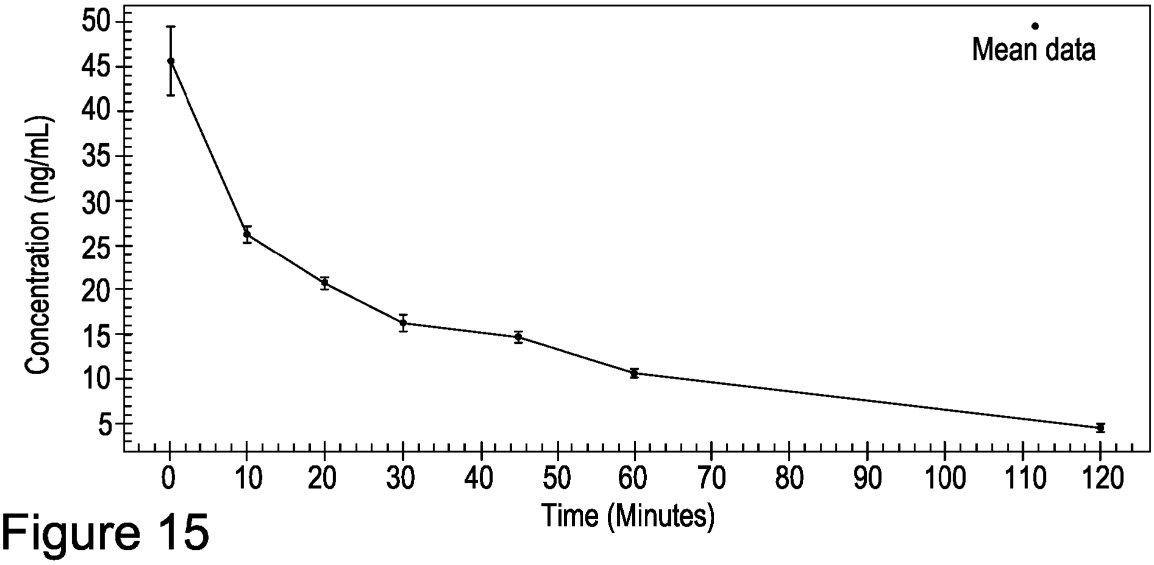


Figure 15

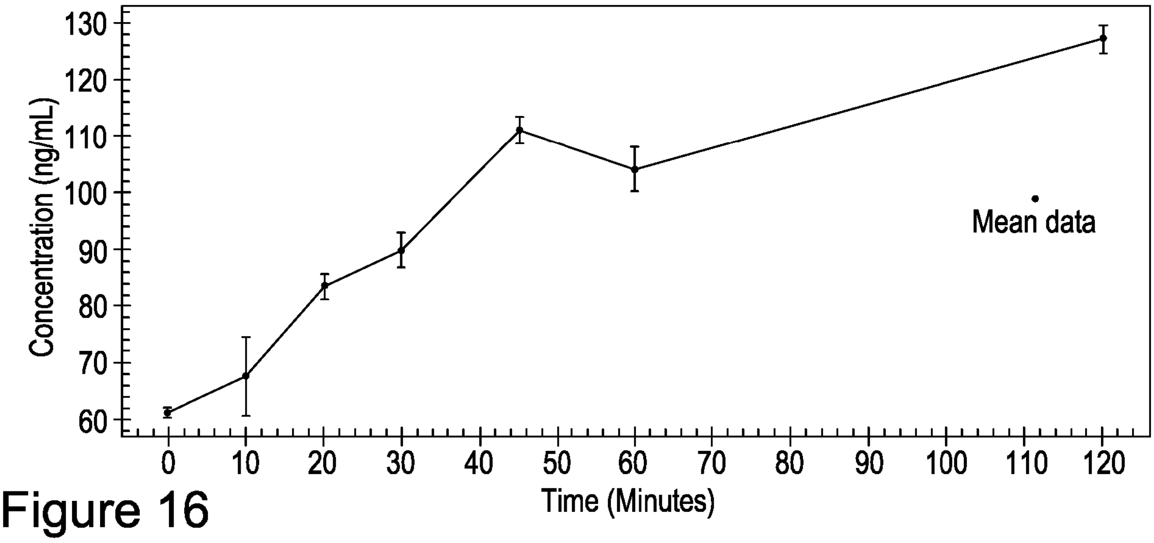


Figure 16

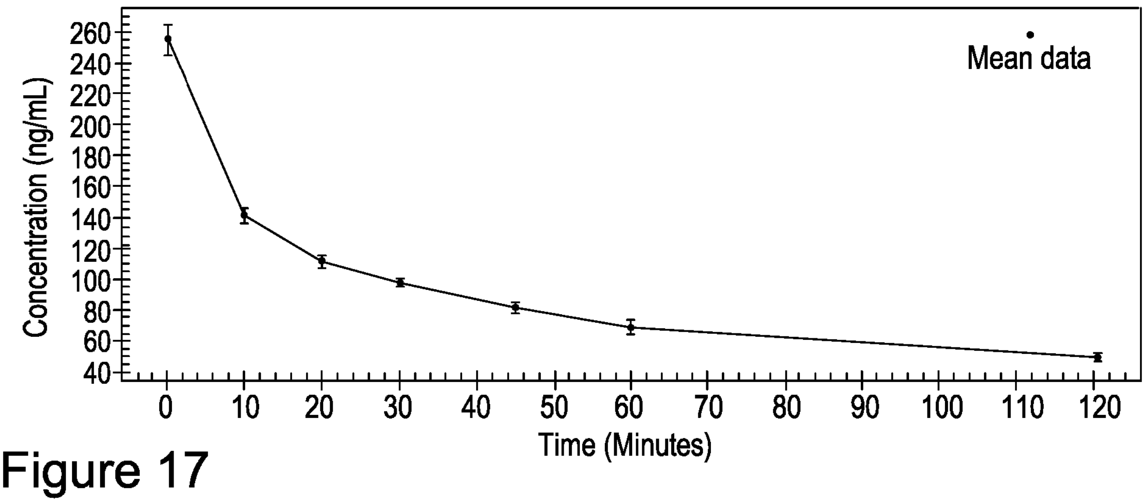


Figure 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040033253 A
- WO 2009120889 A
- US 4722928 A
- CH 683005

### Non-patent literature cited in the description

- Guideline on Bioanalytical Method Validation. CHMP, July 2011
- Reflection paper for laboratories that perform the analysis or evaluation of clinical trial samples. 28 February 2012
- Guidance for Industry, Bioanalytical Method Validation. U.S. Department of Health and Human Services, May 2001